# EUROPEAN PATENT SPECIFICATION

(11) **EP 3 301 105 B1**
(45) Date of publication and mention of the grant of the patent: **15.04.2020**
(21) Application number: 17191658.8
(22) Date of filing: 15.07.2011
(51) Int. Cl.: C07J 63/00, A61K 31/21, A61K 31/704, A61P 35/00, C07H 15/24

(54) **NEW OLEANANE-24-ESTERS FOR TREATING CANCER AND OTHER DISEASES**
NEUE OLEANAN-24-ESTER VERBINDUNGEN ZUR BEHANDLUNG VON KREBS UND ANDEREN KRANKHEITEN
NOUVEAUX 24-ESTERS DE L'OLÉANANE POUR LE TRAITEMENT DU CANCER ET D'AUTRES MALADIES

(30) Priority: 16.07.2010 WO PCT/US2010/042240; 13.08.2010 US 856322
(43) Date of publication of application: 04.04.2018
(62) Divisional of application: 11807584.5
(73) Proprietor: Pacific Arrow Limited, Tortola, VG1110 (VG)
(72) Inventor: CHAN, Pui-Kwong, Sugarland, Texas 77479 (US); MAK, May Sung, Kowloon, Hong Kong (CN)
(74) Representative: Brann AB

(56) References cited:
- WO-A1-2009/117196
- WANG P ET AL: "Cytotoxicity and inhibition of DNA topoisomerase I of polyhydroxylated triterpenoids and triterpenoid glycosides", BIOORGANIC & MEDICINAL CHEMISTRY LETTERS, PERGAMON, AMSTERDAM, NL, vol. 20, no. 9, 1 May 2010 (2010-05-01), pages 2790-2796, XP027012834, ISSN: 0960-894X [retrieved on 2010-03-15]
- IRMENTRAUT LÖW: "Über Acylwanderungen bei Protoäscigeninestern", HOPPE-SEYLER'S ZEITSCHRIFT FÜR PHYSIOLOGISCHE CHEMIE, vol. 348, no. Jahresband, 1 January 1967 (1967-01-01), pages 839-842, XP055122744, ISSN: 0018-4888, DOI: 10.1515/bchm2.1967.348.1.839
- DATABASE CA [Online] CHEMICAL ABSTRACTS SERVICE, COLUMBUS, OHIO, US; IGARASHI, KIKUO ET AL: "13,28-Epoxyolcananes", XP002778196, retrieved from STN Database accession no. 1975:497648 & JP S50 64265 A 31 May 1975 (1975-05-31)

## Description

### FIELD OF THE INVENTION

This invention provides compounds, compositions and compounds for inhibiting cancer invasion, cell invasion, or cancer cell invasion.

### BACKGROUND OF THE INVENTION

This invention provides methods of synthesing new compounds for pharmaceutical uses. This invention provides methods, compounds and compositions for treating cancer, inhibiting cancer invasion, cell invasion, or cancer cell invasion, wherein the cancers comprise breast, leukocytic, liver, ovarian, bladder, prostatic, skin, bone, brain, leukemia, lung, colon, CNS, melanoma, renal, cervical, esophageal, testicular, spleenic, kidney, lymphatic, pancreatic, stomach and thyroid cancers.

WO-A-2009/117196 and Bioorg. Med. Chem. Lett, Vol 20(9), pp 2790-2796 (01.05.2010) disclose esters of proto-aescin and their anti-cancer activity.

### SUMMARY OF THE INVENTION

This invention provides compounds, compositions, and compounds for treating cancer, inhibiting cancer invasion, cell invasion, cancer cell invasion, and metastasis. This invention provides a use of compounds, compositions, for manufacturing medicament for treating cancer, inhibiting cancer invasion, and metastasis. This invention provides compounds for use as mediator or inhibitor of adhesion protein or angiopoietin, This invention provides compounds for use in a method of modulating attachment or adhesion of cells or angiogenesis, by modulating or inhibiting adhesion protein or angiopoietin, The compounds comprise the structures selected from the formulae as specified in the claims, wherein the cancers comprise breast, leukocytic, liver, ovarian, bladder, prostatic, skin, bone, brain, leukemia, lung, colon, CNS, melanoma, renal, cervical, esophageal, testicular, spleenic, kidney, lymphatic, pancreatic, stomach and thyroid cancers. This invention provides these compounds for use as a mediator for cell circulating, cell moving and inflammatory diseases.

### DETAILED DESCRIPTION OF THE FIGURES

**Figure 1** HPLC profiles of esterification products of E4A with Tigloyl chloride (A) from different times of esterification reaction. Reaction products obtained from each time of reaction (5 sec, 1 min, 2 min, 5 min, and 10 min) were fractionated by HPLC. The profile is plotted according to HPLC elution time and optical density of fractions. Reaction was performed at Room temperature (Top row) and 0 C (bottom row).
**Figure 2** HPLC profiles of esterification products of E4A with 3,3-dimethylacryloly chloride (B) from different times of esterification reaction. Reaction products obtained from each time of reaction (5 sec, 1 min, 2 min, 5 min, and 10 min) were fractionated by HPLC. The profile is plotted according to HPLC elution time and optical density of fractions. Reaction was performed at Room temperature (Top row) and 0 C (bottom row).
**Figure 3** HPLC profiles of esterification products of E4A with 4-Pentenoyl chloride (C) from different times of esterification reaction. Reaction products obtained from each time of reaction (5 sec, 1 min, 2 min, 5 min, and 10 min) were fractionated by HPLC. The profile is plotted according to HPLC elution time and optical density of fractions. Reaction was performed at Room temperature.

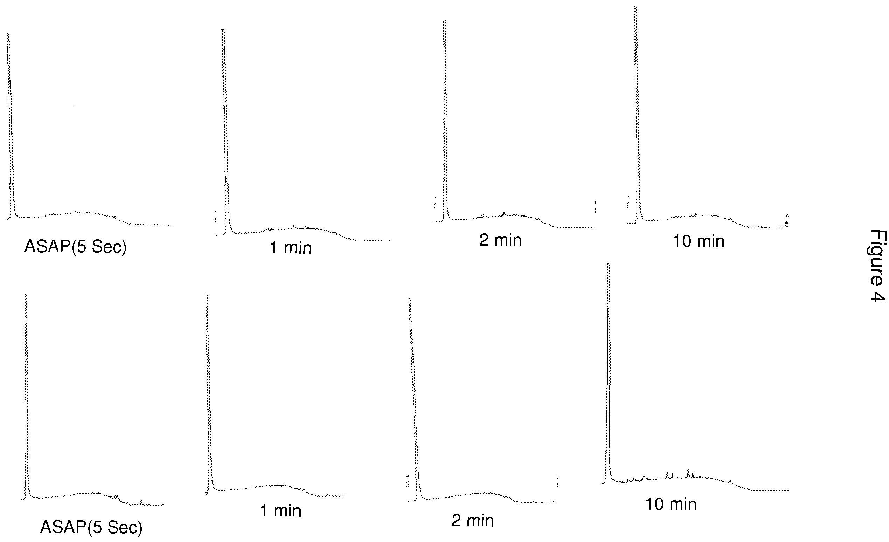

**Figure 4** HPLC profiles of esterification products of E4A with Hexanoly chloride (D) from different times of esterification reaction. Reaction products obtained from each time of reaction (5 sec, 1 min, 2 min, and 10 min) were fractionated by HPLC. The profile is plotted according to HPLC elution time and optical density of fractions. Reaction was performed at 0C (Top row); and shows the results of HPLC profiles of esterification products of E4A with 2-ethylbutyryl chloride (E) from different times of esterification reaction. Reaction products obtained from each time of reaction (5 sec, 1 min, 2 min, and 10 min) were fractionated by HPLC. The profile is plotted according to HPLC elution time and optical density of fractions. Reaction was performed at 0C.(bottom row)
**Figure 5** HPLC profiles of esterification products of E4A with Acetyl chloride (H) from different times of esterification reaction. Reaction products obtained from each time of reaction (1 min, 2 min, 5 min and 10 min) were fractionated by HPLC. The profile is plotted according to HPLC elution time and optical density of fractions. Reaction was performed at Room temperature.
**Figure 6** HPLC profiles of esterification products of E4A with Crotonoyl chloride (I) from different times of esterification reaction. Reaction products obtained from each time of reaction (5 sec, 1 min, 2 min, 5 min and 10 min) were fractionated by HPLC. The profile is plotted according to HPLC elution time and optical density of fractions. Reaction was performed at Room temperature.
**Figure 7** HPLC profiles of esterification products of E4A with Cinnamoyl chloride (J) from different times of esterification reaction. Reaction products obtained from each time of reaction (1 min, 1hour, 2 hours, 18 hours, 18 hours(heat)) were fractionated by HPLC. The profile is plotted according to HPLC elution time and optical density of fractions. Reaction was performed at Room temperature and 75C.
**Figure 8** HPLC profiles of esterification products of E4A with Benzoyl chloride (K) from different times of esterification reaction. Reaction products obtained from each time of reaction (5 sec, 1 min, 2 min, 5 min, and 10 min) were fractionated by HPLC. The profile is plotted according to HPLC elution time and optical density of fractions. Reaction was performed at 0C.
**Figure 9** MTT cytotoxic activity of times study at room temperature for A: E4A-Tigloyl; B: E4A- 3,3-dimethylacryloly; C: E4A-4-pentenoyl.
**Figure 10** MTT cytotoxic activity of times study at 0C for A: E4A-Tigloyl; B: E4A-3,3-dimethylacryloly; C: E4A-4-pentenoyl.
**Figure 11** MTT cytotoxic activity of times study_for J: E4A-cinnamoyl; D: E4A-hexanoyl; E: E4A-2-ethylbutyryl; and controls: Tig control is tigloyl chloride without E4A; AC control is acetyl chloride without E4A; H is acetyl chloride with E4A reaction 1 min.
**Figure 12** MTT cytotoxic activity of times study for H: E4A-acetyl; I: E4A-crotonoyl
**Figure 13** MTT cytotoxic activity of times study for E4A-Tig in 1min, 15 min, 30 min, 1 hour, 2 hours
**Figure 14** HPLC profiles of E4A-Tig in 1min and 2 hours
**Figure 15** MTT cytotoxic activity of times study for E4A-Tig. Results: E4A-Tigs from reaction of 5 sec to 1 min are most active. Activity decrease after 1 min of reaction. Minimum to no activity was obtained at 10 minutes or longer.
**Figure 16** Results of HPLC profiles of E4A-Tigs : E4A, E4A-ASAP (5 sec), E4A-1min, E4A-2min, E4A-5min, E4A-10min, E4A-30min.
**Figure 17** Results of Activity order: M, N, O, P, Q, R, S, T, E4A; M = E4A has no activity.
**Figure 18** Results of MTT cytotoxic activity of E4A-Tig-R in Cancer cells of different organs: A, Bone (U2OS) IC50 = 4.5 ug/ml; B, Bladder (TB9): IC50 = 2.5 ug/ml; C, Lung (H460): IC50 = 4.8 ug/ml; D, Ovary (ES2): IC50 = 2.8 ug/ml
**Figure 19** Results of MTT cytotoxic activity of E4A-Tig-R in Cancer cells of different organs: E, Colon (HCT116) IC50 = 5.2 ug/ml; F, Pancreas (Capan) IC50 = 2.4 ug/ml; G, Ovary (OVCAR3) IC50 = 5.8 ug/ml; H, Breast (MCF-7) IC50 = 4.5 ug/ml
**Figure 20** Results of MTT cytotoxic activity of E4A-Tig-R in Cancer cells of different organs: I, Prostate (DU145) IC50 = 3.6 ug/ml;J, Skin (SK-Mel-5) IC 50 = 5.1 ug/ml; K, Mouth (KB) IC 50 = 3 ug/ml; L, Kidney (A498) IC 50 = 3.5 ug/ml
**Figure 21** Results of MTT cytotoxic activity of E4A-Tig-R in Cancer cells of different organs: M, Liver (HepG2) IC50 = 6 ug/ml; N, Brain (T98G) IC50 = 8 ug/ml; P, Leukemia (K562) IC 50 = 2 ug/ml; Q, Cervix (HeLa) IC 50 = 5 ug/ml

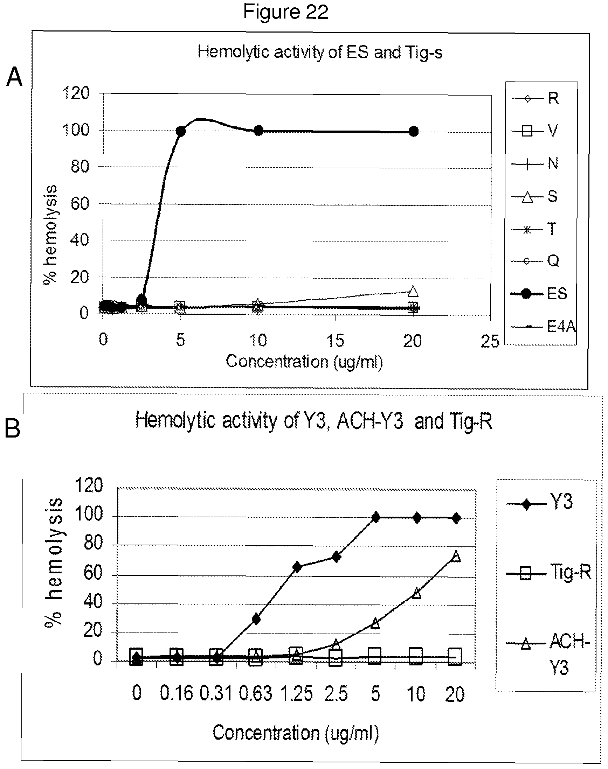

**Figure 22**(A) Results: Tig-N, -Q, -R, -T -S and -V do not have hemolytic activity up to 20 ug/ml. The original compound ES lyse 100% red blood cells (RBC) at 5 ug/ml. (B) Results: compare to Y3, the ACH-Y3 is less potent in hemolytic activity. Tig-R has no hemolytic activity
**Figure 23**(A) Results of HPLC profiles of reaction products. Multiple fractions were obtained. Individual fractions were collected for further studies. (B) Results of purification of E4A-Tig-R.
**Figure 24** Results of MTT assay of E4A-Tig-R with bone U2OS cell
**Figure 25** Results of HNMR of E4A-Tig-R.
**Figure 26** Results of CNMR of E4A-Tig-R.
**Figure 27** Results of HMQC of E4A-Tig-R.
**Figure 28** Results of HMBC of E4A-Tig-R.
**Figure 29** The Mass spectrum of Tig-R (M+H) is 671.4509. The mass is consistent with the proposed structure
**Figure 30** The Chemical Structure of E4A-Tig-R, 24,28-O-Tigloyl-3β,16α, 21β, 22α, 24β, 28-hexahydroxyolean-12-ene, Formular:C40H62O8, FW : 670.91548
**Figure 31**
   (A) Results of HPLC profiles of reaction products. Multiple fractions were obtained. Individual fractions were collected for further studies.
   (B) Results of purification of E4A-Tig-N. (C) Results of purification of E4A-Tig-S; (D) Results of purification of E4A-Tig-T.

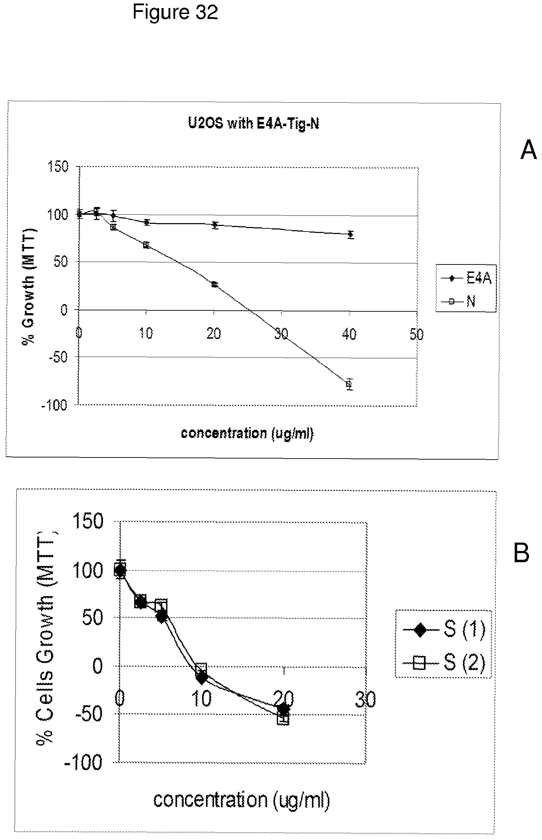

**Figure 32**(A) Results of MTT assay of E4A-Tig-N with bone U2OS cell; (B) Results of MTT assay of E4A-Tig-S with bone U2OS cell

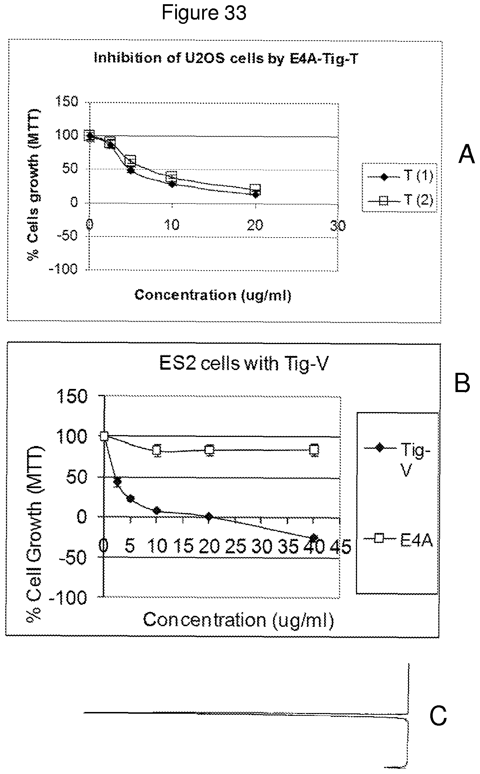

**Figure 33**(A) Results of MTT assay of E4A-Tig-T with bone U2OS cell; (B)shows the results of MTT assay of E4A-Tig-V with bone Ovary ES2 cell. IC50 = 2 ug/ml; (C) shows the results of purification of E4A-Tig-V.

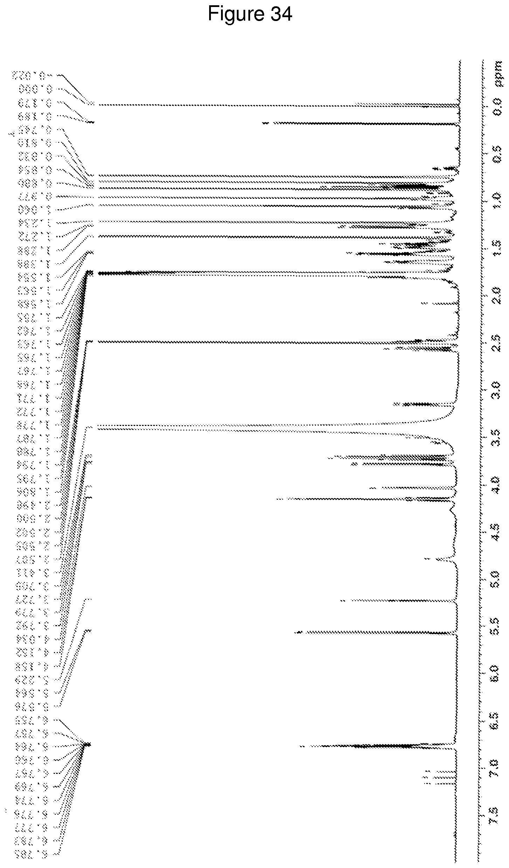

**Figure 34** Results of HNMR of E4A-Tig-V.

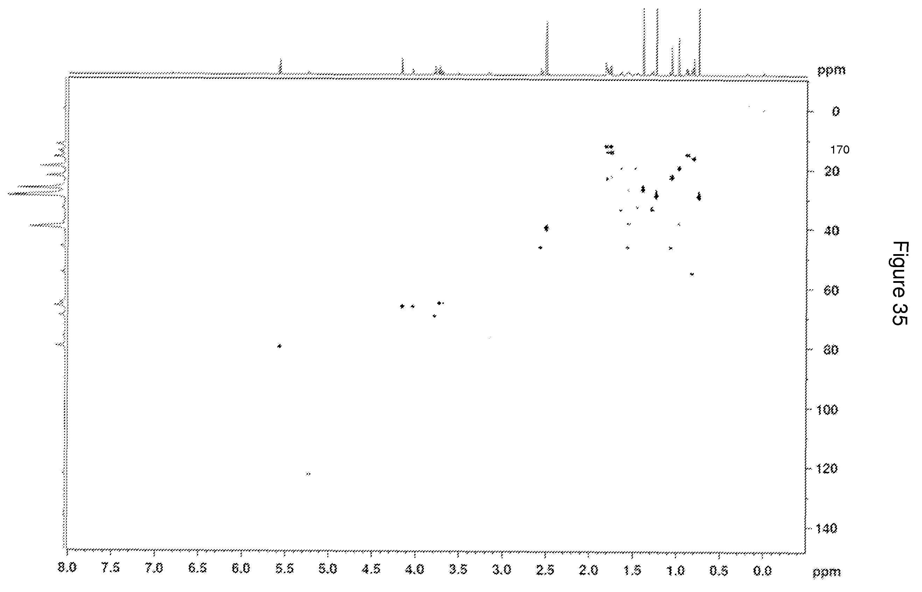

**Figure 35** Results of HMQC of E4A-Tig-V.

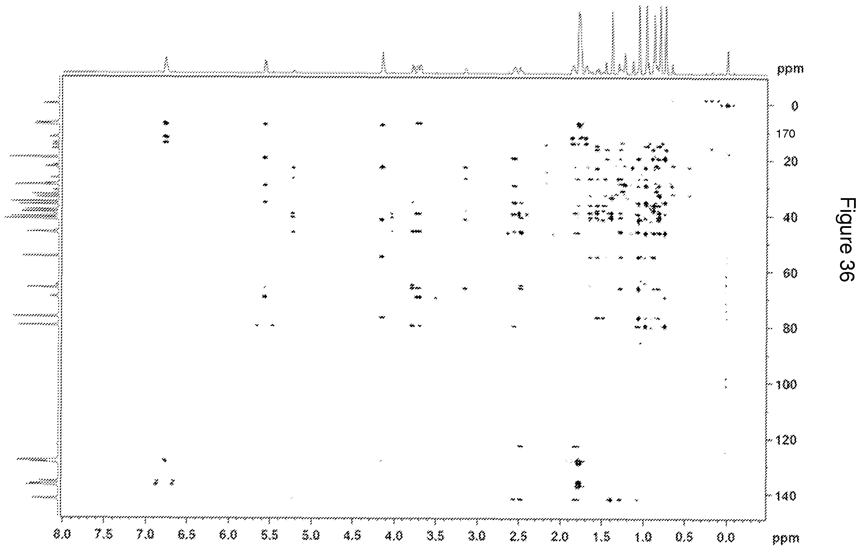

**Figure 36** Results of HMBC of E4A-Tig-V.

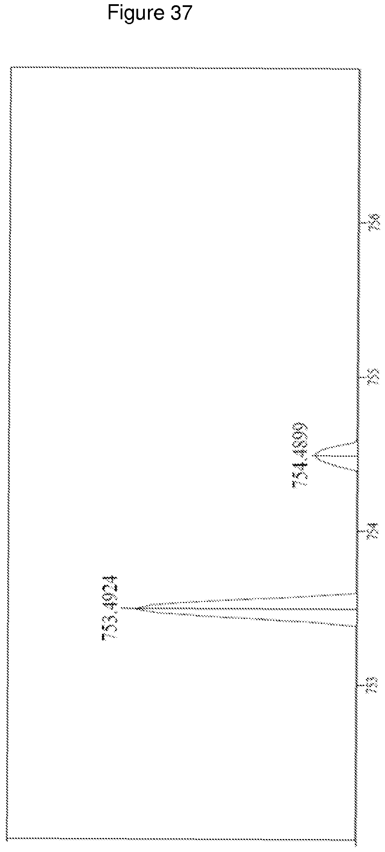

**Figure 37** Results of Mass Spectrum of E4A-Tig-V. The Tig-R (M+H) mass is 753.4924 which is consistent with the proposed formula (C45H68O9).

### DETAILED DESCRIPTION OF THE INVENTION

This invention provides compounds for use in anti adhesion therapy which uses the compound as a mediator or inhibitor of adhesion proteins and angiopoietins. It inhibits excess adhesion and inhibits cell attachment. It modulates angiogenesis. The compounds also use as mediator of cell adhesion receptor.

This invention provides compounds or a composition comprising the compounds provided in the invention for treating cancers; for inhibiting cancer growth, for inhibiting viruses; for preventing cerebral aging; for improving memory; improving cerebral functions; for curing enuresis, frequent micturition, urinary incontinence; dementia, Alzheimer's disease, autism, brain trauma, Parkinson's disease or other diseases caused by cerebral dysfunctions; for treating arthritis, rheumatism, poor circulation, arteriosclerosis, Raynaud's syndrome, angina pectoris, cardiac disorder, coronary heart disease, headache, dizziness, kidney disorder; cerebrovascular diseasea; inhibiting NF-Kappa B activation; for treating brain edema, severe acute respiratory syndrome, respiratory viral diseases, chronic venous insufficiency, hypertension, chronic venous disease, oedema, inflammation, hemonhoids, peripheral edema formation, varicose vein disease, flu, post traumatic edema and postoperative swelling; for inhibiting blood clots, for inhibiting ethanol absorption; for lowering blood sugar; for regulating adrenocorticotropin and corticosterone levels. This invention provides a composition for AntiMS, antianeurysm, antiasthmatic, anti-oedematous, anti-inflammatory, antibradykinic, anticapillarihemorrhagic, anticephalagic, anticervicobrachialgic, antieclamptic, antiedemic, antiencaphalitic, antiepiglottitic, antiexudative, antiflu, antifracture, antigingivitic, antihematomic, antiherpetic, antihistaminic, antihydrathritic, antimeningitic, antioxidant, antiperiodontic, antiphlebitic, antipleuritic, antiraucedo, antirhinitic, antitonsilitic, antiulcer, antivaricose, antivertiginous, cancerostatic, corticosterogenic, diuretic, fungicide, hemolytic, hyaluronidase inhibitor, lymphagogue, natriuretic, pesticide, pituitary stimulant, thymolytic, vasoprotective, inhibiting leishmaniases, modulating adhesion or angiogenesis of cancer cells, antiparasitic; increase the expression of the genes: ANGPT2, DDIT3, LIF and NFKB1Z, and manufacturing an adjuvant composition and venotonic treatment.

This invention provides compounds, compositions and compounds for treating cancer diseases, inhibiting cancer invasion, for inhibiting cancer growth or for inhibiting cancer metastasis, wherein the compounds comprise the structures selected from the formulae specified in the claims, wherein the cancers comprise breast cancer, leukocytic cancer, liver cancer, ovarian cancer, bladder cancer, prostatic cancer, skin cancer, bone cancer, brain cancer, leukemia cancer, lung cancer, colon cancer, CNS cancer, melanoma cancer, renal cancer, cervical cancer, esophageal cancer, testicular cancer, spleenic cancer, kidney cancer, lymphhatic cancer, pancreatic cancer, stomach cancer and thyroid cancer; wherein the cells comprise breast cell, leukocytic cell, liver cell, ovarian cell, bladder cell, prostatic cell, skin cell, bone cell, brain cell, leukemia cell, lung cell, colon cell, CNS cell, melanoma cell, renal cell, cervical cell, esophageal cell, testicular cell, spleenic cell, kidney cell, lymphhatic cell, pancreatic cell, stomach cell and thyroid cell.

This invention shows that the presence of Tigloyl, angeloyl, Crotonoyl, senecioyl, Cinnamoyl, Pentenoyl, Hexanoyl, benzoyl, Ethylbutyryl, at a pentacyclic triterpene, triterpene, triterpeniod, triterpeniod saponin or compound selected from formulae as specified in the claims, produces inhbition of cancer growth, cancer invasion, cells invasion, cancer cell invasion, cell adhesion, cell circulation or cell attachment.

This invention shows that the presence of Tigloyl, angeloyl, Crotonoyl, senecioyl, Cinnamoyl, Pentenoyl, Hexanoyl, benzoyl, Ethylbutyryl, in the compounds of the formulae as specified in the claims, produces inhibition of cancer growth, cancer invasion, cells invasion or cancer cell invasion. In an embodiment, the presence of group(s) selected from Tigloyl, angeloyl, Crotonoyl, senecioyl, Cinnamoyl, Pentenoyl, Hexanoyl, benzoyl, Ethylbutyryl, in the compounds of the formulae as specified in the claims, produces activities including inhibition of cancer growth, cancer invasion, cells invasion, cancer cell invasion, cell adhesion, cell attachment or cell circulating.

There is disclosed herein a method of synthesizing active compound of the formulae as specified in the claims, by attaching functional group to a core compound, wherein the functional group(s) is/are selected from tigloyl, angeloyl, crotonoyl, senecioyl, cinnamoyl, pentenoyl, hexanoyl, benzoyl, ethylbutyryl, wherein the core compound is a 5 ring triterpene. The compounds provided in the invention are for treating cancers, inhibition of cancer growth, cancer invasion, cells invasion, cancer cell invasion; cell adhesion, cell attachment, cell circulating; for inhibiting viruses; for preventing cerebral aging; for improving memory; improving cerebral functions; for curing enuresis, frequent micturition, urinary incontinence; dementia, Alzheimer's disease, autism, brain trauma, Parkinson's disease or other diseases caused by cerebral dysfunctions; for treating arthritis, rheumatism, poor circulation, arteriosclerosis, Raynaud's syndrome, angina pectoris, cardiac disorder, coronary heart disease, headache, dizziness, kidney disorder; cerebrovascular diseasea; inhibiting NF-Kappa B activation; for treating brain edema, severe acute respiratory syndrome, respiratory viral diseases, chronic venous insufficiency, hypertension, chronic venous disease, oedema, inflammation, hemonhoids, peripheral edema formation, varicose vein disease, flu, post traumatic edema and postoperative swelling; for inhibiting blood clots, for inhibiting ethanol absorption; for lowering blood sugar; for regulating adrenocorticotropin and corticosterone levels. This invention provides a composition for AntiMS, antianeurysm, antiasthmatic, anti-oedematous, anti-inflammatory, antibradykinic, anticapillarihemorrhagic, anticephalagic, anticervicobrachialgic, antieclamptic, antiedemic, antiencaphalitic, antiepiglottitic, antiexudative, antiflu, antifracture, antigingivitic, antihematomic, antiherpetic, antihistaminic, antihydrathritic, antimeningitic, antioxidant, antiperiodontic, antiphlebitic, antipleuritic, antiraucedo, antirhinitic, antitonsilitic, antiulcer, antivaricose, antivertiginous, cancerostatic, corticosterogenic, diuretic, fungicide, hemolytic, hyaluronidase inhibitor, lymphagogue, natriuretic, pesticide, pituitary stimulant, thymolytic, vasoprotective, inhibiting leishmaniases, modulating adhesion or angiogenesis of cells, antiparasitic; increase the expression of the genes: ANGPT2, DDIT3, LIF and NFKB1Z, and manufacturing an adjuvant composition and venotonic treatment.

Experiments presented in this disclosure showed that the compound AKOH has no effect in inhibiting cancer growth, cancer invasion, cells invasion or cancer cell invasion. AKOH was obtained by removing the angeloyl groups from carbon positions 21 and 22 of the active Xanifolia Y(Y3). This invention shows that the ability for inhibiting cancer invasion, cells invasion or cancer cell invasion of Xanifolia Y(Y3) are lost by removing angeloyl groups from carbon positions 21 and 22.

Experiments presented in this disclosure showed that the core compound including E4A, E5A, Xanifolia Y-core have no effect in inhibiting cancer growth, cancer invasion, cells invasion or cancer cell invasion. Xanifolia Y-core was obtained by removing the angeloyl groups from carbon positions 21 and 22, and the sugar moieties from carbon 3 of the active Xanifolia Y(Y3). E4A (E IV A) was obtained by removing the groups from carbon positions 3, 21 and 22 of the active Escin. E5A (E V A) was obtained by removing the groups from carbon positions 3, 21 and 22 of the active Escin.

This disclosure showed that the core compound including E4A, E5A, Xanifolia Y-core and AKOH have no hemolytic activity and anti cancer activity.

This disclosure showed that Tig-N, Tig -Q, Tig -R, Tig-T Tig-S and Tig-V do not have hemolytic activity up to 20 ug/ml. The original compound ES lyse 100% red blood cells (RBC) at 5 ug/ml. Compare to Y3, the ACH-Y3 is less potent in hemolytic activity. Tig-R has no hemolytic activity. This disclosure showed that Tig-N, Tig -Q, Tig -R, Tig-T Tig-S and Tig-V have anti cancer activities.

This disclosure shows that the ability for inhibiting cancer growth, cancer invasion, cells invasion or cancer cell invasion are maintained when the sugar moieties are removed from carbon position 3 of an active compound, triterpene, triterpeniod, or triterpeniod saponin. Experiments presented in this disclosure showed that the compound ACH-Y3 has the ability to inhibit cancer invasion, cells invasion or cancer cell invasion. The compound ACH-Y3 was obtained by removing the sugar moieties from carbon position 3 of a active Xanifolia Y(Y3). This disclosure shows that the ability for inhibiting cancer invasion, cells invasion or cancer cell invasion are maintained when the sugar moieties are removed from the carbon position 3 of active Xanifolia Y(Y3).

A compound which has bio-activities including inhibiting cancer growth, inhibiting cancer invasion, cells invasion or cancer cell invasion is called active compound.

This invention provides compounds for treating cancers, inhibition of cancer growth, cancer invasion, cells invasion, cancer cell invasion; cell adhesion, cell attachment, cell circulating, or for inhibiting cancer metastasis, wherein the compounds comprise the structures selected from the formulae as specified in the claims, wherein the compounds can be synthesized or isolated, wherein the cells comprise cancer cells, wherein the cancers comprise breast cancer, leukocytic cancer, liver cancer, ovarian cancer, bladder cancer, prostatic cancer, skin cancer, bone cancer, brain cancer, leukemia cancer, lung cancer, colon cancer, CNS cancer, melanoma cancer, renal cancer, cervical cancer, esophageal cancer, testicular cancer, spleenic cancer, kidney cancer, lymphhatic cancer, pancreatic cancer, stomach cancer and thyroid cancer.. The method of inhibiting cancer invasion, cells invasion or cancer cell invasion activities uses non-cytotoxic drug concentrations. The method of inhibiting metastasis uses non-cytotoxic drug concentrations. There is no noticeable change in cell morphology.

This invention provides the compounds of the formulae as specified in the claims, for treating cancers, inhibition of cancer growth, cancer invasion, cells invasion, cancer cell invasion; cell adhesion, cell attachment, cell circulating, migration, metastasis or growth of cancers, wherein the methods comprise affecting gene expression, wherein the methods comprise stimulating gene expression, or wherein the methods comprise inhibiting the gene expression, or wherein the methods comprise administering to a subject an effective amount of compounds, compositions in this application.

In vitro studies show that a compound selected from structure (2A) or (K) inhibits cell adhesion to culture flasks. The compound blocks the function of these adhesive molecules on cells. In an embodiment, the selected compound blocks the function of these adhesive molecules on cells. In an embodiment, the selected compound blocks the function of these adhesive molecules on carcinoma cells. In an embodiment, the selected compound blocks the function of these adhesive molecules on the mesothelial cells. This invention provides the compounds as specified in the claims, for use in an anti adhesion therapy which uses the compound as a mediator or inhibitor of adhesion proteins and angiopoietins. It inhibits excess adhesion and inhibits cell attachment. This invention provides the claimed compounds for use as a mediator for cell circulating, cell moving and inflammatory diseases. In an embodiment, the selected compound binds to the adhesive proteins (by masking) on the membrane and inhibits the interaction of adhesion proteins with their receptors. In an embodiment, the selected compound's action on the membrane affects adhesion proteins' function in the membrane. The lost of adhesion activity of cancer cells is result from direct or indirect action of the selected compound on membrane proteins.

(Our purification methods and biological assays include the MTT assay in International Application No. PCT/US05/31900, filed September 7, 2005, U.S. Serial No. 11/289142, filed November 28, 2005, and U.S. Serial No. 11/131551, filed May 17, 2005, and PCT/US2008/002086, 1188-ALA-PCT, filed February 15, 2008, the cell invasion experiments methods in International Application PCT/US2010/0042240, filed July 16, 2010.

This invention provides the compounds as specified in the claims *per se* and also for use in methods for inhibiting cancer invasion, cell invasion, cancer cell invasion, migration, metastasis or growth of cancers, wherein this invention comprises a process and method for administration of the composition, wherein administration is by intravenous injection, intravenous drip, intraperitoneal injection or oral administration; wherein administration is by intravenous drip: 0.003-0.03mg/kg body weight of compound dissolved in 250ml of 10% glucose solution or in 250ml of 0.9% NaCl solution, or by intravenous injection: 0.003-0.03mg/kg body weight per day of compound dissolved in 10-20ml of 10% glucose solution or of 0.9% NaCl solution, or 0.01-0.03mg/kg body weight of compound dissolved in 250ml of 10% glucose solution or in 250ml of 0.9% NaCl solution, or by intravenous injection: 0.01-0.03mg/kg body weight per day of compound dissolved in 10-20ml of 10% glucose solution or of 0.9% NaCl solution, or 0.01-0.05mg/kg body weight of compound dissolved in 250ml of 10% glucose solution or in 250ml of 0.9% NaCl solution, or by intravenous injection: 0.01-0.05mg/kg body weight per day of compound dissolved in 10-20ml of 10% glucose solution or of 0.9% NaCl solution, or 0.05-0.2mg/kg body weight of compound dissolved in 250ml of 10% glucose solution or in 250ml of 0.9% NaCl solution, or by intravenous injection: 0.05-0.2mg/kg body weight per day of compound dissolved in 10-20ml of 10% glucose solution or of 0.9% NaCl solution, or by intravenous drip: 0.1-0.2mg/kg body weight per day of compound dissolved in 250ml of 10% glucose solution or in 250ml of 0.9% NaCl solution, or by intravenous injection: 0.1-0.2mg/kg body weight per day compound dissolved in 10-20ml of 10% glucose solution or of 0.9% NaCl solution, or by intraperitoneal injection(I.P.): 2.5mg/kg body weight per day compound dissolved in 10% glucose solution or of 0.9% NaCl solution, or by oral administration wherein the dosage of mammal is 1-10mg/kg, 10-30mg/kg, 30-60mg/kg, or 60-90mg/kg body weight of compound, or by intravenous injection or intravenous drip wherein the dosage of mammal is 0.01- 0.1mg/kg body weight , 0.1-0.2mg/kg, 0.2 - 0.4mg/kg body weight, or 0.4 - 0.6 mg/kg body weight of compound, or by intraperitoneal injection (I.P.) wherein the dosage of mammal is 1-3mg/kg, 3-5mg/kg, 4-6mg/kg, or 6-10mg/kg body weight of compound.

This invention provides the compounds as specified in the claims per se and also for use in methods for treating cancers, inhibition of cancer growth, cancer invasion, cells invasion, cancer cell invasion; cell adhesion, cell attachment, cell circulating, migration, metastasis or growth of cancers, wherein the invention comprises a pharmaceutical composition comprising the compound of this invention or a pharmaceutically acceptable salt thereof, and a pharmaceutically acceptable carrier or diluent, wherein said compound is present in a concentration of 0.01 ug/ml to 65ug/ml, or wherein said compound is present in a concentration of 0.01 ug/ml to 40ug/ml, or wherein said compound is present in a concentration of 0.01 ug/ml to 30ug/ml, or wherein said compound is present in a concentration of 0.01ug/ml to 10ug/ml, or wherein said compound is present in a concentration of 0.01ug/ml to 5ug/ml, or wherein said compound is present in a concentration of 5ug/ml to 10ug/ml, or wherein said compound is present in a concentration of 0.1 ug/ml to 5ug/ml, or wherein said compound is present in a concentration of 0.1ug/ml to 7.5ug/ml, or wherein said compound is present in a concentration of 0.1ug/ml to 10ug/ml, or wherein said compound is present in a concentration of 0.1ug/ml to 15ug/ml, or wherein said compound is present in a concentration of 0.1ug/ml to 20ug/ml, or wherein said compound is present in a concentration of 0.1ug/ml to 30ug/ml, or wherein said compound is present in a concentration of 1 ug/ml to 5ug/ml, or wherein said compound is present in a concentration of 1ug/ml to 7.5ug/ml, or wherein said compound is present in a concentration of 1ug/ml to 10ug/ml, or wherein said compound is present in a concentration of 1ug/ml to 15ug/ml, or wherein said compound is present in a concentration of 1ug/ml to 20ug/ml, or wherein said compound is present in a concentration of 1ug/ml to 30ug/ml, or wherein said compound is present in a concentration of 3ug/ml to 5ug/ml, or wherein said compound is present in a concentration of 3ug/ml to 7.5ug/ml, or wherein said compound is present in a concentration of 3ug/ml to 10ug/ml, or wherein said compound is present in a concentration of 3ug/ml to 15ug/ml, or wherein said compound is present in a concentration of 3ug/ml to 20ug/ml, or wherein said compound is present in a concentration of 3ug/ml to 30ug/ml, or wherein said compound is present in a concentration of 4ug/ml to 5ug/ml, or wherein said compound is present in a concentration of 4ug/ml to 7.5ug/ml, or wherein said compound is present in a concentration of 4ug/ml to 10ug/ml, or wherein said compound is present in a concentration of 4ug/ml to 15ug/ml, or wherein said compound is present in a concentration of 4ug/ml to 20ug/ml, or wherein said compound is present in a concentration of 4ug/ml to 30ug/ml, or wherein said compound is present in a concentration of 5ug/ml to 8ug/ml, or wherein said compound is present in a concentration of 5ug/ml to 9ug/ml, or wherein said compound is present in a concentration of 5ug/ml to 10ug/ml, or wherein said compound is present in a concentration of 5ug/ml to 15ug/ml, or wherein said compound is present in a concentration of 5ug/ml to 20ug/ml, or wherein said compound is present in a concentration of 5ug/ml to 30ug/ml, or wherein said compound is present in a concentration of 7ug/ml to 8ug/ml, or wherein said compound is present in a concentration of 7ug/ml to 9ug/ml, or wherein said compound is present in a concentration of 7ug/ml to 10ug/ml, or wherein said compound is present in a concentration of 7ug/ml to 15ug/ml, or wherein said compound is present in a concentration of 7ug/ml to 20ug/ml, or wherein said compound is present in a concentration of 7ug/ml to 30ug/ml.

This invention provides the compounds as specified in the claims per se and also for use in methods for treating cancers, inhibition of cancer growth, cancer invasion, cells invasion, cancer cell invasion; cell adhesion, cell attachment, cell circulating, migration, metastasis or growth of cancers, wherein the invention comprises a pharmaceutical composition comprising the compound of this invention or a pharmaceutically acceptable salt thereof, and a pharmaceutically acceptable carrier or diluent, wherein said compound is present in a concentration of 0.008uM to 80uM, or wherein said compound is present in a concentration of 0.01uM to 60uM, or wherein said compound is present in a concentration of 0.01uM to 50uM, or wherein said compound is present in a concentration of 0.01uM to 40uM, or wherein said compound is present in a concentration of 0.01uM to 30uM, or wherein said compound is present in a concentration of 0.01uM to 20uM, or wherein said compound is present in a concentration of 0.01uM to 10uM, or wherein said compound is present in a concentration of 5uM to 10uM, or wherein said compound is present in a concentration of 0.1uM to 5uM, or wherein said compound is present in a concentration of 0.1uM to 7.5uM, or wherein said compound is present in a concentration of 0.1uM to 10uM, or wherein said compound is present in a concentration of 0.1uM to 15uM, or wherein said compound is present in a concentration of 0.1uM to 20uM, or wherein said compound is present in a concentration of 0.1uM to 30uM or wherein said compound is present in a concentration of 0.1uM to 40uM, or wherein said compound is present in a concentration of 0.1uM to 50uM or wherein said compound is present in a concentration of 0.1uM to 60uM, or wherein said compound is present in a concentration of 0.1uM to 80uM, or wherein said compound is present in a concentration of 1uM to 5uM, or wherein said compound is present in a concentration of 1uM to 7.5uM, or wherein said compound is present in a concentration of 1uM to 10uM, or wherein said compound is present in a concentration of 1uM to 15uM, or wherein said compound is present in a concentration of 1uM to 20uM, or wherein said compound is present in a concentration of 1uM to 30uM or wherein said compound is present in a concentration of 1uM to 40uM, or wherein said compound is present in a concentration of 1uM to 50uM or wherein said compound is present in a concentration of 1uM to 60uM, or wherein said compound is present in a concentration of 1uM to 80uM, or wherein said compound is present in a concentration of 3uM to 5uM, or wherein said compound is present in a concentration of 3uM to 7.5uM, or wherein said compound is present in a concentration of 3uM to 10uM, or wherein said compound is present in a concentration of 3uM to 15uM, or wherein said compound is present in a concentration of 3uM to 20uM, or wherein said compound is present in a concentration of 3uM to 30uM or wherein said compound is present in a concentration of 3uM to 40uM, or wherein said compound is present in a concentration of 3 uM to 50uM or wherein said compound is present in a concentration of 3 uM to 60uM, or wherein said compound is present in a concentration of 3uM to 80uM, or wherein said compound is present in a concentration of 5uM to 8uM, or wherein said compound is present in a concentration of 5uM to 10uM, or wherein said compound is present in a concentration of 5uM to 15uM, or wherein said compound is present in a concentration of 5uM to 20uM, or wherein said compound is present in a concentration of 5uM to 30uM or wherein said compound is present in a concentration of 5uM to 40uM, or wherein said compound is present in a concentration of 5uM to 50uM or wherein said compound is present in a concentration of 5uM to 60uM, or wherein said compound is present in a concentration of 5uM to 80uM. or wherein said compound is present in a concentration of 7uM to 8uM, or wherein said compound is present in a concentration of 7uM to 10uM, or wherein said compound is present in a concentration of 7uM to 15uM, or wherein said compound is present in a concentration of 7uM to 20uM, or wherein said compound is present in a concentration of 7uM to 30uM or wherein said compound is present in a concentration of 7uM to 40uM, or wherein said compound is present in a concentration of 7uM to 50uM or wherein said compound is present in a concentration of 7uM to 60uM, or wherein said compound is present in a concentration of 7uM to 80uM.

It is to be noted that the transitional term "comprising", which is synonymous with "including", "containing" or "characterized by", is inclusive or open-ended and does not exclude additional, un-recited elements or method steps.

In the following examples, the compounds B4-B7, B29-B32, C4-C7, C29-C32, D4-D7, D29-D32, E4-E7, E29-E32, G4-G7, G29-G32, H29-H32, 14-17, 129-132, J4-J7, J29-J32, K4-K7, K29-K32,Tig-Sen-2 to Tig-Sen-6, Tig-Cro-2 to Tig-Cro-6, Tig-Acy-2 to Tig-Acy-6, Tig-Pen-2 to Tig-Pen-6, Tig-Hex-2 to Tig-Hex-6, Tig-Cin-2 to Tig-Cin-6, Tig-Ang-2 to Tig-Ang-6 and Tig-Eth-2 to Tig-Eth-6 are the compounds of the present invention. All other compounds appearing in the examples are present for the purposes of comparison.

### Example 1

**Tablet for dose containing 10mg, 20mg 30mg of active compound**

| | | | | | |
|---|---|---|---|---|---|
| Active compound | 1mg | 5mg | 10mg | 20mg | 30mg |
| Microcrystalline cellulose | 20mg | 20mg | 19.75mg | 60mg | 100mg |
| Corn starch | 29mg | 24.5mg | 19.75mg | 19.25mg | 18.5mg |
| Magnesium stearate | 0mg | 0.5mg | 0.5mg | 0.75mg | 1.5mg |

The active compound, cellulose, and a portion of the corn starch are mixed and granulated to 10% corn starch paste. The resulting granulation is sieved, dried and blended with the remainder of the corn starch and the magnesium stearate. The resulting granulation is then compressed into tablets containing 1, 5, 10, 20, 30mg, respectively of active ingredient per tablet.

### Example 2

### Intravenous solution preparation

An intravenous dosage form of the active compound is prepared as follows:
Active compound 1-10ug
Sodium citrate 5-50 mg
Citric acid 1-15 mg
Sodium chloride 1-8 mg
Water for injection (USP) q.s. to 1mL

Utilizing the above quantities, the active compound is dissolved at room temperature in a prepared solution of sodium chloride, citric acid, and sodium citrate in water for injection.

### Example 3

### Intravenous drip preparation

0.25-2.5mg compound dissolved in 250ml of 10% glucose solution or in 250ml of 0.9% NaCl solution.

Intravenous drip preparation: 1-2.mg compound dissolved in 250ml of 10% glucose solution or in 250ml of 0.9% NaCl solution

Treatment of angelic acid with one of the many standard chlorinating reagents including phosphorus ocychloride, phosphorus trichloride and thionyl chloride produces tigloyl chloride. Oxalyl chloride produces a 2:1 ratio of angeloyl chloride to tigloyl chloride. Treatment of potassium salt in diethyl ether with oxalyl chloride and catalytic DMF for 2 hr at 0C produces pure angeloyl chloride.

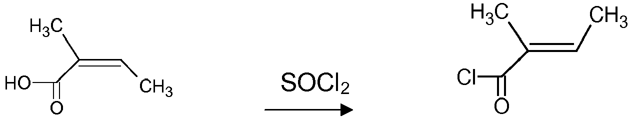

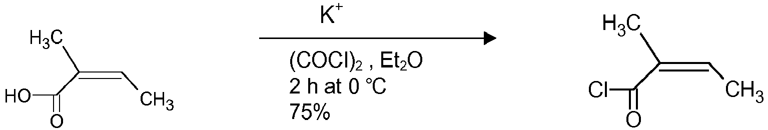

### Acid Hydrolysis of the following compounds:

### a) Xanifolia(Y),

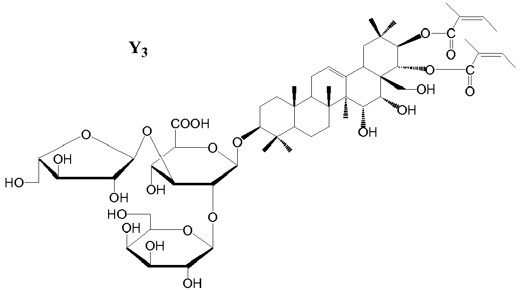

or chemical name: 3-O-[β-D-galactopyranosyl (1→2)]-α-L-arabinofuranosy (1→3)-β-D-glucuronopyranosyl-21,22-O-diangeloyl-3β, 15α, 16α, 21β, 22α, 28-hexahydroxyolean-12-ene;

### c) Xanifolia (Y2),

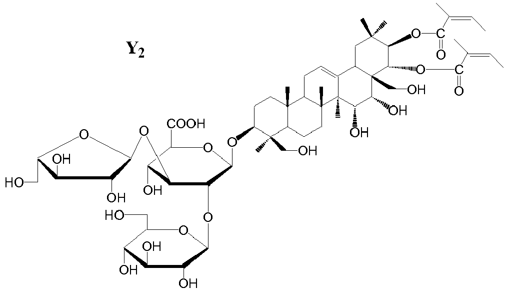

or chemical name: 3-O-[β-D-glucopyranosyl-(1→2)]-α-L-arabinofuranosy (1→3)-β-D-glucuronopyranosyl-21,22-O-diangeloyl-3β, 15α, 16α, 21β, 22α, 24β, 28-heptahydroxyolean-12-ene;

### d) Xanifolia (Y8),

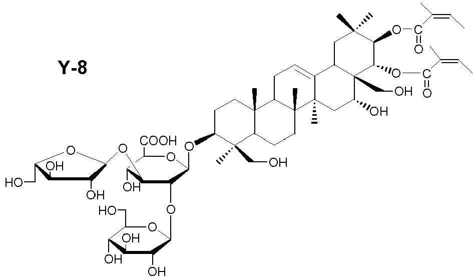

or chemical name: 3-O-[*β*-glucopyranosyl (1→2)]-*α*-arabinofuranosyl (1→3)-*β*-glucuronopyranosyl-21, 22-O-diangeloyl-3*β*, 16*α*, 21*β*, 22*α*, 24*β*, 28-hexahydroxyolean-12-ene;

### f) Xanifolia (Y10),

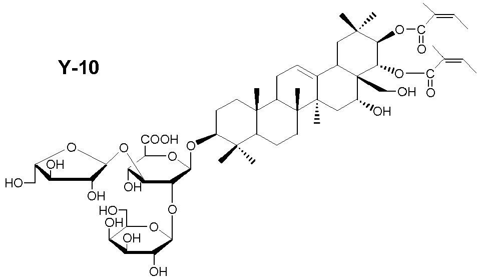

or chemical name: 3-O-[*β*-galactopyranosyl (1→2)]-*α*-arabinofuranosyl (1→3)-*β*-glucuronopyranosyl-21, 22-O-diangeloyl-3*β*, 16*α*, 21*β*, 22*α*, 28-pentahydroxyolean-12-ene.

### j) structure (M10)

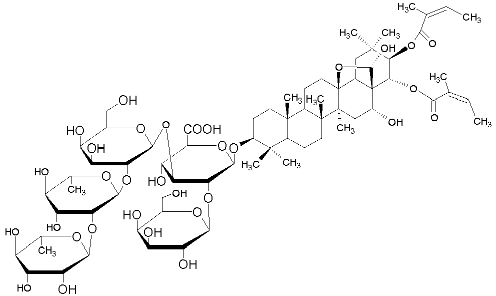

### m) structure (bES):

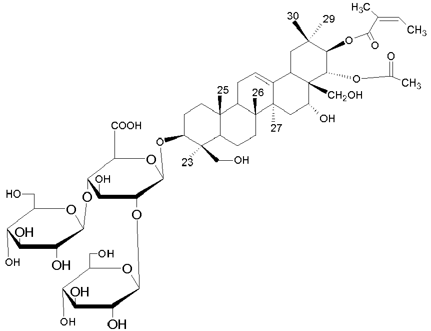

After acid hydrolysis of the above, an isolated, purified or synthesized compound is produced having a structure (ACH) selected from following:

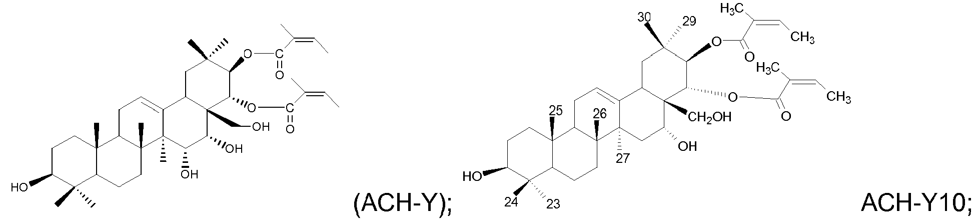

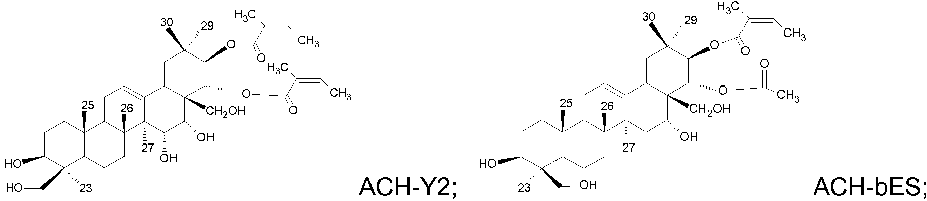

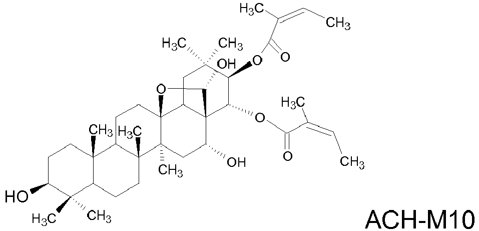

The composition comprises bioactive compounds from natural plants or synthesis. The program is based on our purification methods and biological assays including the MTT assay. See International Application No. PCT/US05/31900, filed September 7, 2005, U.S. Serial No. 11/289142, filed November 28, 2005, and U.S. Serial No. 11/131551, filed May 17, 2005, and PCT/US2008/002086, 1188-ALA-PCT, filed February 15, 2008, 12/344,682, 1020-B1-US, filed December 29, 2008. The details of Analysis of gene expression of ES2 cells after Y-treatment by Microarray, Data Analysis Methods and Western blot in PCT/US2008/002086, 1188-ALA-PCT, filed February 15, 2008, and the cell invasion experiments methods in International Application PCT/US2010/0042240, filed July 16, 2010.

### The haemolytic assay

Erythrocytes (RBC) were isolated from human blood (EDTA whole blood, collected randomly). 50ul of the 10% RBC suspension (in PBS) was added to 2 ml of sample solutions (concentration range from 0.1 ug/ml to 400 ug/ml) in PBS. The mixture was vortexed briefly and sat for 60 min at room temperature. The mixture was spun at 3K for 10 min and the relative amounts of lysed hemoglobin in the supernatant were measured at 540 nm. The synthetic compounds of present application were tested with this method.

### Acid Hydrolysis of Saponin

15mg Xanifolia-Y was dissolved in 1 ml of methanol. 1ml of 2N HCl was then added. The mixture was refluxed in 80C water bath for 5 hours. The solution was then neutralized by adding 2ml of 1N NaOH (to final pH 4-6). The aglycone was then extracted with ethylacetate 3ml x 2. The extracts were collected and pooled. Further isolation of aglycone (ACH-Y) was achieved by HPLC with isocratic elution of 80 -100% acetonitrile. Repeating the experiment with compounds Z4, Y10, Y2, Y8, Y7, Y0, X, M10 and ESCIN(bES) gives the following compounds respectively: ACH-Z4, ACH-Y10, ACH-Y2, ACH-Y8, ACH-Y7, ACH-Y0, ACH-X, ACH-E, ACH-Z5, ACH-M10 and ACH-bES. Experiments methods in International Application PCT/US2010/0042240, filed July 16, 2010.

### Removal of the acyl group by alkaline hydrolysis

20mg of Xanifolia-Y was dissolved in 0.5ml of 1N NaOH. The solution was incubated in 80C water bath for 4 hours. It was cooled to room temperature before being neutralized with 0.5ml 1N HCl (adjust pH to about 3). The mixture was extracted with 2ml 1-butanol 3 times. The butanol fractions were collected and lyophilized. The hydrolyzed saponin was further purified with HPLC in a C-18 column eluted with 25% acetonitrile.

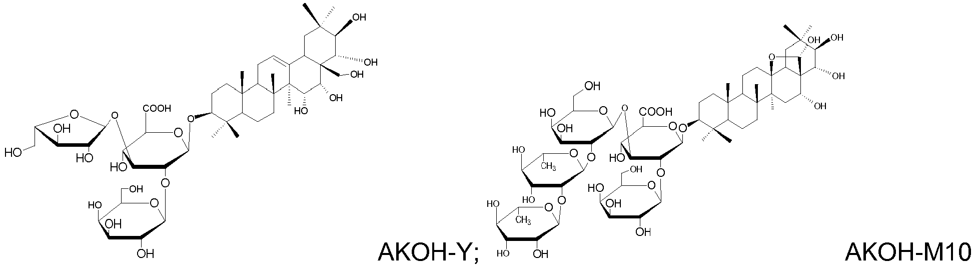

Compounds AKOH-Y and AKOH-M10 do not show the ability to inhibit cancer growth, cancer invasion, cells invasion or cancer cell invasion.

### Core compound

A core compound or pentacyclic triterpenes, hydroxylated triterpenes is obtained by acid and alkaline hydroysis of saponin from natural sources. A pentacyclic triterpene can also be obtained by synthetic methods. A method for synthesizing the core compound is as follows:
Beta-Escin, compound Y, Y10, Y2, Y8, Y7, Y0, X, or M10 dissolved in 1M NaOH (20 mg/ml) was incubated at 70C for 5 hours. The hydrolyzed solution was neutralized with HCl and the water was evaporated by lyophilization. The product was dissolved in 50% methanol and 1N HCl. The mixture was incubated at 70C for 5 hours. The solution was neutralized with NaOH. The hydrolyzed product was extracted with ethylacetate, which was subsequently removed by evaporation. Further purification of the hydrolyzed product of core compounds including (E4A) were archived with FPLC chromatography in a C18 column equilibrated with 70% acetonitrile/TFA at the flow rate of 1 ml/min. The core compounds are obtained.

The core compounds do not show the ability to inhibit cancer growth, cancer invasion, or cell adhesion.

The structures of core compounds: 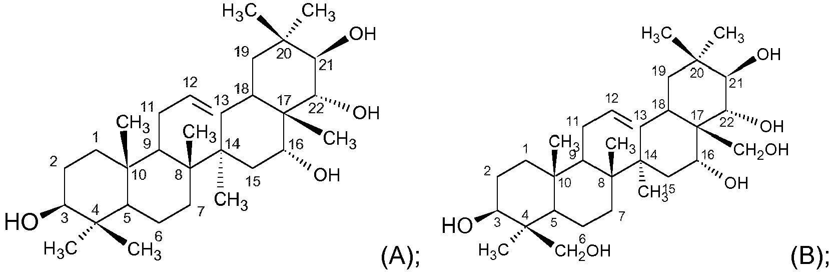 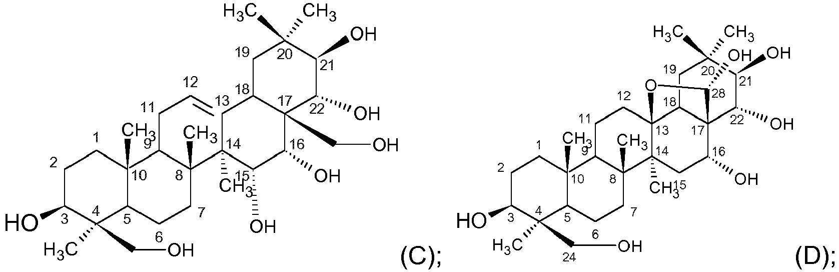 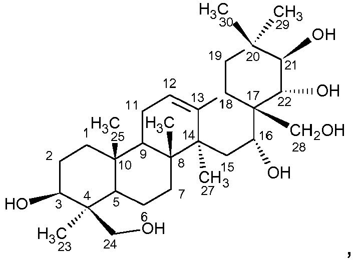 also named as bES-core, , E IV A, ES4A, E4A or (E); 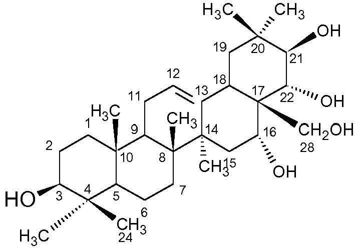 also named as ES V, E5A or (F) 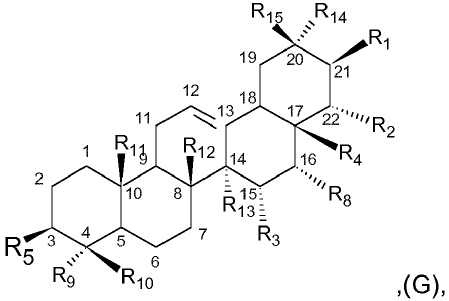 wherein R1, R2, R5, R8 represent OH; R3 represents OH, H or absent; R4, R10 represent CH3 or CH2OH; R9, R11, R12, R13, R14, R15 represent CH3; 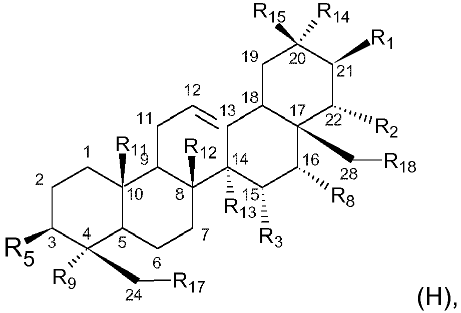 wherein R1, R2, R5, R8, R17, R18 represent OH; R3 represents OH, H or absent; R9, R11, R12, R13, R14, R15 represent CH3.

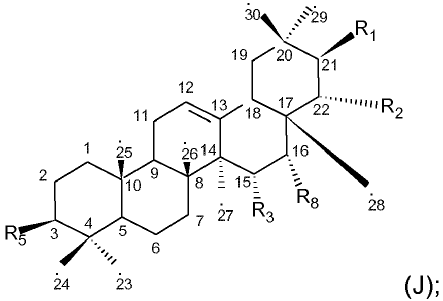

A typical numbering 1 to 30 of carbon positions of a pentacyclic triterpene. 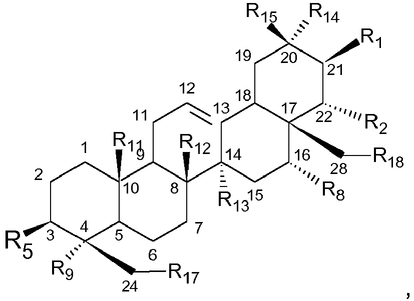 wherein R1, R2, R5, R8, R17, R18 represent OH; R9, R11, R12, R13, R14, R15 represent CH3, also named E4A or (E).

This invention provides a method of synthesizing new active compounds. A method of attaching functional groups to the core compounds [including but not limited to (A), (B), (C), (D), (E), (F), (G), (H)] involves esterification of core compounds with acyl chloride including but not limited to Tigloyl chloride, angeloyl chloride, Acetyl chloride, Crotonoyl chloride, 3,3-Dimethylartyloyl chloride, senecioyl chloride, Cinnamoyl chloride, Pentenoyl chloride, Hexanoyl chloride, benzoyl chloride or Ethylbutyryl chloride for 5 sec, 1 min, 2 min, 5 min, 10 min, 30 min, 1hr, 2 hr, 18 hr, 2 days or 3 days at 0C, 25C or 75C temperature. At the end of reaction, 5 ml of 2N HCl or 1M NaHCO3 is added to the reaction mixture. The solution is then extracted 3 times with 10 ml of ethyl acetate which is then evaporated under vacuum and at 45C and lyophilization. The reaction product is dissolved in 80% acetonitrile - 0.005% Trifluoroacetic acid. The active esterification products are purified with HPLC. MTT activity was performed to test the activity of acyl chloride, solution after the reaction, individual fractions, and individual compounds. The core compounds are synthetic, semi synthetic or from natural source. The core compounds are including terpene, isoprene, triterpenes, and hydroxylated triterpenes.

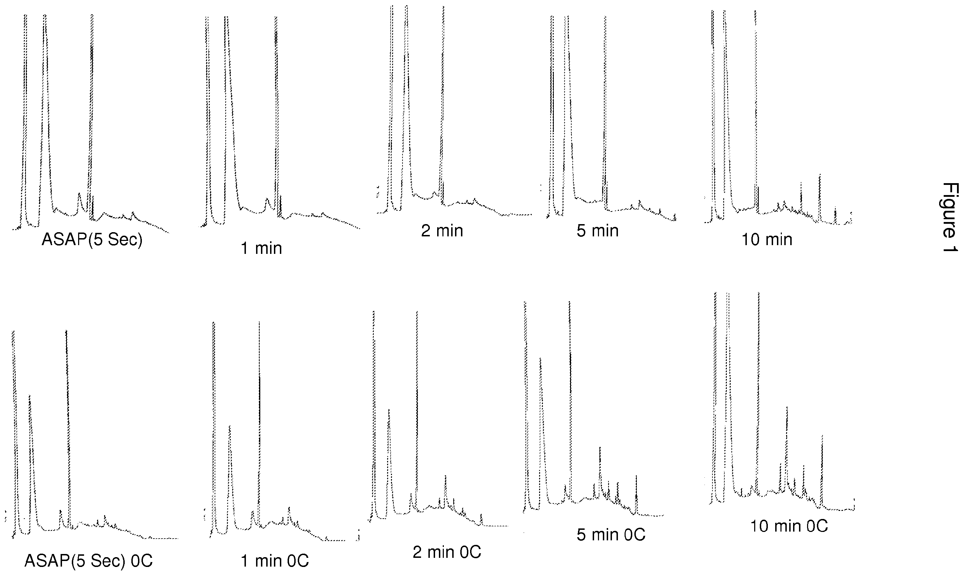

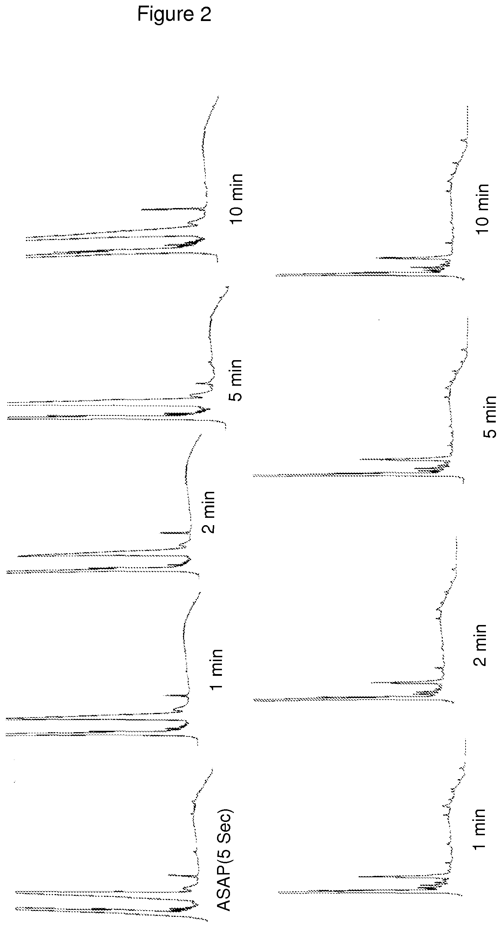

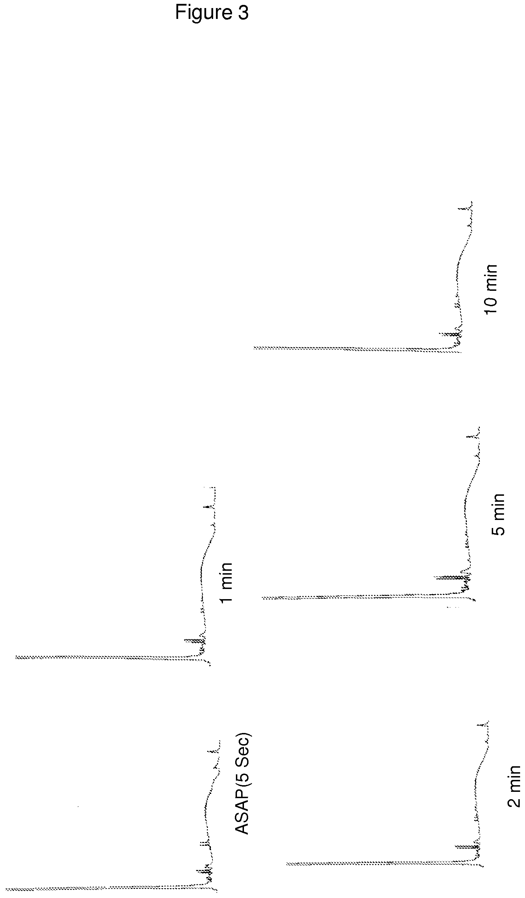

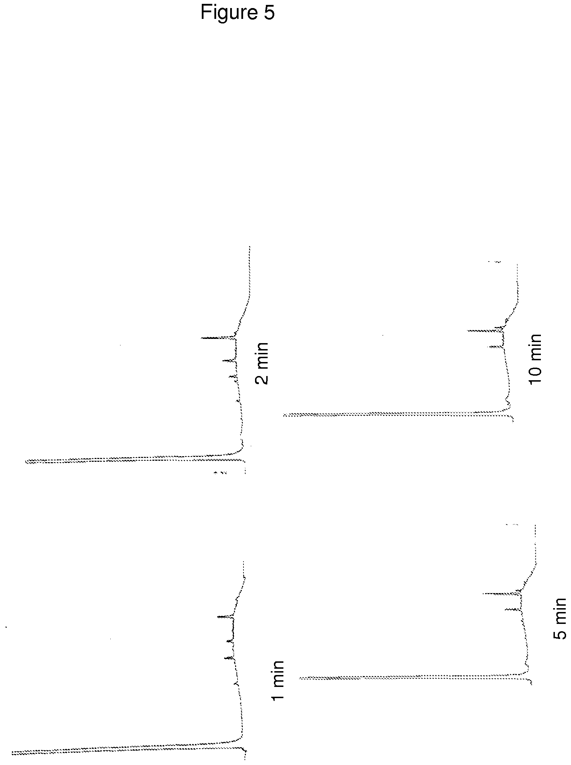

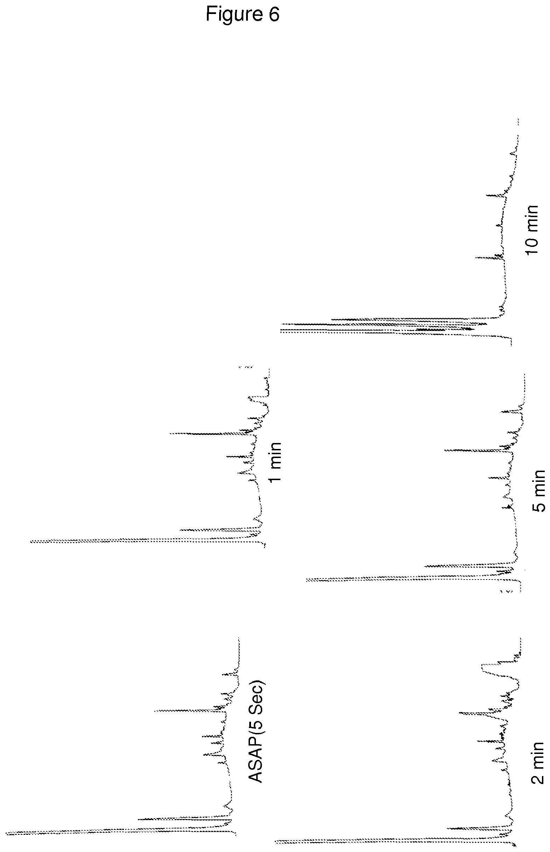

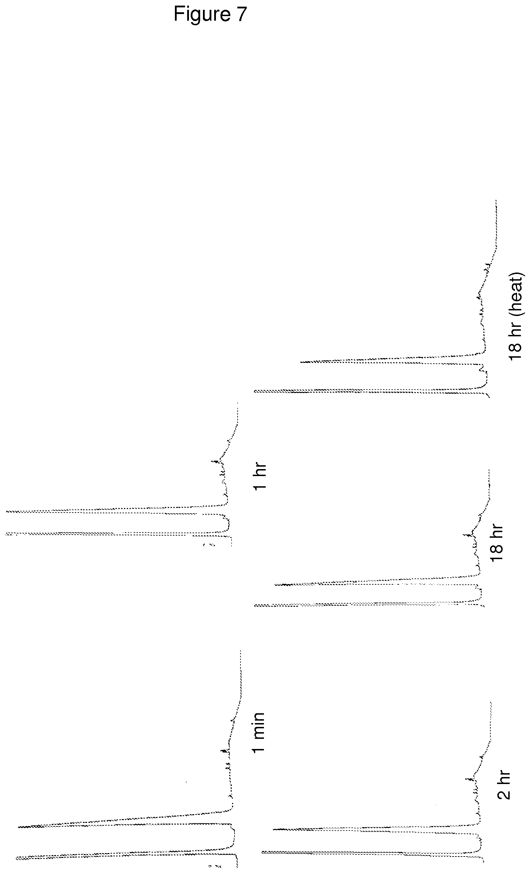

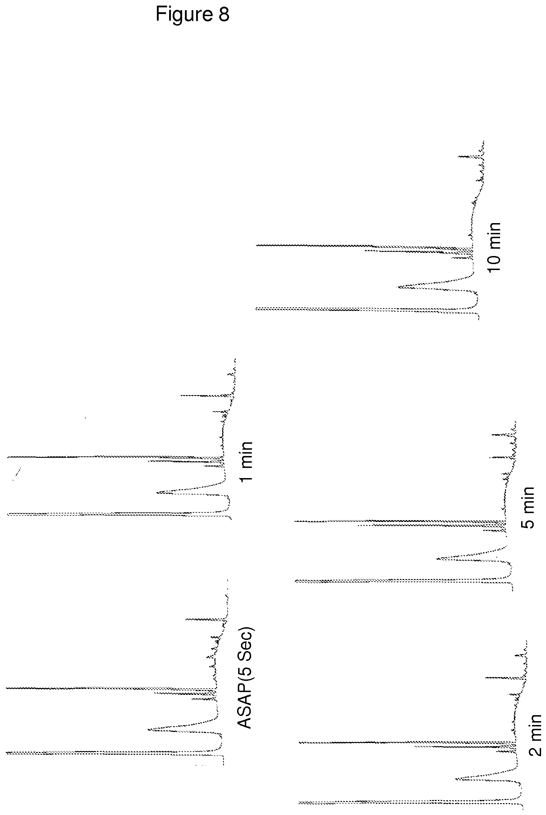

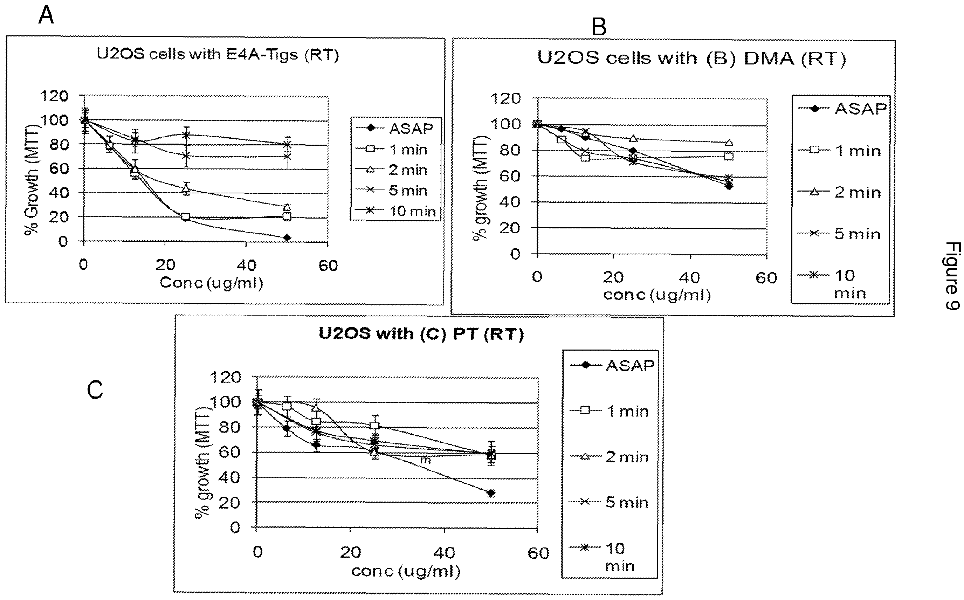

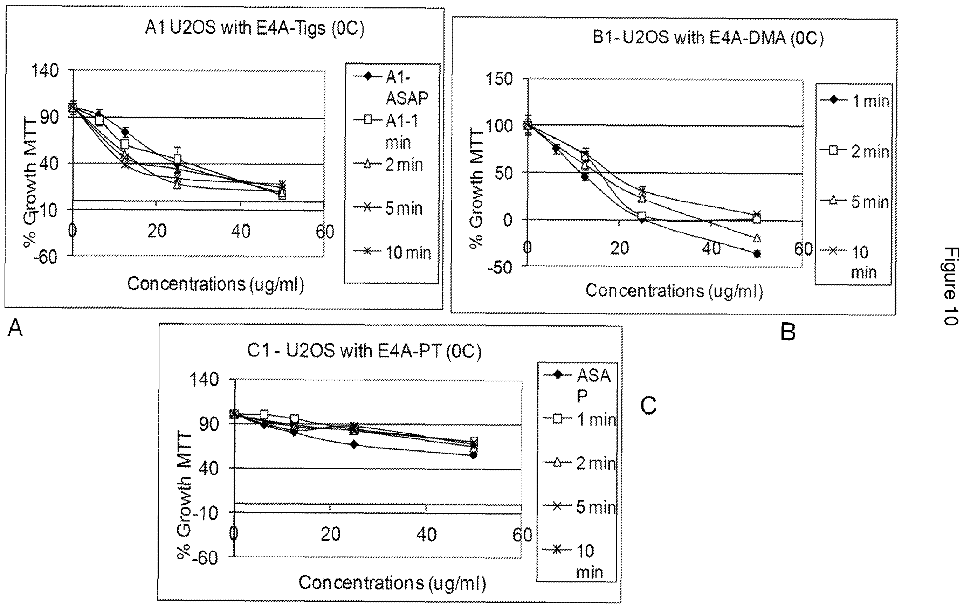

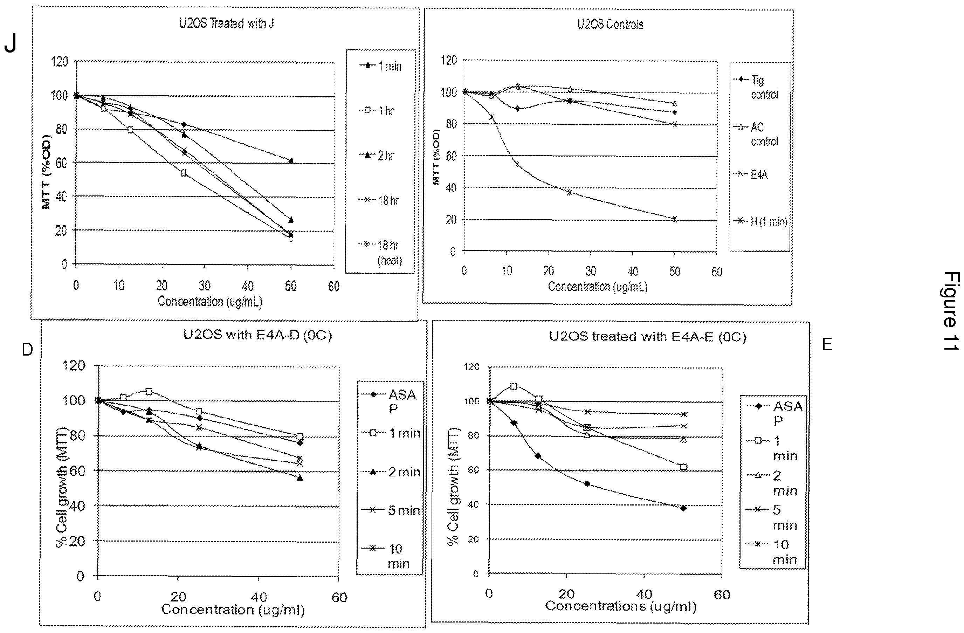

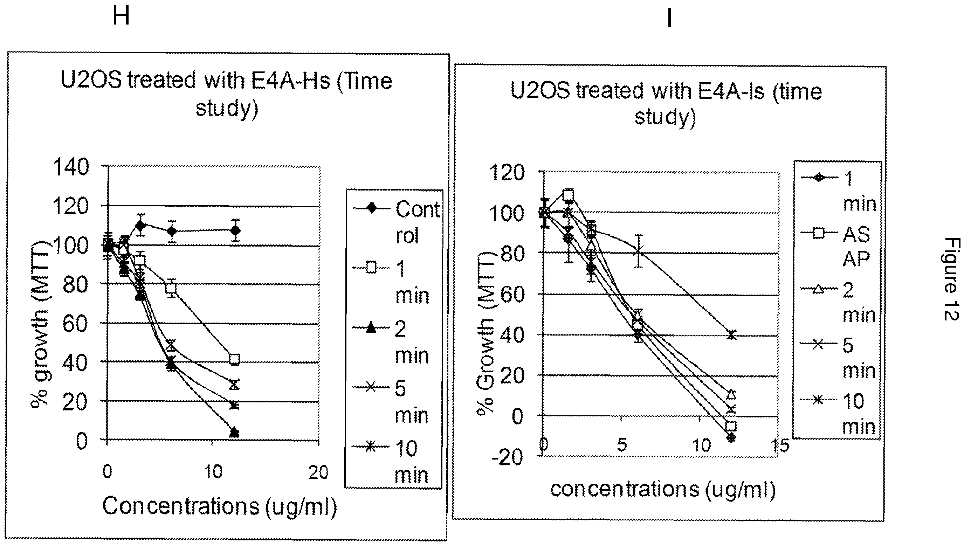

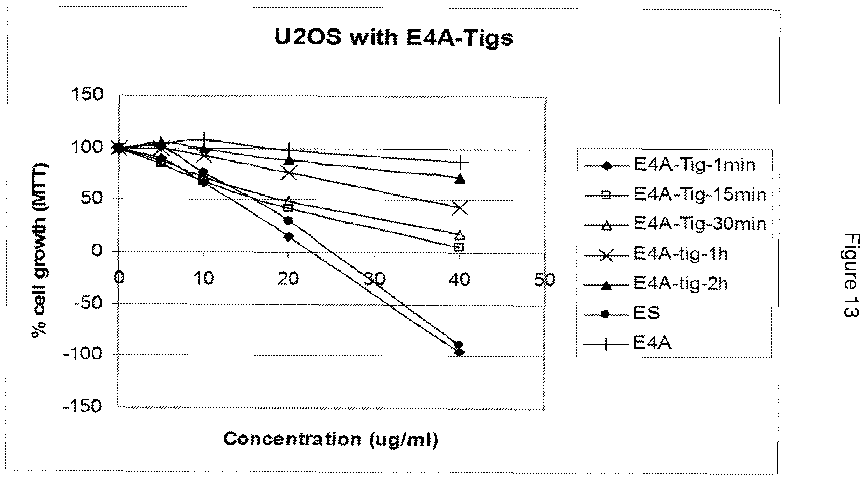

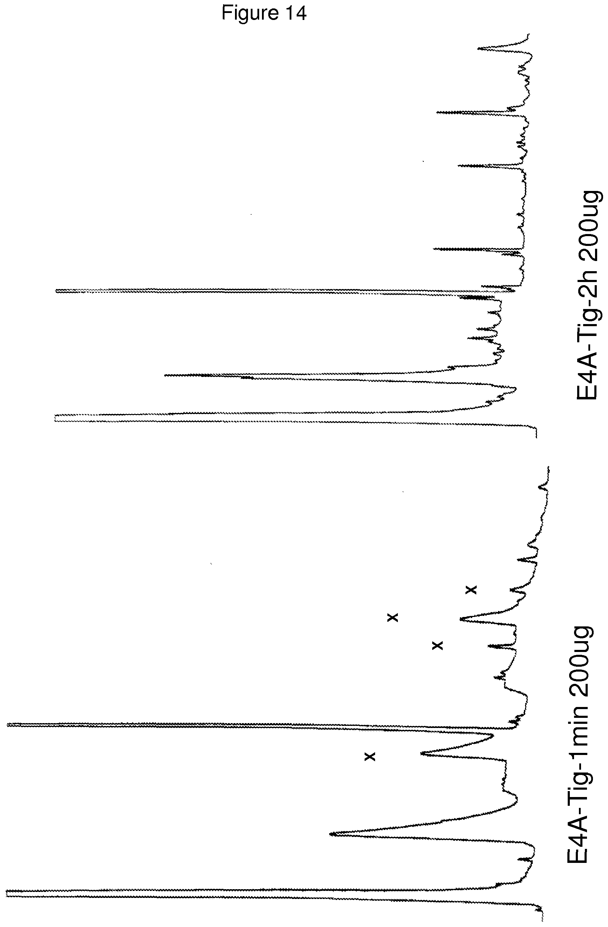

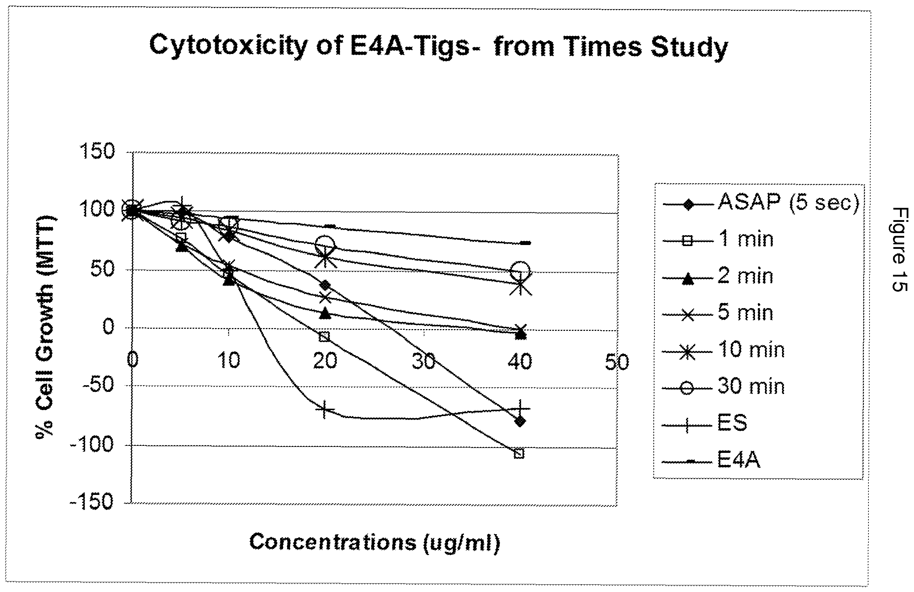

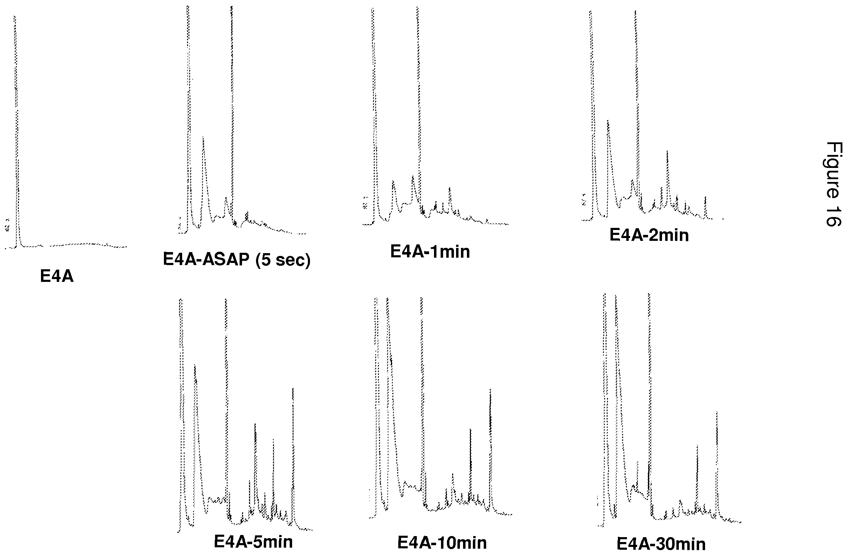

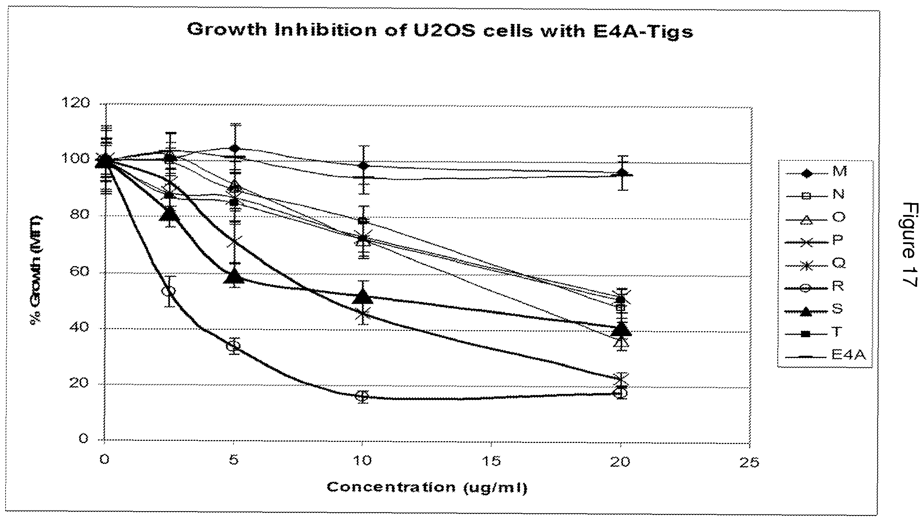

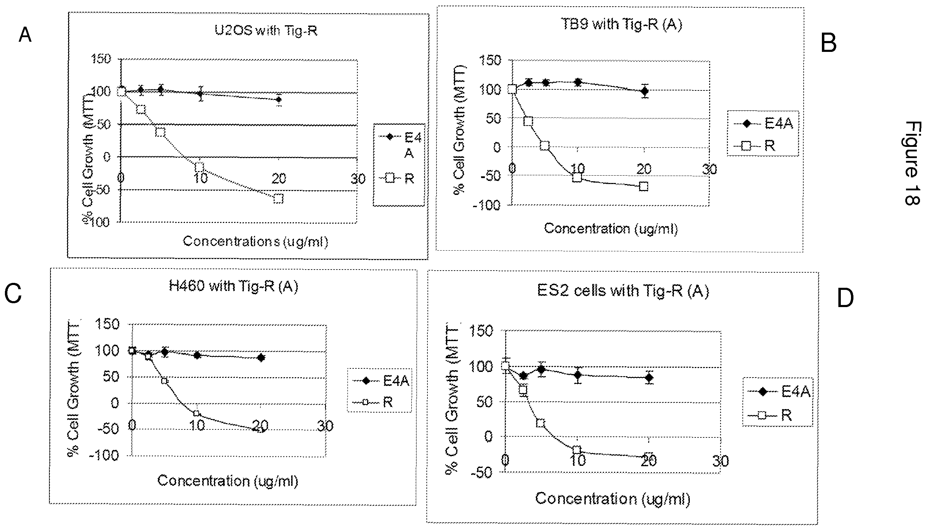

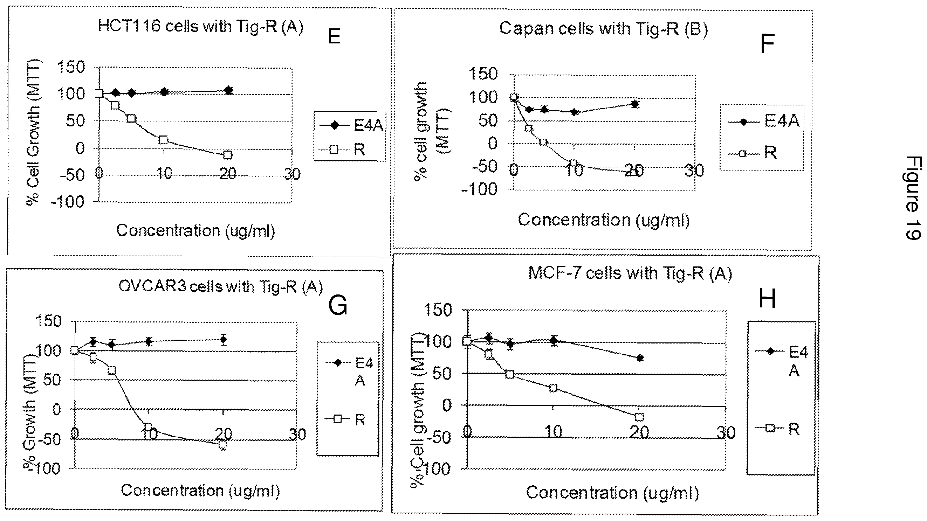

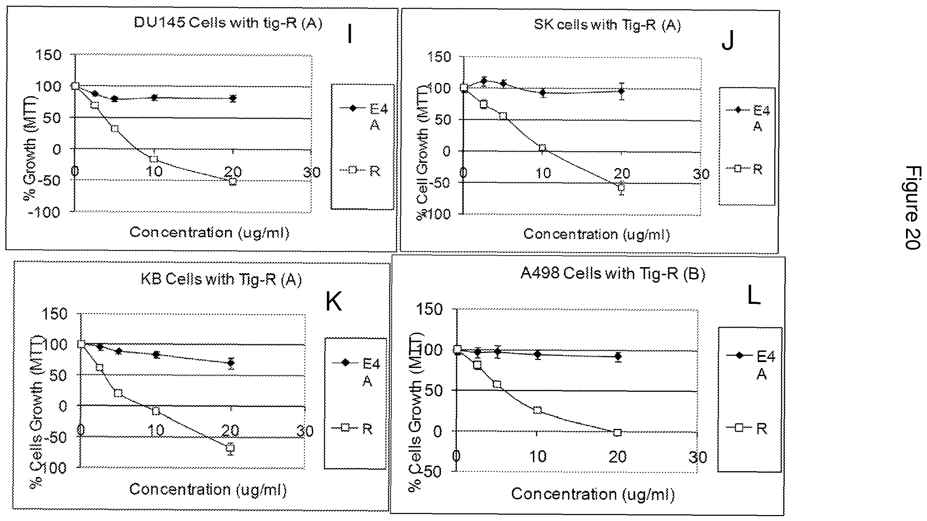

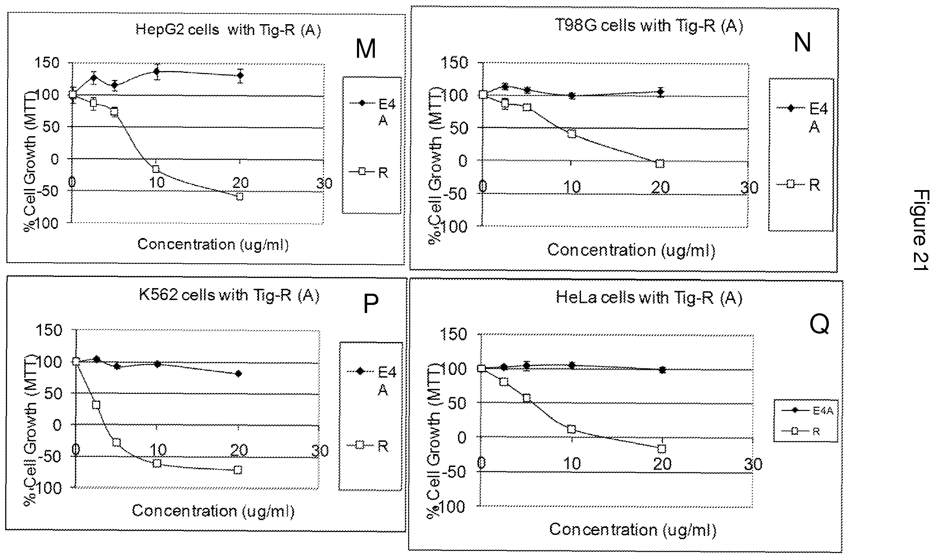

MTT activity of acylation of core compounds in different reaction time period of (ASAP)5 sec, 1 min, 2 min, 5 min, 10 min, 30 min, 1hr, 2 hr, 18 hr, 2 days or 3 days at 0C, 25C or 75C temperature were studied. HPLC profiles of esterification products of core compound E4A with acyl chloride including tigloyl chloride, angeloyl chloride, acetyl chloride, crotonoyl chloride, 3,3-Dimethylartyloyl chloride, senecioyl chloride, cinnamoyl chloride, pentenoyl chloride, hexanoyl chloride, benzoyl chloride or ethylbutyryl chloride show that the compounds vary in composition when the time or temperature of the reaction is changed. See Figures 1-21

The peaks, fractions and compounds are selected according to the activities of times studies and the changes of peaks. Selecting the HPLC fractions for isolation is according to the cytotoxic activity of the reaction product obtained at a specific time. The compounds having strong to weak activities are selected and isolated. The anti cancer activities are the MTT studies of bone (U2OS), lung (H460), bladder(HTB-9), ovary (ES2), colon (HCT116), pancreas (Capan), ovary(OVCAR3), prostate (DU145), skin (SK-Mel-5), mouth (KB), kidney (A498), breast (MCF-7), liver (HepG2), brain (T98G), luekemia (K562), cervix (HeLa).

Esterification of core compound E4A with Tigloyl chloride and isolation of the compounds with HPLC give the following compounds:

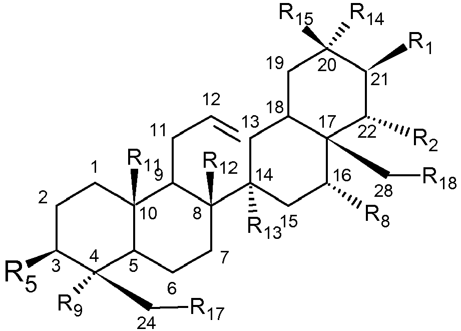

| | R1 | R2 | R5 | R8 | R17 | R18 | Cytotoxicity activity |
|---|---|---|---|---|---|---|---|
| E4A | OH | OH | OH | OH | OH | OH | none |
| A1 | OH | OH | OH | OH | O-Tig | OH | moderate |
| A2 | OH | OH | OH | OH | OH | O-Tig | moderate |
| A3 | OH | OH | OH | OH | O-Tig | O-Tig | strong |
| A4 | O-Tig | OH | OH | OH | O-Tig | O-Tig | moderate |
| A5 | OH | O-Tig | OH | OH | O-Tig | O-Tig | moderate |
| A6 | OH | OH | O-Tig | OH | O-Tig | O-Tig | moderate |
| A7 | OH | OH | OH | O-Tig | O-Tig | O-Tig | moderate |
| A8 | O-Tig | O-Tig | OH | OH | O-Tig | O-Tig | weak |
| A9 | OH | O-Tig | O-Tig | OH | O-Tig | O-Tig | weak |
| A10 | OH | OH | O-Tig | O-Tig | O-Tig | O-Tig | weak |
| A11 | O-Tig | OH | O-Tig | OH | O-Tig | O-Tig | weak |
| A12 | OH | O-Tig | OH | O-Tig | O-Tig | O-Tig | weak |
| A13 | O-Tig | OH | OH | O-Tig | O-Tig | O-Tig | weak |
| A14 | OH | O-Tig | O-Tig | OH | O-Tig | O-Tig | weak |
| A15 | O-Tig | O-Tig | O-Tig | OH | O-Tig | O-Tig | weak |
| A16 | O-Tig | O-Tig | OH | O-Tig | O-Tig | O-Tig | weak |
| A17 | O-Tig | OH | O-Tig | O-Tig | O-Tig | O-Tig | weak |
| A18 | OH | O-Tig | O-Tig | O-Tig | O-Tig | O-Tig | weak |
| A19 | O-Tig | O-Tig | O-Tig | O-Tig | O-Tig | O-Tig | none |
| A20 | O-Tig | O-Tig | OH | OH | OH | O-Tig | moderate |
| A21 | O-Tig | O-Tig | OH | OH | O-Tig | OH | moderate |
| A22 | O-Tig | O-Tig | OH | O-Tig | OH | OH | moderate |
| A23 | O-Tig | O-Tig | O-Tig | OH | OH | OH | moderate |
| A24 | O-Tig | O-Tig | OH | OH | OH | OH | moderate |
| A25 | O-Tig | OH | OH | OH | OH | O-Tig | moderate |
| A26 | OH | O-Tig | OH | OH | OH | O-Tig | moderate |
| A27 | OH | OH | O-Tig | OH | OH | O-Tig | moderate |
| A28 | OH | OH | OH | O-Tig | OH | O-Tig | moderate |
| A29 | O-Tig | OH | OH | OH | O-Tig | OH | moderate |
| A30 | OH | O-Tig | OH | OH | O-Tig | OH | moderate |
| A31 | OH | OH | O-Tig | OH | O-Tig | OH | moderate |
| A32 | OH | OH | OH | O-Tig | O-Tig | OH | moderate |

Esterification of core compound E4A with Angeloyl chloride and isolation of the compounds with HPLC give the following compounds:

| | R1 | R2 | R5 | R8 | R17 | R18 | Cytotoxicity activity |
|---|---|---|---|---|---|---|---|
| E4A | OH | OH | OH | OH | OH | OH | none |
| G1 | OH | OH | OH | OH | O-Ang | OH | moderate |
| G2 | OH | OH | OH | OH | OH | O-Ang | moderate |
| G3 | OH | OH | OH | OH | O-Ang | O-Ang | strong |
| G4 | O-Ang | OH | OH | OH | O-Ang | O-Ang | moderate |
| G5 | OH | O-Ang | OH | OH | O-Ang | O-Ang | moderate |
| G6 | OH | OH | O-Ang | OH | O-Ang | O-Ang | moderate |
| G7 | OH | OH | OH | O-Ang | O-Ang | O-Ang | moderate |
| G8 | O-Ang | O-Ang | OH | OH | O-Ang | O-Ang | weak |
| G9 | OH | O-Ang | O-Ang | OH | O-Ang | O-Ang | weak |
| G10 | OH | OH | O-Ang | O-Ang | O-Ang | O-Ang | weak |
| G11 | O-Ang | OH | O-Ang | OH | O-Ang | O-Ang | weak |
| G12 | OH | O-Ang | OH | O-Ang | O-Ang | O-Ang | weak |
| G13 | O-Ang | OH | OH | O-Ang | O-Ang | O-Ang | weak |
| G14 | OH | O-Ang | O-Ang | OH | O-Ang | O-Ang | weak |
| G15 | O-Ang | O-Ang | O-Ang | OH | O-Ang | O-Ang | weak |
| G16 | O-Ang | O-Ang | OH | O-Ang | O-Ang | O-Ang | weak |
| G17 | O-Ang | OH | O-Ang | O-Ang | O-Ang | O-Ang | weak |
| G18 | OH | O-Ang | O-Ang | O-Ang | O-Ang | O-Ang | weak |
| G19 | O-Ang | O-Ang | O-Ang | O-Ang | O-Ang | O-Ang | none |
| G20 | O-Ang | O-Ang | OH | OH | OH | O-Ang | moderate |
| G21 | O-Ang | O-Ang | OH | OH | O-Ang | OH | moderate |
| G22 | O-Ang | O-Ang | OH | O-Ang | OH | OH | moderate |
| G23 | O-Ang | O-Ang | O-Ang | OH | OH | OH | moderate |
| G24 | O-Ang | O-Ang | OH | OH | OH | OH | moderate |
| G25 | O-Ang | OH | OH | OH | OH | O-Ang | moderate |
| G26 | OH | O-Ang | OH | OH | OH | O-Ang | moderate |
| G27 | OH | OH | O-Ang | OH | OH | O-Ang | moderate |
| G28 | OH | OH | OH | O-Ang | OH | O-Ang | moderate |
| G29 | O-Ang | OH | OH | OH | O-Ang | OH | moderate |
| G30 | OH | O-Ang | OH | OH | O-Ang | OH | moderate |
| G31 | OH | OH | O-Ang | OH | O-Ang | OH | moderate |
| G32 | OH | OH | OH | O-Ang | O-Ang | OH | moderate |

Esterification of core compound E4A with (3,3-Dimethylartyloyl chloride) senecioyl chloride and isolation of the compounds with HPLC give the following compounds:
Sen=senecioyl

| | R1 | R2 | R5 | R8 | R17 | R18 | Cytotoxicity activity |
|---|---|---|---|---|---|---|---|
| E4A | OH | OH | OH | OH | OH | OH | none |
| B1 | OH | OH | OH | OH | O-Sen | OH | moderate |
| B2 | OH | OH | OH | OH | OH | O-Sen | moderate |
| B3 | OH | OH | OH | OH | O-Sen | O-Sen | strong |
| B4 | O-Sen | OH | OH | OH | O-Sen | O-Sen | moderate |
| B5 | OH | O-Sen | OH | OH | O-Sen | O-Sen | moderate |
| B6 | OH | OH | O-Sen | OH | O-Sen | O-Sen | moderate |
| B7 | OH | OH | OH | O-Sen | O-Sen | O-Sen | moderate |
| B8 | O-Sen | O-Sen | OH | OH | O-Sen | O-Sen | weak |
| B9 | OH | O-Sen | O-Sen | OH | O-Sen | O-Sen | weak |
| B10 | OH | OH | O-Sen | O-Sen | O-Sen | O-Sen | weak |
| B11 | O-Sen | OH | O-Sen | OH | O-Sen | O-Sen | weak |
| B12 | OH | O-Sen | OH | O-Sen | O-Sen | O-Sen | weak |
| B13 | O-Sen | OH | OH | O-Sen | O-Sen | O-Sen | weak |
| B14 | OH | O-Sen | O-Sen | OH | O-Sen | O-Sen | weak |
| B15 | O-Sen | O-Sen | O-Sen | OH | O-Sen | O-Sen | weak |
| B16 | O-Sen | O-Sen | OH | O-Sen | O-Sen | O-Sen | weak |
| B17 | O-Sen | OH | O-Sen | O-Sen | O-Sen | O-Sen | weak |
| B18 | OH | O-Sen | O-Sen | O-Sen | O-Sen | O-Sen | weak |
| B19 | O-Sen | O-Sen | O-Sen | O-Sen | O-Sen | O-Sen | none |
| B20 | O-Sen | O-Sen | OH | OH | OH | O-Sen | moderate |
| B21 | O-Sen | O-Sen | OH | OH | O-Sen | OH | moderate |
| B22 | O-Sen | O-Sen | OH | O-Sen | OH | OH | moderate |
| B23 | O-Sen | O-Sen | O-Sen | OH | OH | OH | moderate |
| B24 | O-Sen | O-Sen | OH | OH | OH | OH | moderate |
| B25 | O-Sen | OH | OH | OH | OH | O-Sen | moderate |
| B26 | OH | O-Sen | OH | OH | OH | O-Sen | moderate |
| B27 | OH | OH | O-Sen | OH | OH | O-Sen | moderate |
| B28 | OH | OH | OH | O-Sen | OH | O-Sen | moderate |
| B29 | O-Sen | OH | OH | OH | O-Sen | OH | moderate |
| B30 | OH | O-Sen | OH | OH | O-Sen | OH | moderate |
| B31 | OH | OH | O-Sen | OH | O-Sen | OH | moderate |
| B32 | OH | OH | OH | O-Sen | O-Sen | OH | moderate |

Esterification of core compound E4A with 4-Pentenoyl chloride and isolation of the compounds with HPLC give the following compounds:
Pen=4-Pentenoyl

| | R1 | R2 | R5 | R8 | R17 | R18 | Cytotoxicity activity |
|---|---|---|---|---|---|---|---|
| E4A | OH | OH | OH | OH | OH | OH | none |
| C1 | OH | OH | OH | OH | O-Pen | OH | moderate |
| C2 | OH | OH | OH | OH | OH | O-Pen | moderate |
| C3 | OH | OH | OH | OH | O-Pen | O-Pen | strong |
| C4 | O-Pen | OH | OH | OH | O-Pen | O-Pen | moderate |
| C5 | OH | O-Pen | OH | OH | O-Pen | O-Pen | moderate |
| C6 | OH | OH | O-Pen | OH | O-Pen | O-Pen | moderate |
| C7 | OH | OH | OH | O-Pen | O-Pen | O-Pen | moderate |
| C8 | O-Pen | O-Pen | OH | OH | O-Pen | O-Pen | weak |
| C9 | OH | O-Pen | O-Pen | OH | O-Pen | O-Pen | weak |
| C10 | OH | OH | O-Pen | O-Pen | O-Pen | O-Pen | weak |
| C11 | O-Pen | OH | O-Pen | OH | O-Pen | O-Pen | weak |
| C12 | OH | O-Pen | OH | O-Pen | O-Pen | O-Pen | weak |
| C13 | O-Pen | OH | OH | O-Pen | O-Pen | O-Pen | weak |
| C14 | OH | O-Pen | O-Pen | OH | O-Pen | O-Pen | weak |
| C15 | O-Pen | O-Pen | O-Pen | OH | O-Pen | O-Pen | weak |
| C16 | O-Pen | O-Pen | OH | O-Pen | O-Pen | O-Pen | weak |
| C17 | O-Pen | OH | O-Pen | O-Pen | O-Pen | O-Pen | weak |
| C18 | OH | O-Pen | O-Pen | O-Pen | O-Pen | O-Pen | weak |
| C19 | O-Pen | O-Pen | O-Pen | O-Pen | O-Pen | O-Pen | none |
| C20 | O-Pen | O-Pen | OH | OH | OH | O-Pen | moderate |
| C21 | O-Pen | O-Pen | OH | OH | O-Pen | OH | moderate |
| C22 | O-Pen | O-Pen | OH | O-Pen | OH | OH | moderate |
| C23 | O-Pen | O-Pen | O-Pen | OH | OH | OH | moderate |
| C24 | O-Pen | O-Pen | OH | OH | OH | OH | moderate |
| C25 | O-Pen | OH | OH | OH | OH | O-Pen | moderate |
| C26 | OH | O-Pen | OH | OH | OH | O-Pen | moderate |
| C27 | OH | OH | O-Pen | OH | OH | O-Pen | moderate |
| C28 | OH | OH | OH | O-Pen | OH | O-Pen | moderate |
| C29 | O-Pen | OH | OH | OH | O-Pen | OH | moderate |
| C30 | OH | O-Pen | OH | OH | O-Pen | OH | moderate |
| C31 | OH | OH | O-Pen | OH | O-Pen | OH | moderate |
| C32 | OH | OH | OH | O-Pen | O-Pen | OH | moderate |

Esterification of core compound E4A with Hexanoyl chloride and isolation of the compounds with HPLC give the following compounds:
Hex= Hexanoyl

| | R1 | R2 | R5 | R8 | R17 | R18 | Cytotoxicity activity |
|---|---|---|---|---|---|---|---|
| E4AF | OH | OH | OH | OH | OH | OH | none |
| D1 | OH | OH | OH | OH | O-Hex | OH | moderate |
| D2 | OH | OH | OH | OH | OH | O-Hex | moderate |
| D3 | OH | OH | OH | OH | O-Hex | O-Hex | strong |
| D4 | O-Hex | OH | OH | OH | O-Hex | O-Hex | moderate |
| D5 | OH | O-Hex | OH | OH | O-Hex | O-Hex | moderate |
| D6 | OH | OH | O-Hex | OH | O-Hex | O-Hex | moderate |
| D7 | OH | OH | OH | O-Hex | O-Hex | O-Hex | moderate |
| D8 | O-Hex | O-Hex | OH | OH | O-Hex | O-Hex | weak |
| D9 | OH | O-Hex | O-Hex | OH | O-Hex | O-Hex | weak |
| D10 | OH | OH | O-Hex | O-Hex | O-Hex | O-Hex | weak |
| D11 | O-Hex | OH | O-Hex | OH | O-Hex | O-Hex | weak |
| D12 | OH | O-Hex | OH | O-Hex | O-Hex | O-Hex | weak |
| D13 | O-Hex | OH | OH | O-Hex | O-Hex | O-Hex | weak |
| D14 | OH | O-Hex | O-Hex | OH | O-Hex | O-Hex | weak |
| D15 | O-Hex | O-Hex | O-Hex | OH | O-Hex | O-Hex | weak |
| D16 | O-Hex | O-Hex | OH | O-Hex | O-Hex | O-Hex | weak |
| D17 | O-Hex | OH | O-Hex | O-Hex | O-Hex | O-Hex | weak |
| D18 | OH | O-Hex | O-Hex | O-Hex | O-Hex | O-Hex | weak |
| D19 | O-Hex | O-Hex | O-Hex | O-Hex | O-Hex | O-Hex | none |
| D20 | O-Hex | O-Hex | OH | OH | OH | O-Hex | moderate |
| D21 | O-Hex | O-Hex | OH | OH | O-Hex | OH | moderate |
| D22 | O-Hex | O-Hex | OH | O-Hex | OH | OH | moderate |
| D23 | O-Hex | O-Hex | O-Hex | OH | OH | OH | moderate |
| D24 | O-Hex | O-Hex | OH | OH | OH | OH | moderate |
| D25 | O-Hex | OH | OH | OH | OH | O-Hex | moderate |
| D26 | OH | O-Hex | OH | OH | OH | O-Hex | moderate |
| D27 | OH | OH | O-Hex | OH | OH | O-Hex | moderate |
| D28 | OH | OH | OH | O-Hex | OH | O-Hex | moderate |
| D29 | O-Hex | OH | OH | OH | O-Hex | OH | moderate |
| D30 | OH | O-Hex | OH | OH | O-Hex | OH | moderate |
| D31 | OH | OH | O-Hex | OH | O-Hex | OH | moderate |
| D32 | OH | OH | OH | O-Hex | O-Hex | OH | moderate |

Esterification of core compound E4A with 2-Ethylbutyryl chloride and isolation of the compounds with HPLC give the following compounds:
Eth= 2-Ethylbutyryl

| | R1 | R2 | R5 | R8 | R17 | R18 | Cytotoxicity activity |
|---|---|---|---|---|---|---|---|
| E4AF | OH | OH | OH | OH | OH | OH | none |
| E1 | OH | OH | OH | OH | O-Eth | OH | moderate |
| E2 | OH | OH | OH | OH | OH | O-Eth | moderate |
| E3 | OH | OH | OH | OH | O-Eth | O-Eth | strong |
| E4 | O-Eth | OH | OH | OH | O-Eth | O-Eth | moderate |
| E5 | OH | O-Eth | OH | OH | O-Eth | O-Eth | moderate |
| E6 | OH | OH | O-Eth | OH | O-Eth | O-Eth | moderate |
| E7 | OH | OH | OH | O-Eth | O-Eth | O-Eth | moderate |
| E8 | O-Eth | O-Eth | OH | OH | O-Eth | O-Eth | weak |
| E9 | OH | O-Eth | O-Eth | OH | O-Eth | O-Eth | weak |
| E10 | OH | OH | O-Eth | O-Eth | O-Eth | O-Eth | weak |
| E11 | O-Eth | OH | O-Eth | OH | O-Eth | O-Eth | weak |
| E12 | OH | O-Eth | OH | O-Eth | O-Eth | O-Eth | weak |
| E13 | O-Eth | OH | OH | O-Eth | O-Eth | O-Eth | weak |
| E14 | OH | O-Eth | O-Eth | OH | O-Eth | O-Eth | weak |
| E15 | O-Eth | O-Eth | O-Eth | OH | O-Eth | O-Eth | weak |
| E16 | O-Eth | O-Eth | OH | O-Eth | O-Eth | O-Eth | weak |
| E17 | O-Eth | OH | O-Eth | O-Eth | O-Eth | O-Eth | weak |
| E18 | OH | O-Eth | O-Eth | O-Eth | O-Eth | O-Eth | weak |
| E19 | O-Eth | O-Eth | O-Eth | O-Eth | O-Eth | O-Eth | none |
| E20 | O-Eth | O-Eth | OH | OH | OH | O-Eth | moderate |
| E21 | O-Eth | O-Eth | OH | OH | O-Eth | OH | moderate |
| E22 | O-Eth | O-Eth | OH | O-Eth | OH | OH | moderate |
| E23 | O-Eth | O-Eth | O-Eth | OH | OH | OH | moderate |
| E24 | O-Eth | O-Eth | OH | OH | OH | OH | moderate |
| E25 | O-Eth | OH | OH | OH | OH | O-Eth | moderate |
| E26 | OH | O-Eth | OH | OH | OH | O-Eth | moderate |
| E27 | OH | OH | O-Eth | OH | OH | O-Eth | moderate |
| E28 | OH | OH | OH | O-Eth | OH | O-Eth | moderate |
| E29 | O-Eth | OH | OH | OH | O-Eth | OH | moderate |
| E30 | OH | O-Eth | OH | OH | O-Eth | OH | moderate |
| E31 | OH | OH | O-Eth | OH | O-Eth | OH | moderate |
| E32 | OH | OH | OH | O-Eth | O-Eth | OH | moderate |

Esterification of core compound E4A with Acetyl chloride (H) and isolation of the compounds with HPLC give the following compounds:
Acy =Acetyl

| | R1 | R2 | R5 | R8 | R17 | R18 | Cytotoxicity activity |
|---|---|---|---|---|---|---|---|
| E4A | OH | OH | OH | OH | OH | OH | none |
| H1 | OH | OH | OH | OH | O-Acy | OH | moderate |
| H2 | OH | OH | OH | OH | OH | O-Acy | moderate |
| H3 | OH | OH | OH | OH | O-Acy | O-Acy | strong |
| H4 | O-Acy | OH | OH | OH | O-Acy | O-Acy | moderate |
| H5 | OH | O-Acy | OH | OH | O-Acy | O-Acy | moderate |
| H6 | OH | OH | O-Acy | OH | O-Acy | O-Acy | moderate |
| H7 | OH | OH | OH | O-Acy | O-Acy | O-Acy | moderate |
| H8 | O-Acy | O-Acy | OH | OH | O-Acy | O-Acy | weak |
| H9 | OH | O-Acy | O-Acy | OH | O-Acy | O-Acy | weak |
| H10 | OH | OH | O-Acy | O-Acy | O-Acy | O-Acy | weak |
| H11 | O-Acy | OH | O-Acy | OH | O-Acy | O-Acy | weak |
| H12 | OH | O-Acy | OH | O-Acy | O-Acy | O-Acy | weak |
| H13 | O-Acy | OH | OH | O-Acy | O-Acy | O-Acy | weak |
| H14 | OH | O-Acy | O-Acy | OH | O-Acy | O-Acy | weak |
| H15 | O-Acy | O-Acy | O-Acy | OH | O-Acy | O-Acy | weak |
| H16 | O-Acy | O-Acy | OH | O-Acy | O-Acy | O-Acy | weak |
| H17 | O-Acy | OH | O-Acy | O-Acy | O-Acy | O-Acy | weak |
| H18 | OH | O-Acy | O-Acy | O-Acy | O-Acy | O-Acy | weak |
| H19 | O-Acy | O-Acy | O-Acy | O-Acy | O-Acy | O-Acy | none |
| H20 | O-Acy | O-Acy | OH | OH | OH | O-Acy | moderate |
| H21 | O-Acy | O-Acy | OH | OH | O-Acy | OH | moderate |
| H22 | O-Acy | O-Acy | OH | O-Acy | OH | OH | moderate |
| H23 | O-Acy | O-Acy | O-Acy | OH | OH | OH | moderate |
| H24 | O-Acy | O-Acy | OH | OH | OH | OH | moderate |
| H25 | O-Acy | OH | OH | OH | OH | O-Acy | moderate |
| H26 | OH | O-Acy | OH | OH | OH | O-Acy | moderate |
| H27 | OH | OH | O-Acy | OH | OH | O-Acy | moderate |
| H28 | OH | OH | OH | O-Acy | OH | O-Acy | moderate |
| H29 | O-Acy | OH | OH | OH | O-Acy | OH | moderate |
| H30 | OH | O-Acy | OH | OH | O-Acy | OH | moderate |
| H31 | OH | OH | O-Acy | OH | O-Acy | OH | moderate |
| H32 | OH | OH | OH | O-Acy | O-Acy | OH | moderate |

Esterification of core compound E4A with Crotonoyl chloride and isolation of the compounds with HPLC give the following compounds:
Cro= Crotonoyl

| | R1 | R2 | R5 | R8 | R17 | R18 | Cytotoxicity activity |
|---|---|---|---|---|---|---|---|
| E4A | OH | OH | OH | OH | OH | OH | none |
| I1 | OH | OH | OH | OH | O-Cro | OH | moderate |
| I2 | OH | OH | OH | OH | OH | O-Cro | moderate |
| I3 | OH | OH | OH | OH | O-Cro | O-Cro | strong |
| I4 | O-Cro | OH | OH | OH | O-Cro | O-Cro | moderate |
| I5 | OH | O-Cro | OH | OH | O-Cro | O-Cro | moderate |
| I6 | OH | OH | O-Cro | OH | O-Cro | O-Cro | moderate |
| I7 | OH | OH | OH | O-Cro | O-Cro | O-Cro | moderate |
| I8 | O-Cro | O-Cro | OH | OH | O-Cro | O-Cro | weak |
| I9 | OH | O-Cro | O-Cro | OH | O-Cro | O-Cro | weak |
| I10 | OH | OH | O-Cro | O-Cro | O-Cro | O-Cro | weak |
| I11 | O-Cro | OH | O-Cro | OH | O-Cro | O-Cro | weak |
| I12 | OH | O-Cro | OH | O-Cro | O-Cro | O-Cro | weak |
| I13 | O-Cro | OH | OH | O-Cro | O-Cro | O-Cro | weak |
| I14 | OH | O-Cro | O-Cro | OH | O-Cro | O-Cro | weak |
| I15 | O-Cro | O-Cro | O-Cro | OH | O-Cro | O-Cro | weak |
| I16 | O-Cro | O-Cro | OH | O-Cro | O-Cro | O-Cro | weak |
| I17 | O-Cro | OH | O-Cro | O-Cro | O-Cro | O-Cro | weak |
| I18 | OH | O-Cro | O-Cro | O-Cro | O-Cro | O-Cro | weak |
| I19 | O-Cro | O-Cro | O-Cro | O-Cro | O-Cro | O-Cro | none |
| I20 | O-Cro | O-Cro | OH | OH | OH | O-Cro | moderate |
| I21 | O-Cro | O-Cro | OH | OH | O-Cro | OH | moderate |
| I22 | O-Cro | O-Cro | OH | O-Cro | OH | OH | moderate |
| I23 | O-Cro | O-Cro | O-Cro | OH | OH | OH | moderate |
| I24 | O-Cro | O-Cro | OH | OH | OH | OH | moderate |
| I25 | O-Cro | OH | OH | OH | OH | O-Cro | moderate |
| I26 | OH | O-Cro | OH | OH | OH | O-Cro | moderate |
| I27 | OH | OH | O-Cro | OH | OH | O-Cro | moderate |
| I28 | OH | OH | OH | O-Cro | OH | O-Cro | moderate |
| I29 | O-Cro | OH | OH | OH | O-Cro | OH | moderate |
| I30 | OH | O-Cro | OH | OH | O-Cro | OH | moderate |
| I31 | OH | OH | O-Cro | OH | O-Cro | OH | moderate |
| I32 | OH | OH | OH | O-Cro | O-Cro | OH | moderate |

Esterification of core compound E4A with Cinnamoyl chloride and isolation of the compounds with HPLC give the following compounds:
Cin= Cinnamoyl

| | R1 | R2 | R5 | R8 | R17 | R18 | Cytotoxicity activity |
|---|---|---|---|---|---|---|---|
| E4A | OH | OH | OH | OH | OH | OH | none |
| J1 | OH | OH | OH | OH | O-Cin | OH | moderate |
| J2 | OH | OH | OH | OH | OH | O-Cin | moderate |
| J3 | OH | OH | OH | OH | O-Cin | O-Cin | strong |
| J4 | O-Cin | OH | OH | OH | O-Cin | O-Cin | moderate |
| J5 | OH | O-Cin | OH | OH | O-Cin | O-Cin | moderate |
| J6 | OH | OH | O-Cin | OH | O-Cin | O-Cin | moderate |
| J7 | OH | OH | OH | O-Cin | O-Cin | O-Cin | moderate |
| J8 | O-Cin | O-Cin | OH | OH | O-Cin | O-Cin | weak |
| J9 | OH | O-Cin | O-Cin | OH | O-Cin | O-Cin | weak |
| J10 | OH | OH | O-Cin | O-Cin | O-Cin | O-Cin | weak |
| J11 | O-Cin | OH | O-Cin | OH | O-Cin | O-Cin | weak |
| J12 | OH | O-Cin | OH | O-Cin | O-Cin | O-Cin | weak |
| J13 | O-Cin | OH | OH | O-Cin | O-Cin | O-Cin | weak |
| J14 | OH | O-Cin | O-Cin | OH | O-Cin | O-Cin | weak |
| J15 | O-Cin | O-Cin | O-Cin | OH | O-Cin | O-Cin | weak |
| J16 | O-Cin | O-Cin | OH | O-Cin | O-Cin | O-Cin | weak |
| J17 | O-Cin | OH | O-Cin | O-Cin | O-Cin | O-Cin | weak |
| J18 | OH | O-Cin | O-Cin | O-Cin | O-Cin | O-Cin | weak |
| J19 | O-Cin | O-Cin | O-Cin | O-Cin | O-Cin | O-Cin | none |
| J20 | O-Cin | O-Cin | OH | OH | OH | O-Cin | moderate |
| J21 | O-Cin | O-Cin | OH | OH | O-Cin | OH | moderate |
| J22 | O-Cin | O-Cin | OH | O-Cin | OH | OH | moderate |
| J23 | O-Cin | O-Cin | O-Cin | OH | OH | OH | moderate |
| J24 | O-Cin | O-Cin | OH | OH | OH | OH | moderate |
| J25 | O-Cin | OH | OH | OH | OH | O-Cin | moderate |
| J26 | OH | O-Cin | OH | OH | OH | O-Cin | moderate |
| J27 | OH | OH | O-Cin | OH | OH | O-Cin | moderate |
| J28 | OH | OH | OH | O-Cin | OH | O-Cin | moderate |
| J29 | O-Cin | OH | OH | OH | O-Cin | OH | moderate |
| J30 | OH | O-Cin | OH | OH | O-Cin | OH | moderate |
| J31 | OH | OH | O-Cin | OH | O-Cin | OH | moderate |
| J32 | OH | OH | OH | O-Cin | O-Cin | OH | moderate |

Esterification of core compound E4A with benzoyl chloride and isolation of the compounds with HPLC give the following compounds:
Ben= benzoyl

| | R1 | R2 | R5 | R8 | R17 | R18 | Cytotoxicity activity |
|---|---|---|---|---|---|---|---|
| E4A | OH | OH | OH | OH | OH | OH | none |
| K1 | OH | OH | OH | OH | O-Ben | OH | moderate |
| K2 | OH | OH | OH | OH | OH | O-Ben | moderate |
| K3 | OH | OH | OH | OH | O-Ben | O-Ben | strong |
| K4 | O-Ben | OH | OH | OH | O-Ben | O-Ben | moderate |
| K5 | OH | O-Ben | OH | OH | O-Ben | O-Ben | moderate |
| K6 | OH | OH | O-Ben | OH | O-Ben | O-Ben | moderate |
| K7 | OH | OH | OH | O-Ben | O-Ben | O-Ben | moderate |
| K8 | O-Ben | O-Ben | OH | OH | O-Ben | O-Ben | weak |
| K9 | OH | O-Ben | O-Ben | OH | O-Ben | O-Ben | weak |
| K10 | OH | OH | O-Ben | O-Ben | O-Ben | O-Ben | weak |
| K11 | O-Ben | OH | O-Ben | OH | O-Ben | O-Ben | weak |
| K12 | OH | O-Ben | OH | O-Ben | O-Ben | O-Ben | weak |
| K13 | O-Ben | OH | OH | O-Ben | O-Ben | O-Ben | weak |
| K14 | OH | O-Ben | O-Ben | OH | O-Ben | O-Ben | weak |
| K15 | O-Ben | O-Ben | O-Ben | OH | O-Ben | O-Ben | weak |
| K16 | O-Ben | O-Ben | OH | O-Ben | O-Ben | O-Ben | weak |
| K17 | O-Ben | OH | O-Ben | O-Ben | O-Ben | O-Ben | weak |
| K18 | OH | O-Ben | O-Ben | O-Ben | O-Ben | O-Ben | weak |
| K19 | O-Ben | O-Ben | O-Ben | O-Ben | O-Ben | O-Ben | none |
| K20 | O-Ben | O-Ben | OH | OH | OH | O-Ben | moderate |
| K21 | O-Ben | O-Ben | OH | OH | O-Ben | OH | moderate |
| K22 | O-Ben | O-Ben | OH | O-Ben | OH | OH | moderate |
| K23 | O-Ben | O-Ben | O-Ben | OH | OH | OH | moderate |
| K24 | O-Ben | O-Ben | OH | OH | OH | OH | moderate |
| K25 | O-Ben | OH | OH | OH | OH | O-Ben | moderate |
| K26 | OH | O-Ben | OH | OH | OH | O-Ben | moderate |
| K27 | OH | OH | O-Ben | OH | OH | O-Ben | moderate |
| K28 | OH | OH | OH | O-Ben | OH | O-Ben | moderate |
| K29 | O-Ben | OH | OH | OH | O-Ben | OH | moderate |
| K30 | OH | O-Ben | OH | OH | O-Ben | OH | moderate |
| K31 | OH | OH | O-Ben | OH | O-Ben | OH | moderate |
| K32 | OH | OH | OH | O-Ben | O-Ben | OH | moderate |

Esterification of E4A-Tig-N with senecioyl chloride and isolation of the compounds with HPLC give the following compounds:

| | R1 | R2 | R5 | R8 | R17 | R18 | Cytotoxicity activity |
|---|---|---|---|---|---|---|---|
| E4A-Tig-N | OH | OH | OH | OH | O-Tig | OH | moderate |
| Tig-Sen-1 | OH | OH | OH | OH | O-Tig | O-Sen | strong |
| Tig-Sen-2 | O-Sen | OH | OH | OH | O-Tig | O-Sen | moderate |
| Tig-Sen-3 | OH | O-Sen | OH | OH | O-Tig | O-Sen | moderate |
| Tig-Sen-4 | OH | OH | O-Sen | OH | O-Tig | O-Sen | moderate |
| Tig-Sen-5 | O-Sen | OH | OH | OH | O-Tig | OH | moderate |
| Tig-Sen-6 | OH | O-Sen | OH | OH | O-Tig | OH | moderate |

Esterification of E4A-Tig-N with Crotonoyl chloride and isolation of the compounds with HPLC give the following compounds:

| | R1 | R2 | R5 | R8 | R17 | R18 | Cytotoxicity activity |
|---|---|---|---|---|---|---|---|
| E4A-Tig-N | OH | OH | OH | OH | O-Tig | OH | moderate |
| Tig-Cro-1 | OH | OH | OH | OH | O-Tig | O-Cro | strong |
| Tig-Cro-2 | O-Cro | OH | OH | OH | O-Tig | O-Cro | moderate |
| Tig-Cro-3 | OH | O-Cro | OH | OH | O-Tig | O-Cro | moderate |
| Tig-Cro-4 | OH | OH | O-Cro | OH | O-Tig | O-Cro | moderate |
| Tig-Cro-5 | O-Cro | OH | OH | OH | O-Tig | OH | moderate |
| Tig-Cro-6 | OH | O-Cro | OH | OH | O-Tig | OH | moderate |

Esterification of E4A-Tig-N with Acetyl chloride and isolation of the compounds with HPLC give the following compounds:

| | R1 | R2 | R5 | R8 | R17 | R18 | Cytotoxicity activity |
|---|---|---|---|---|---|---|---|
| E4A-Tig-N | OH | OH | OH | OH | O-Tig | OH | moderate |
| Tig-Acy-1 | OH | OH | OH | OH | O-Tig | O-Acy | strong |
| Tig-Acy-2 | O-Acy | OH | OH | OH | O-Tig | O-Acy | moderate |
| Tig-Acy-3 | OH | O-Acy | OH | OH | O-Tig | O-Acy | moderate |
| Tig-Acy-4 | OH | OH | O-Acy | OH | O-Tig | O-Acy | moderate |
| Tig-Acy-5 | O-Acy | OH | OH | OH | O-Tig | OH | moderate |
| Tig-Acy-6 | OH | O-Acy | OH | OH | O-Tig | OH | moderate |

Esterification of E4A-Tig-N with 4-Pentenoyl chloride and isolation of the compounds with HPLC give the following compounds:

| | R1 | R2 | R5 | R8 | R17 | R18 | Cytotoxicity activity |
|---|---|---|---|---|---|---|---|
| E4A-Tig-N | OH | OH | OH | OH | O-Tig | OH | moderate |
| Tig-Pen-1 | OH | OH | OH | OH | O-Tig | O-Pen | strong |
| Tig-Pen-2 | O-Pen | OH | OH | OH | O-Tig | O-Pen | moderate |
| Tig-Pen-3 | OH | O-Pen | OH | OH | O-Tig | O-Pen | moderate |
| Tig-Pen-4 | OH | OH | O-Pen | OH | O-Tig | O-Pen | moderate |
| Tig-Pen-5 | O-Pen | OH | OH | OH | O-Tig | OH | moderate |
| Tig-Pen-6 | OH | O-Pen | OH | OH | O-Tig | OH | moderate |

Esterification of E4A-Tig-N with Hexanoly chloride and isolation of the compounds with HPLC give the following compounds:

| | R1 | R2 | R5 | R8 | R17 | R18 | Cytotoxicity activity |
|---|---|---|---|---|---|---|---|
| E4A-Tig-N | OH | OH | OH | OH | O-Tig | OH | moderate |
| Tig-Hex-1 | OH | OH | OH | OH | O-Tig | O-Hex | strong |
| Tig-Hex-2 | O-Hex | OH | OH | OH | O-Tig | O-Hex | moderate |
| Tig-Hex-3 | OH | O-Hex | OH | OH | O-Tig | O-Hex | moderate |
| Tig-Hex-4 | OH | OH | O-Hex | OH | O-Tig | O-Hex | moderate |
| Tig-Hex-5 | O-Hex | OH | OH | OH | O-Tig | OH | moderate |
| Tig-Hex-6 | OH | O-Hex | OH | OH | O-Tig | OH | moderate |

Esterification of E4A-Tig-N with Cinnamoyl chloride and isolation of the compounds with HPLC give the following compounds:

| | R1 | R2 | R5 | R8 | R17 | R18 | Cytotoxicity activity |
|---|---|---|---|---|---|---|---|
| E4A-Tig-N | OH | OH | OH | OH | O-Tig | OH | moderate |
| Tig-Cin-1 | OH | OH | OH | OH | O-Tig | O-Cin | strong |
| Tig-Cin-2 | O-Cin | OH | OH | OH | O-Tig | O-Cin | moderate |
| Tig-Cin-3 | OH | O-Cin | OH | OH | O-Tig | O-Cin | moderate |
| Tig-Cin-4 | OH | OH | O-Cin | OH | O-Tig | O-Cin | moderate |
| Tig-Cin-5 | O-Cin | OH | OH | OH | O-Tig | OH | moderate |
| Tig-Cin-6 | OH | O-Cin | OH | OH | O-Tig | OH | moderate |

Esterification of E4A-Tig-N with Angeloyl chloride and isolation of the compounds with HPLC give the following compounds:

| | R1 | R2 | R5 | R8 | R17 | R18 | Cytotoxicity activity |
|---|---|---|---|---|---|---|---|
| E4A-Tig-N | OH | OH | OH | OH | O-Tig | OH | moderate |
| Tig-Ang-1 | OH | OH | OH | OH | O-Tig | O-Ang | strong |
| Tig-Ang-2 | O-Ang | OH | OH | OH | O-Tig | O-Ang | moderate |
| Tig-Ang-3 | OH | O-Ang | OH | OH | O-Tig | O-Ang | moderate |
| Tig-Ang-4 | OH | OH | O-Ang | OH | O-Tig | O-Ang | moderate |
| Tig-Ang-5 | O-Ang | OH | OH | OH | O-Tig | OH | moderate |
| Tig-Ang-6 | OH | O-Ang | OH | OH | O-Tig | OH | moderate |

Esterification of E4A-Tig-N with 2-Ethylbutyryl chloride and isolation of the compounds with HPLC give the following compounds:

| | R1 | R2 | R5 | R8 | R17 | R18 | Cytotoxicity activity |
|---|---|---|---|---|---|---|---|
| E4A-Tig-N | OH | OH | OH | OH | O-Tig | OH | moderate |
| Tig-Eth-1 | OH | OH | OH | OH | O-Tig | O-Eth | strong |
| Tig-Eth-2 | O-Eth | OH | OH | OH | O-Tig | O-Eth | moderate |
| Tig-Eth-3 | OH | O-Eth | OH | OH | O-Tig | O-Eth | moderate |
| Tig-Eth-4 | OH | OH | O-Eth | OH | O-Tig | O-Eth | moderate |
| Tig-Eth-5 | O-Eth | OH | OH | OH | O-Tig | OH | moderate |
| Tig-Eth-6 | OH | O-Eth | OH | OH | O-Tig | OH | moderate |

Esterification of compound (A), (B), (C), (D), (E), (F), (G), (H) with acyl chloride including Tigloyl chloride, angeloyl chloride, Acetyl chloride, Crotonoyl chloride, 3,3-Dimethylartyloyl chloride, senecioyl chloride, Cinnamoyl chloride, Pentenoyl chloride, Hexanoyl chloride, benzoyl chloride or Ethylbutyryl chloride The compounds vary in composition when the time or temperature of the reaction is changed. The peaks, fractions and compounds are selected according to the activities of times studies and the changes of peaks. The compounds having strong to weak activities are selected and isolated. The anti cancer activities are the MTT studies of bone (U2OS), lung (H460), bladder(HTB-9), ovary (ES2), colon (HCT116), pancreas (Capan), ovary(OVCAR3), prostate (DU145), skin (SK-Mel-5), mouth (KB), kidney (A498), breast (MCF-7), liver (HepG2), brain (T98G), luekemia (K562), cervix (HeLa). The active esterification products are purified with HPLC. The reaction product of mixtures and individual compounds are tested with MTT Cytotoxic Assay. Details of method are in Experiment 3 of the present application. A second esterification of compound can be selected from the above experiment results to produce new active compounds. A partial esterification compound is selected from the above experiments to perform a second or repeated with a third esterification with different acyl chloride in order to produce new active compounds with the experiments in the present application.

A method is 1) Dissolving core compound or triterpenes core, hydroxylated triterpenes core in pyridine; 2) Adding acyl chloride; 3, The mixture is stirred for length of time including 5 sec, 1 min, 2 min, 5 min, 10 min, 30 min, 1 hr, 2 hr, 18 hr, 2 days or 3 days at different temperature; 4) At the end of reaction, aqueous solution of acid or weak base, or water is added to the reaction mixture; 5) The solution is then extracted of ethyl acetate and lyophilization; 6) Dissolving the reaction product in acetonitrile with Trifluoroacetic acid or DMSO; 7) Testing the reaction product of mixtures and individual fractions with MTT cytotoxic assay; 8) Selecting the HPLC fractions for isolation is according to the cytotoxic activity of the reaction product obtained at a specific reaction time; 10) Purifiing the active esterification products with HPLC; 11) Collecting the products; 12) Testing the products; wherein the core compound is terpene, isoprene, or triterpene core or hydroxylated triterpenes core; wherein the core compound was dissolved in pyridine; wherein the acyl chloride including Tigloyl chloride, angeloyl chloride, Acetyl chloride, Crotonoyl chloride, 3,3-Dimethylartyloyl chloride, senecioyl chloride, Cinnamoyl chloride, Pentenoyl chloride, Hexanoyl chloride, benzoyl chloride and Ethylbutyryl chloride; wherein the reaction time for the mixture is stirred for 5 sec, 1 min, 2 min, 5 min, 10 min, 30 min, 1 hr, 2 hr, 18 hr, 2 days or 3 days; wherein the temperature is 0C, 25C, 50 or 75C temperature; wherein the acid including HCl or the base including NaHCO3 is added to the reaction mixture; wherein the solution is then extracted 3 times with ethyl acetate and lyophilization; wherein the reaction product is dissolved in 80% acetonitrile - 0.005% Trifluoroacetic acid or DMSO; wherein selecting the HPLC fractions for isolation is according to the cytotoxic activity of the reaction product obtained at a reaction time of 5 sec, 1 min, 2 min, 5 min, 10 min, 30 min, 1 hr, 2 hr, 18 hr, 2 days or 3 days. In an embodiment, the reaction time may be ove 3 days. In an embodiment, the experiment may be performed under 0C. In an embodiment, the experiment may be performed over 75C.

The anti cancer activities of Tig-R compound: IC50 of bone (U2OS) is 4.5 ug/ml, lung (H460) is 4.8 ug/ml, bladder(HTB-9) is 2.5 ug/ml, ovary (ES2) is 2.8 ug/ml, colon (HCT116) is 5.2 ug/ml, pancreas (Capan) 2.4 ug/ml, ovary(OVCAR3) is 5.8, prostate (DU145) is 3.6 ug/ml, skin (SK-Mel-5) is 5.1ug/ml, mouth (KB) is 3 ug/ml, kidney (A498) is 3.5 ug/ml, breast (MCF-7) is 4.5 ug/ml, liver (HepG2) is 6 ug/ml, brain (T98G) is 8 ug/ml), leukemia (K562) is 2 ug/ml, cervix (HeLa) is 5 ug/ml.

The anti cancer activities of Tig-V compound: IC50 of bone (U2OS) is 7 ug/ml, lung (H460) is 6.8 ug/ml, bladder(HTB-9) is 4 ug/ml, ovary (ES2) is 2 ug/ml, colon (HCT116) is 8 ug/ml, pancreas (Capan) 5 ug/ml, ovary(OVCAR3) is 9, prostate (DU145) is 4 ug/ml, skin (SK-Mel-5) is 6ug/ml, mouth (KB) is 4.5 ug/ml, kidney (A498) is 4.8 ug/ml, breast (MCF-7) is 9 ug/ml, liver (HepG2) is 12 ug/ml, brain (T98G) is 14 ug/ml), leukemia (K562) is 4 ug/ml, cervix (HeLa) is 7 ug/ml.

The anti cancer activities of Tig-N compound: IC50 of bone (U2OS) is 15 ug/ml, lung (H460) is 13 ug/ml, bladder(HTB-9) is 7.5 ug/ml, ovary (ES2) is 9 ug/ml, colon (HCT116) is 15 ug/ml, pancreas (Capan) 8 ug/ml, ovary(OVCAR3) is 18, prostate (DU145) is 4.8 ug/ml, skin (SK-Mel-5) is 15 ug/ml, mouth (KB) is 9 ug/ml, kidney (A498) is 11 ug/ml, breast (MCF-7) is 13 ug/ml, liver (HepG2) is 18 ug/ml, brain (T98G) is 19 ug/ml), leukemia (K562) is 6 ug/ml, cervix (HeLa) is 15 ug/ml.

The anti cancer activities of Tig-Q compound: IC50 of bone (U2OS) is 20 ug/ml, lung (H460) is 18 ug/ml, bladder(HTB-9) is 10 ug/ml, ovary (ES2) is 12 ug/ml, colon (HCT116) is 22 ug/ml, pancreas (Capan) 9 ug/ml, ovary(OVCAR3) is 23, prostate (DU145) is 15 ug/ml, skin (SK-Mel-5) is 20ug/ml, mouth (KB) is 12 ug/ml, kidney (A498) is 13 ug/ml, breast (MCF-7) is 18 ug/ml, liver (HepG2) is 24 ug/ml, brain (T98G) is 29 ug/ml), leukemia (K562) is 6 ug/ml, cervix (HeLa) is 20 ug/ml.

The anti cancer activities of Tig-T compound: IC50 of bone (U2OS) is 20 ug/ml, lung (H460) is 21 ug/ml, bladder (HTB-9) is 12 ug/ml, ovary (ES2) is 14 ug/ml, colon (HCT116) is 23 ug/ml, pancreas (Capan) 10 ug/ml, ovary(OVCAR3) is 25, prostate (DU145) is 16 ug/ml, skin (SK-Mel-5) is 22ug/ml, mouth (KB) is 13 ug/ml, kidney (A498) is 15 ug/ml, breast (MCF-7) is 20 ug/ml, liver (HepG2) is 26 ug/ml, brain (T98G) is 26 ug/ml), leukemia (K562) is 9 ug/ml, cervix (HeLa) is 18 ug/ml.

The anti cancer activities of Tig-S compound: IC50 of bone (U2OS) is 5.2 ug/ml, lung (H460) is 5.6 ug/ml, bladder(HTB-9) is 3.5 ug/ml, ovary (ES2) is 4.2 ug/ml, colon (HCT116) is 6.6 ug/ml, pancreas (Capan) 2.9 ug/ml, ovary(OVCAR3) is 6.5, prostate (DU145) is 4.3 ug/ml, skin (SK-Mel-5) is 5.8ug/ml, mouth (KB) is 4 ug/ml, kidney (A498) is 4.8 ug/ml, breast (MCF-7) is 6.3 ug/ml, liver (HepG2) is 8.5 ug/ml, brain (T98G) is 9 ug/ml), leukemia (K562) is 4.3 ug/ml, cervix (HeLa) is 7 ug/ml.

The anti cancer activities of Tig-U compound: IC50 of bone (U2OS) is 23 ug/ml, lung (H460) is 19 ug/ml, bladder(HTB-9) is 15 ug/ml, ovary (ES2) is 17 ug/ml, colon (HCT116) is 26 ug/ml, pancreas (Capan) 9 ug/ml, ovary(OVCAR3) is 27, prostate (DU145) is 15 ug/ml, skin (SK-Mel-5) is 24ug/ml, mouth (KB) is 16 ug/ml, kidney (A498) is 18 ug/ml, breast (MCF-7) is 25 ug/ml, liver (HepG2) is 23 ug/ml, brain (T98G) is 22 ug/ml), leukemia (K562) is 10 ug/ml, cervix (HeLa) is 17 ug/ml.

Disclosed herein are compounds, methods, or uses of a compound for the manufacture of a medicament, or uses of a compound for medicament selected from formula (2A), for treating cancer, inhibiting cancer growth, inhibiting cancer invasion, inhibiting cancer metastasis, modulating cell adhesion, modulating cell attachment, using compounds selected from the following: 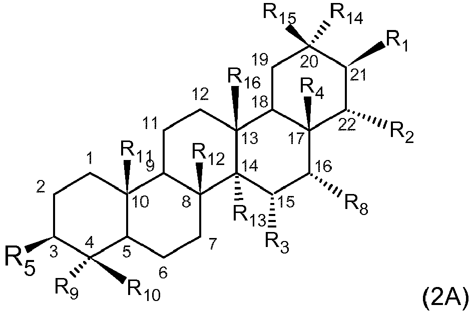
R1, R2, R3, R4, R5, R8, R9, R10, R11, R12, R13, R14, R15 are independently selected from the group of hydrogen, hydroxyl, methyl, O-angeloyl, O-tigloyl, O-senecioyl, O-acetyl, O-Crotonoyl, 0-3,3-Dimethylartyloyl, O-Cinnamoyl, O-Pentenoyl, O-Hexanoyl, O-benzoyl, O-Ethylbutyryl,,O-alkyl, O-dibenzoyl, O-benzoyl, O-alkanoyl, O-alkenoyl, O-benzoyl alkyl substituted O-alkanoyl, O-alkanoyl substituted phenyl, O-alkenoyl substituted phenyl, O-aryl, O-acyl, O-heterocylic, O-heteroraryl, O-alkenylcarbonyl, CH2O-angeloyl, CH2O-tigloyl, CH2O-senecioyl, CH2O-acetyl, CH2O-Crotonoyl, CH2O-3,3-Dimethylartyloyl, CH2O-Cinnamoyl, CH2O-Pentenoyl, CH2O-Hexanoyl, CH2O-benzoyl, CH2O-Ethylbutyryl, CH3, CH2OH, O-alkyl, O-dibenzoyl, O-benzoyl, O-alkanoyl, O-alkenoyl, O-benzoyl alkyl substituted O-alkanoyl, O-alkanoyl substituted phenyl, O-alkenoyl substituted phenyl, O-aryl, O-acyl, O-heterocylic, O-heteroraryl, O-alkenylcarbonyl,alkane, alkene and sugar moiety or derivatives thereof; wherein the structure (2A) comprises at least 2 groups selected from O-angeloyl, O-tigloyl, O-senecioyl, O-acetyl, O-Crotonoyl, 0-3,3-Dimethylartyloyl, O-Cinnamoyl, O-Pentenoyl, O-Hexanoyl, O-benzoyl, O-Ethylbutyryl, O-alkyl, O-dibenzoyl, O-benzoyl, O-alkanoyl, O-alkenoyl, O-benzoyl alkyl substituted O-alkanoyl, O-alkanoyl substituted phenyl, O-alkenoyl substituted phenyl, O-aryl, O-acyl, O-heterocylic, O-heteroraryl, O-alkenylcarbonyl; or wherein R1 and R2 are selected from O-angeloyl, O-tigloyl, O-senecioyl, O-acetyl, O-Crotonoyl, 0-3,3-Dimethylartyloyl, O-Cinnamoyl, O-Pentenoyl, O-Hexanoyl, O-benzoyl, O-Ethylbutyryl, O-alkyl, O-dibenzoyl, O-benzoyl, O-alkanoyl, O-alkenoyl, O-benzoyl alkyl substituted O-alkanoyl, O-alkanoyl substituted phenyl, O-alkenoyl substituted phenyl, O-aryl, O-acyl, O-heterocylic, O-heteroraryl, O-alkenylcarbonyl; In an embodiment, wherein the R1 and R2 are attached OH. In an embodiment, wherein R4, R10 are attached a CH2O-angeloyl, CH2O-tigloyl, CH2O-senecioyl, CH2O-acetyl, CH2O-Crotonoyl, CH2O-3,3-Dimethylartyloyl, CH2O-Cinnamoyl, CH2O-Pentenoyl, CH2O-Hexanoyl, CH2O-benzoyl, or CH2O-Ethylbutyryl. In an embodiment, wherein the R3 and R8 is hydrogen or hydroxyl, In an embodiment, wherein the R9, R11, R12, R13, R14, R15 are independently attached with a methyl. In an embodiment, wherein R4 represents CH3, CHO, CH₂R6 or COR6, wherein R6 is selected from hydroxyl, O-angeloyl, O-tigloyl, O-senecioyl, O-acetyl, O-Crotonoyl, O-3,3-Dimethylartyloyl, O-Cinnamoyl, O-Pentenoyl, O-Hexanoyl, O-Ethylbutyryl, O-alkyl, O-dibenzoyl, O-benzoyl, O-alkanoyl, O-alkenoyl, O-benzoyl alkyl substituted O-alkanoyl, O-alkanoyl substituted phenyl, O-alkenoyl substituted phenyl, O-aryl, O-acyl, O-heterocylic, O-heteroraryl, O-alkenylcarbonyl and derivatives thereof; In an embodiment, wherein R3 is H or OH; In an embodiment, wherein R8 is H or OH; In an embodiment, wherein R16 is H, CH3, OH ,or R4 and R16 may together form -CH2-X-, CH(OH)-X- or C(=O)-X-, wherein the -X- may be O or NH or S; wherein when the C12-13 of ring 3 of the triterpene has a double bond then R16 is absent. In an embodiment, wherein R10 represents CH3, CHO, or CH₂R6, wherein R6 is selected from hydroxyl, O-angeloyl, O-tigloyl, O-senecioyl, O-acetyl, O-Crotonoyl, 0-3,3-Dimethylartyloyl, O-Cinnamoyl, O-Pentenoyl, O-Hexanoyl, O-benzoyl, O-Ethylbutyryl, O-alkyl, O-dibenzoyl, O-benzoyl, O-alkanoyl, O-alkenoyl, O-benzoyl alkyl substituted O-alkanoyl, O-alkanoyl substituted phenyl, O-alkenoyl substituted phenyl, O-aryl, O-acyl, O-heterocylic, O-heteroraryl, O-alkenylcarbonyl and derivatives thereof;
Disclosed herein are compounds wherein R5 is a hydrogen, hydroxyl, heterocyclic or O-sugar moiety(ies), wherein the sugar moiety(ies) is/are selected from a group consisting of glucose, galactose, rhamnose, arabinose, xylose, fucose, allose, altrose, gulose, idose, lyxose, mannose, psicose, ribose, sorbose, tagatose, talose, fructose, alduronic acid, glucuronic acid, galacturonic acid, and derivatives or combinations thereof; wherein R9, R10, R11, R12, R13, R14, R15 are independently attached a group selecting from CH₃, CH₂OH, CHO, COOH, COO-alkyl, COO-aryl, COO-heterocyclic, COO-heteroaryl, CH₂Oaryl, CH₂O- heterocyclic, CH₂O- heteroaryl, alkyls group, hydroxyl, acetyl group; wherein R4 and R16 form a divalent radical of formula CH2O, CH(OR7)O, or COOR7, wherein R7 is hydrogen, alkyl, angeloyl, tigloyl, senecioyl, dibenzoyl, benzoyl, alkanoyl, alkenoyl, benzoyl alkyl substituted alkanoyl, aryl, acyl, heterocylic, heteroraryl, and derivatives thereof; wherein at least two of R1, R2 and R6 are attached a group selected from O-angeloyl, O-tigloyl, O-senecioyl, O-acetyl, O-Crotonoyl, O-3,3-Dimethylartyloyl, O-Cinnamoyl, O-Pentenoyl, O-Hexanoyl, O-benzoyl, O-Ethylbutyryl, O-dibenzoyl, O-benzoyl, O-alkanoyl, O-alkenoyl, O-benzoyl alkyl substituted O-alkanoyl, O-aryl, O-acyl, O-heterocylic, O-heteroraryl, and derivatives thereof; or at least one of R1, R2, and R4 is a sugar moiety having at least two groups selected from a group consisting of angeloyl, acetyl, tigloyl, senecioyl, Crotonoyl, 3,3-Dimethylartyloyl, Cinnamoyl, Pentenoyl, Hexanoyl, benzoyl, Ethylbutyryl, benzoyl, dibenzoyl, alkanoyl, alkenoyl, benzoyl alkyl substituted alkanoyl, aryl, acyl, heterocylic, heteroraryl, and their derivatives thereof; or wherein R4 represents CH₂R6, wherein R6 is selected from hydroxyl, O-angeloyl, O-tigloyl, O-senecioyl, O-acetyl, O-Crotonoyl, O-3,3-Dimethylartyloyl, O-Cinnamoyl, O-Pentenoyl, O-Hexanoyl, O-Ethylbutyryl, O-alkyl, O-dibenzoyl, O-benzoyl, O-alkanoyl, O-alkenoyl, O-benzoyl alkyl substituted O-alkanoyl, O-alkanoyl substituted phenyl, O-alkenoyl substituted phenyl, O-aryl, O-acyl, O-heterocylic, O-heteroraryl, O-alkenylcarbonyl and derivatives thereof; wherein R5 is/are the sugar moiety(ies) selected from the following sugars and alduronic acids: glucose, galactose, rhamnose, arabinose, xylose, fucose, allose, altrose, gulose, idose, lyxose, mannose, psicose, ribose, sorbose, tagatose, talose, fructose, glucuronic acid, galacturonic acid; or their derivatives thereof, In an embodiment, wherein R5 is a hydroxyl, O-angeloyl, O-tigloyl, O-senecioyl, O-acetyl, O-Crotonoyl, O-3,3-Dimethylartyloyl, O-Cinnamoyl, O-Pentenoyl, O-Hexanoyl, O-benzoyl, O-Ethylbutyryl, O-alkyl, O-dibenzoyl, O-benzoyl, O-alkanoyl, O-alkenoyl, O-benzoyl alkyl substituted O-alkanoyl, O-alkanoyl substituted phenyl, O-alkenoyl substituted phenyl, O-aryl, O-acyl, O-heterocylic, O-heteroraryl, O-alkenylcarbonyl and derivatives thereof. In an embodiment, R1, R2, R3, R4, R5, R8, R9, R10, R11, R12, R13, R14 or R15 comprise of one or more sugar moieties. In an embodiment, R1, R2, R3, R4, R5, R8, R9, R10, R11, R12, R13, R14 or R15 comprise of one or more acids. In an embodiment, at least 1, or 2, or 3, or 4 of R1, R2, R3, R4, R5, R8, R9, R10, R11, R12, R13, R14 and R15 is hydroxyl. In an embodiment, at least 2, or 3, or 4, or 5, or 6, or 7 of R1, R2, R3, R4, R5, R8, R9, R10, R11, R12, R13, R14 and R15 are independently attached a group selected from the group of O-acetyl, O-angeloyl, O-tigloyl, O-senecioyl, O-acetyl, O-Crotonoyl, 0-3,3-Dimethylartyloyl, O-Cinnamoyl, O-Pentenoyl, O-Hexanoyl, O-benzoyl, O-Ethylbutyryl, O-alkyl, O-dibenzoyl, O-benzoyl, O-alkanoyl, O-alkenoyl, O-benzoyl alkyl substituted O-alkanoyl, O-alkanoyl substituted phenyl, O-alkenoyl substituted phenyl, O-aryl, O-acyl, O-heterocylic, O-heteroraryl, O-alkenylcarbonyl, alkane, alkene and derivatives thereof, wherein the group is attached to the triterpene directly or by connecting moiety(ies); In an embodiment, at least 1 or 2, or 3, or 4, or 5, or 6, or 7 of R1, R2, R3, R4, R5, R8 and R10 are independently attached a group selected from the group of O-angeloyl, O-tigloyl, O-senecioyl, O-acetyl, O-Crotonoyl, O-3,3-Dimethylartyloyl, O-Cinnamoyl, O-Pentenoyl, O-Hexanoyl, O-benzoyl, O-Ethylbutyryl, O-alkyl, O-dibenzoyl, O-benzoyl, O-alkanoyl, O-alkenoyl, O-benzoyl alkyl substituted O-alkanoyl, O-alkanoyl substituted phenyl, O-alkenoyl substituted phenyl, O-aryl, O-acyl, O-heterocylic, O-heteroraryl, O-alkenylcarbonyl, CH2O-angeloyl, CH2O-tigloyl, CH2O-senecioyl, CH2O-acetyl, CH2O-Crotonoyl, CH2O-3,3-Dimethylartyloyl, CH2O-Cinnamoyl, CH2O-Pentenoyl, CH2O-Hexanoyl, CH2O-benzoyl, CH2O-Ethylbutyryl, CH3, CH2OH, O-alkyl, O-dibenzoyl, O-benzoyl, O-alkanoyl, O-alkenoyl, O-benzoyl alkyl substituted O-alkanoyl, O-alkanoyl substituted phenyl, O-alkenoyl substituted phenyl, O-aryl, O-acyl, O-heterocylic, O-heteroraryl, O-alkenylcarbonyl, and derivatives thereof, wherein the group is attached to the triterpene directly or by connecting moiety(ies). In an embodiment, the cancers comprise breast cancer, leukocytic cancer, liver cancer, ovarian cancer, bladder cancer, prostatic cancer, skin cancer, bone cancer, brain cancer, leukemia cancer, lung cancer, colon cancer, CNS cancer, melanoma cancer, renal cancer, cervical cancer, esophageal cancer, testicular cancer, spleenic cancer, kidney cancer, lymphhatic cancer, pancreatic cancer, stomach cancer and thyroid cancer; wherein the cells comprise breast cell, leukocytic cell, liver cell, ovarian cell, bladder cell, prostatic cell, skin cell, bone cell, brain cell, leukemia cell, lung cell, colon cell, CNS cell, melanoma cell, renal cell, cervical cell, esophageal cell, testicular cell, spleenic cell, kidney cell, lymphhatic cell, pancreatic cell, stomach cell and thyroid cell.

Disclosed herein is also the compound selected from the structure: 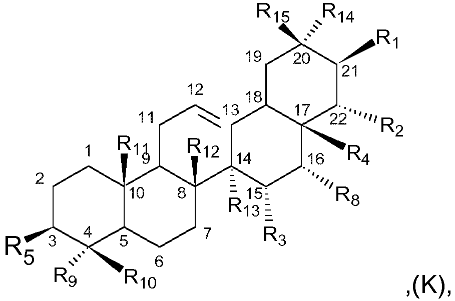
R1, R2, R3, R4, R5, R8, R9, R10, R11, R12, R13, R14, R15 are independently selected from the group of CH3, CH2OH ,hydrogen, hydroxyl, methyl, O-angeloyl, O-tigloyl, O-senecioyl, O-acetyl, O-Crotonoyl, O-3,3-Dimethylartyloyl, O-Cinnamoyl, O-Pentenoyl, O-Hexanoyl, O-benzoyl, O-Ethylbutyryl, O-alkyl, O-dibenzoyl, O-benzoyl, O-alkanoyl, O-alkenoyl, O-benzoyl alkyl substituted O-alkanoyl, O-alkanoyl substituted phenyl, O-alkenoyl substituted phenyl, O-aryl, O-acyl, O-heterocylic, O-heteroraryl, O-alkenylcarbonyl, CH2O-angeloyl, CH2O-tigloyl, CH2O-senecioyl, CH2O-acetyl, CH2O-Crotonoyl, CH2O-3,3-Dimethylartyloyl, CH2O-Cinnamoyl, CH2O-Pentenoyl, CH2O-Hexanoyl, CH2O-benzoyl, CH2O-Ethylbutyryl, CH2O-alkyl, CH2O-dibenzoyl, CH2O-benzoyl, CH2O-alkanoyl, CH2O-alkenoyl, CH2O-benzoyl alkyl substituted O-alkanoyl, CH2O-alkanoyl substituted phenyl, CH2O-alkenoyl substituted phenyl, CH2O-aryl, CH2O-acyl, CH2O-heterocylic, CH2O-heteroraryl, CH2O-alkenylcarbonyl,alkane, alkene and sugar moiety or derivatives thereof; or
wherein any 1 or 2 or 3 or 4 of R1, R2, R3, R4, R5, R8 and R10 are independently attached an O-angeloyl, O-tigloyl, O-senecioyl, O-acetyl, O-Crotonoyl, O-3,3-Dimethylartyloyl, O-Cinnamoyl, O-Pentenoyl, O-Hexanoyl, O-benzoyl, O-Ethylbutyryl, O-alkyl, O-dibenzoyl, O-benzoyl, O-alkanoyl, O-alkenoyl, O-benzoyl alkyl substituted O-alkanoyl, O-alkanoyl substituted phenyl, O-alkenoyl substituted phenyl, O-aryl, O-acyl, O-heterocylic, O-heteroraryl, O-alkenylcarbonyl, CH2O-angeloyl, CH2O-tigloyl, CH2O-senecioyl, CH2O-acetyl, CH2O-Crotonoyl, CH2O-3,3-Dimethylartyloyl, CH2O-Cinnamoyl, CH2O-Pentenoyl, CH2O-Hexanoyl, CH2O-benzoyl, CH2O-Ethylbutyryl; R9, R11, R12, R13, R14, R15 are independently attached a CH3; or wherein R10 is attached an O-angeloyl, O-tigloyl, O-senecioyl, O-acetyl, O-Crotonoyl, O-3,3-Dimethylartyloyl, O-Cinnamoyl, O-Pentenoyl, O-Hexanoyl, O-benzoyl, O-Ethylbutyryl, O-alkyl, O-dibenzoyl, O-benzoyl, O-alkanoyl, O-alkenoyl, O-benzoyl alkyl substituted O-alkanoyl, O-alkanoyl substituted phenyl, O-alkenoyl substituted phenyl, O-aryl, O-acyl, O-heterocylic, O-heteroraryl, O-alkenylcarbonyl, CH2O-angeloyl, CH2O-tigloyl, CH2O-senecioyl, CH2O-acetyl, CH2O-Crotonoyl, CH2O-3,3-Dimethylartyloyl, CH2O-Cinnamoyl, CH2O-Pentenoyl, CH2O-Hexanoyl, CH2O-benzoyl, CH2O-Ethylbutyryl, CH2O-alkyl, CH2O-dibenzoyl, CH2O-benzoyl, CH2O-alkanoyl, CH2O-alkenoyl, CH2O-benzoyl alkyl substituted O-alkanoyl, CH2O-alkanoyl substituted phenyl, CH2O-alkenoyl substituted phenyl, CH2O-aryl, CH2O-acyl, CH2O-heterocylic, CH2O-heteroraryl, CH2O-alkenylcarbonyl; or wherein R4 and R10 are independently attached an O-angeloyl, O-tigloyl, O-senecioyl, O-acetyl, O-Crotonoyl, O-3,3-Dimethylartyloyl, O-Cinnamoyl, O-Pentenoyl, O-Hexanoyl, O-benzoyl, O-Ethylbutyryl, O-alkyl, O-dibenzoyl, O-benzoyl, O-alkanoyl, O-alkenoyl, O-benzoyl alkyl substituted O-alkanoyl, O-alkanoyl substituted phenyl, O-alkenoyl substituted phenyl, O-aryl, O-acyl, O-heterocylic, O-heteroraryl, O-alkenylcarbonyl, CH2O-angeloyl, CH2O-tigloyl, CH2O-senecioyl, CH2O-acetyl, CH2O-Crotonoyl, CH2O-3,3-Dimethylartyloyl, CH2O-Cinnamoyl, CH2O-Pentenoyl, CH2O-Hexanoyl, CH2O-benzoyl, CH2O-Ethylbutyryl, CH2O-alkyl, CH2O-dibenzoyl, CH2O-benzoyl, CH2O-alkanoyl, CH2O-alkenoyl, CH2O-benzoyl alkyl substituted O-alkanoyl, CH2O-alkanoyl substituted phenyl, CH2O-alkenoyl substituted phenyl, CH2O-aryl, CH2O-acyl, CH2O-heterocylic, CH2O-heteroraryl, CH2O-alkenylcarbonyl; wherein R3 is OH or H or absent; wherein R1, R2, R3, R5, R8 are OH or H or absent; wherein R9, R11, R12, R13, R14, and R15 are CH3; or wherein R1, R2, R5, R8 represent OH; R3 represents OH, H or absent; R4, R10 represent CH2Oangeloyl; R9, R11, R12, R13, R14, R15 represent CH3; or wherein R1, R2, R5, R8 represent OH or O-tigloyl; R3 represents OH, H, or absent; R4, R10 represent CH2O tigloyl; R9, R11, R12, R13, R14, R15 represent CH3; wherein the group attaching to the core compound selected from acetyl, angeloyl, tigloyl, senecioyl, Crotonoyl, O-3,3-Dimethylartyloyl, Cinnamoyl, Pentenoyl, Hexanoyl, benzoyl, Ethylbutyryl, alkyl, dibenzoyl, benzoyl, methylbutanoyl, methylpropanoyl, alkanoyl, alkenoyl, benzoyl alkyl substituted alkanoyl, alkanoyl substituted phenyl, alkenoyl substituted phenyl, aryl, acyl, heterocylic, heteroraryl and alkenylcarbonyl are interchangeable. They can be the same group or in combination thereof. Substitution, deletion and/or addition of any group in the above-described compounds by other group(s) will be apparent to one of ordinary skill in the art based on the teachings of this application.

Disclosed herein is a the compound is selected from the structures:

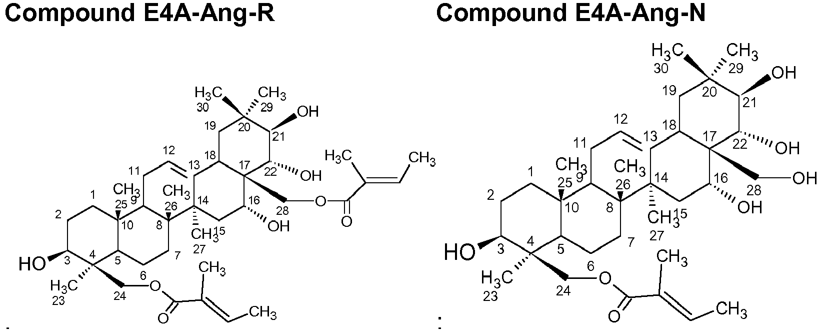

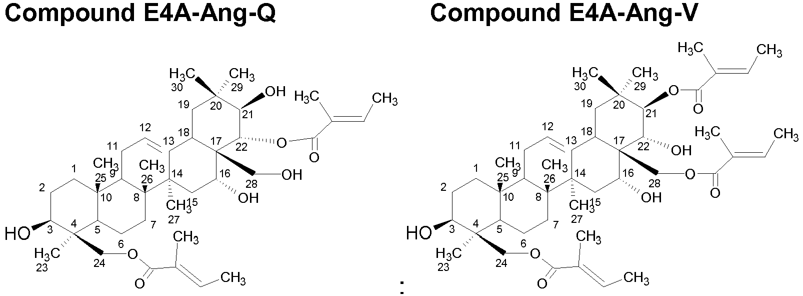

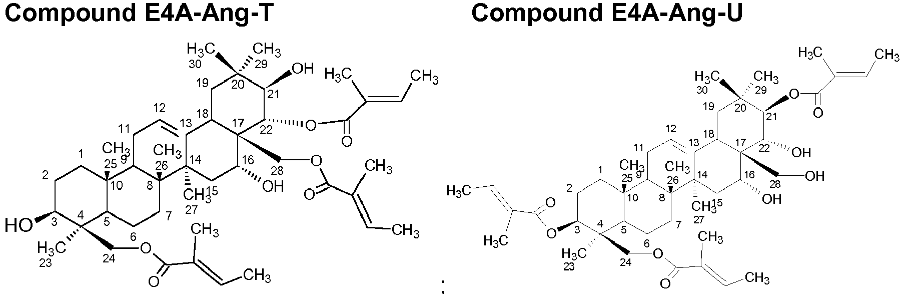

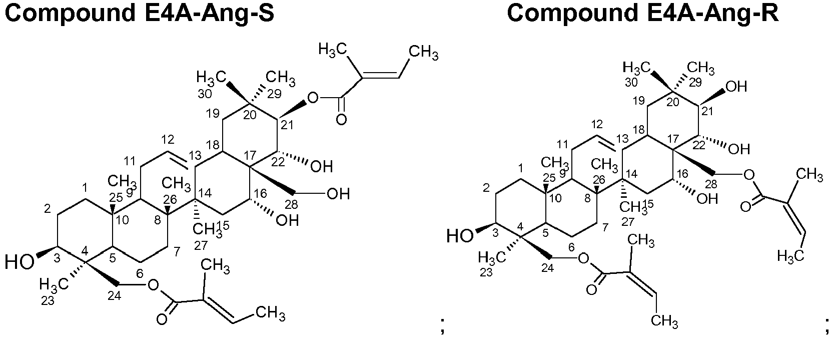

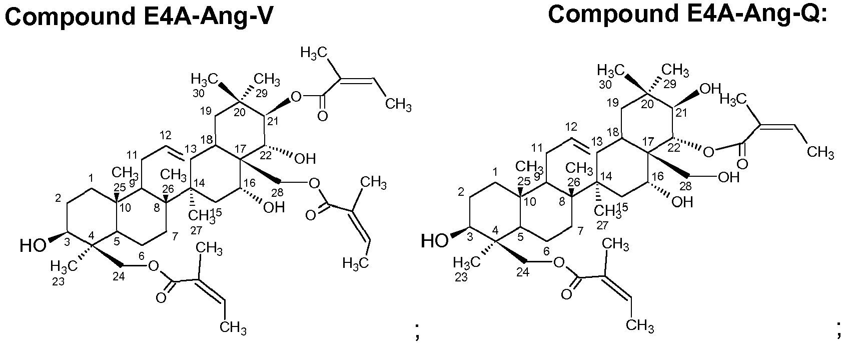

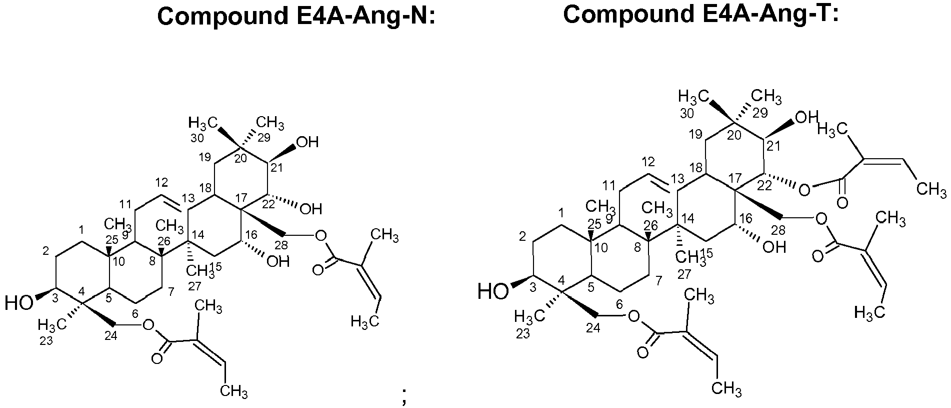

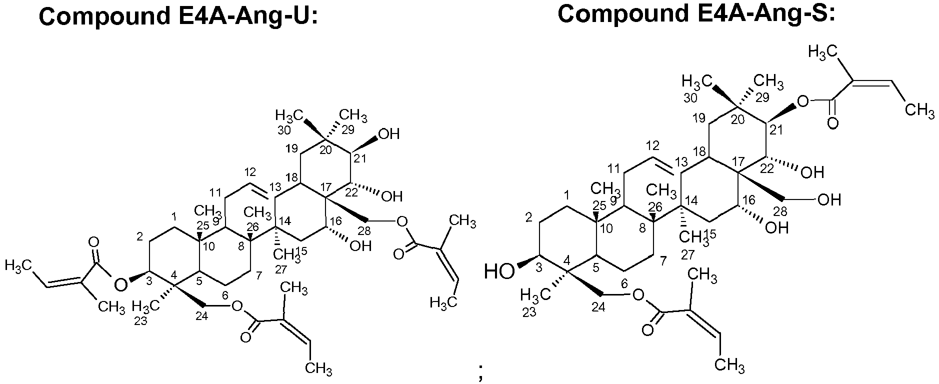

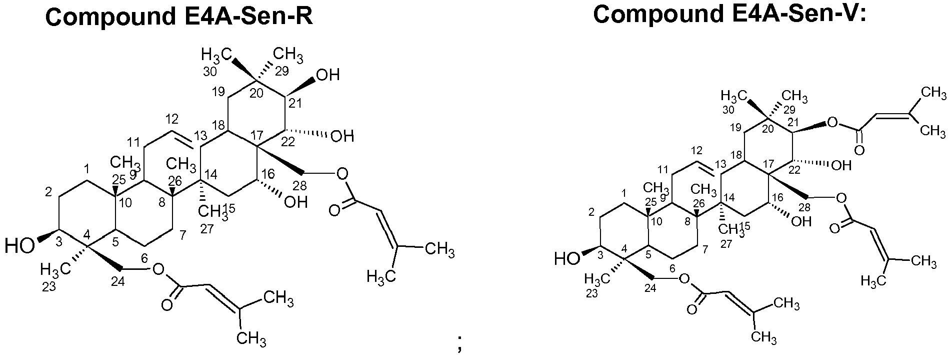

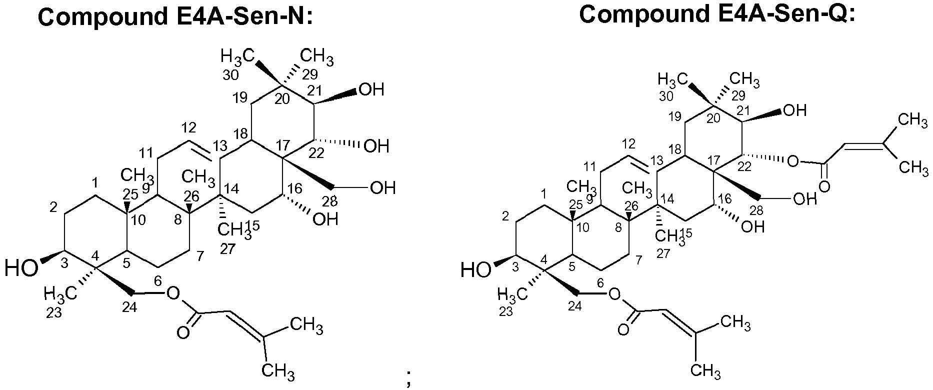

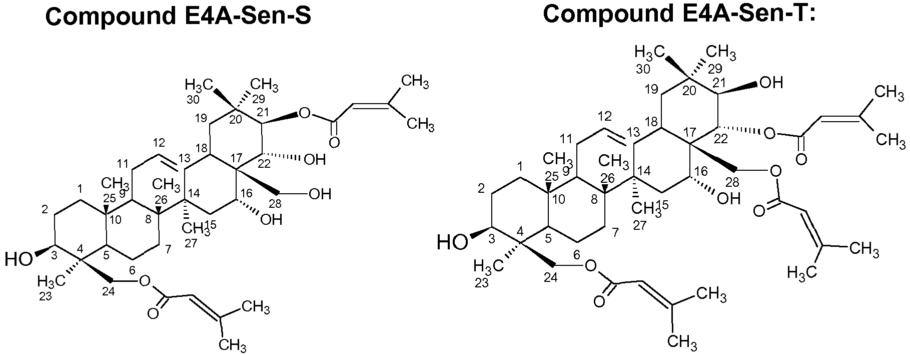

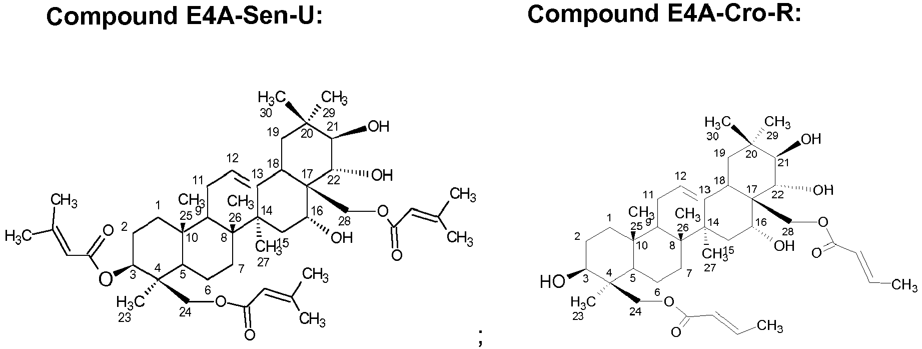

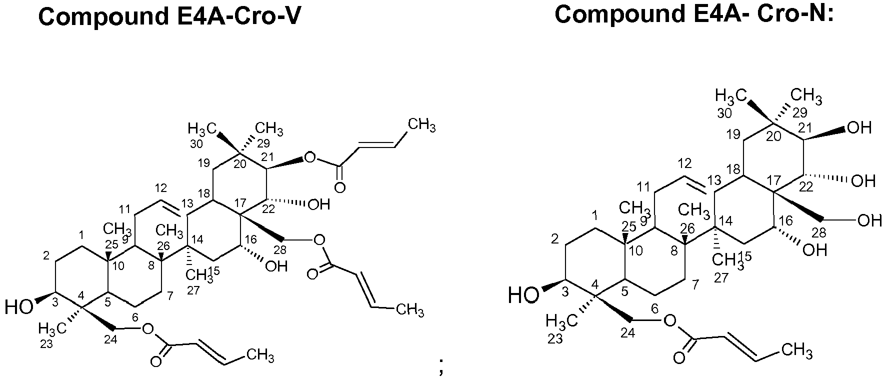

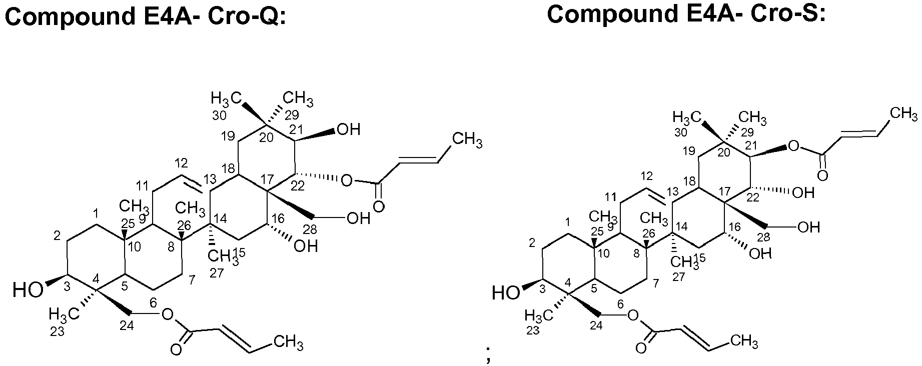

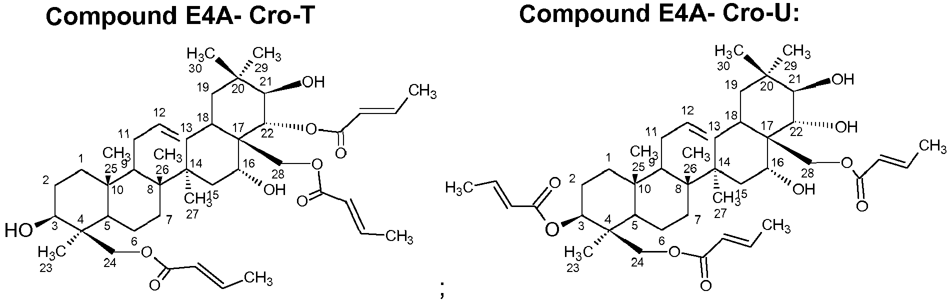

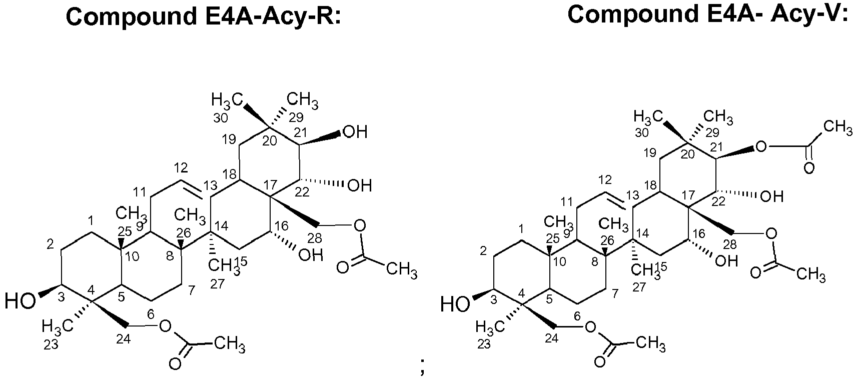

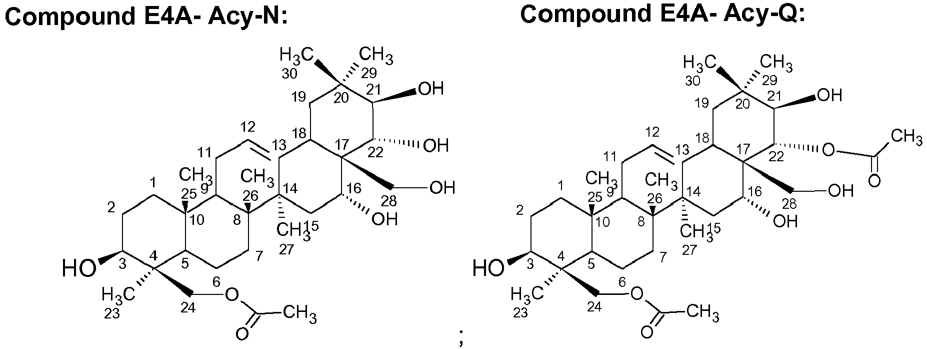

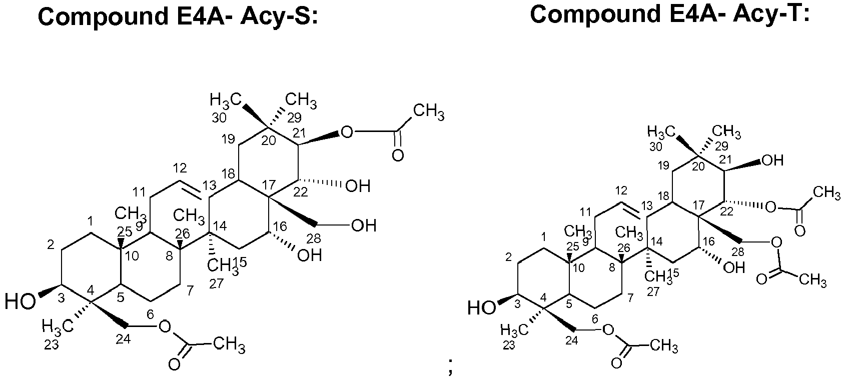

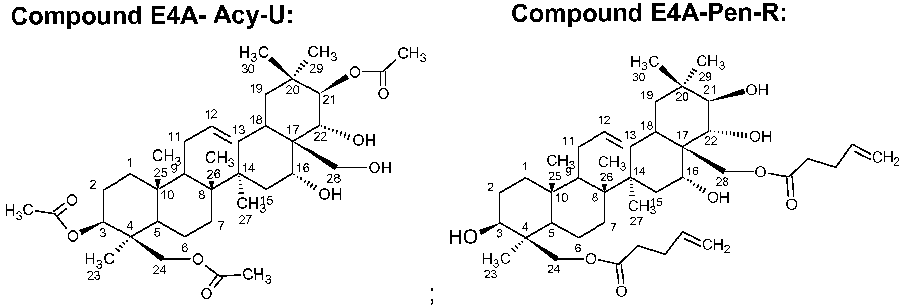

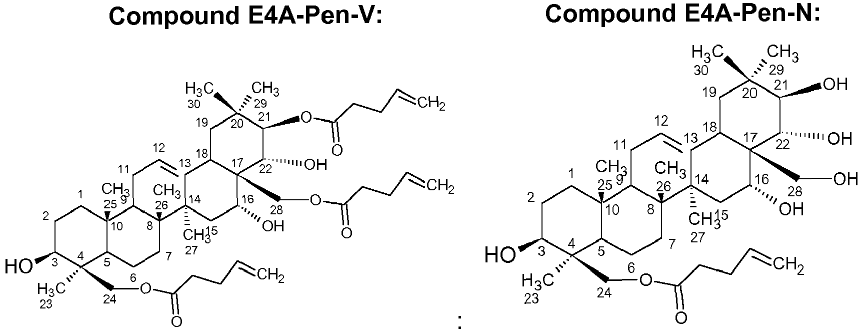

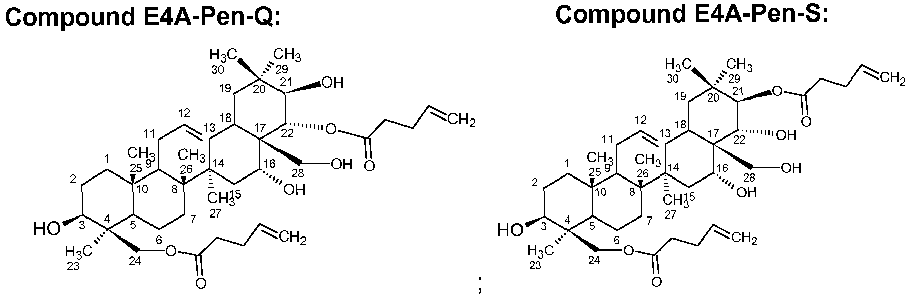

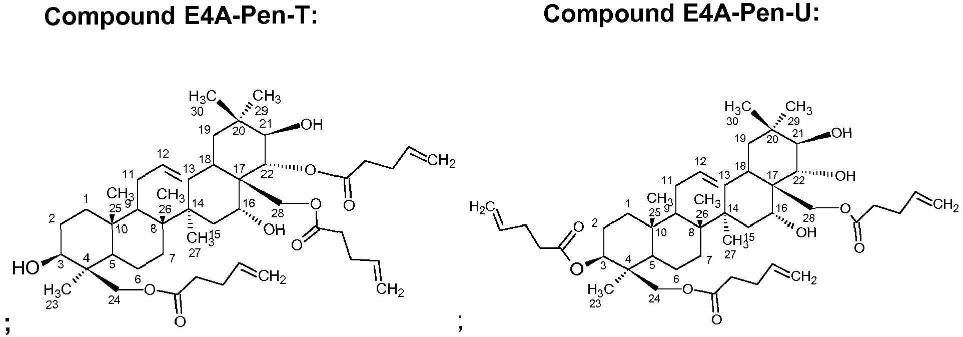

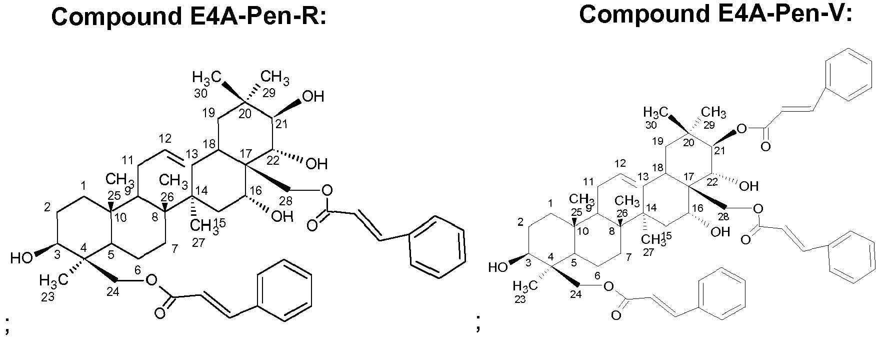

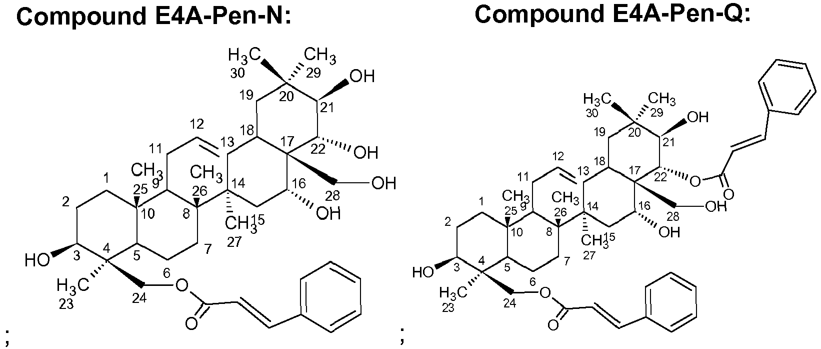

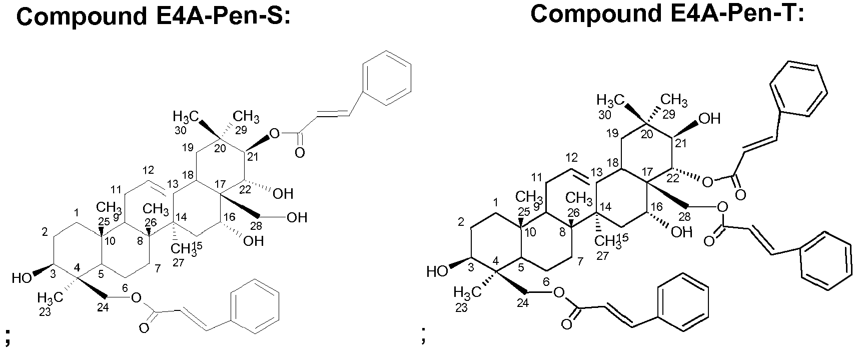

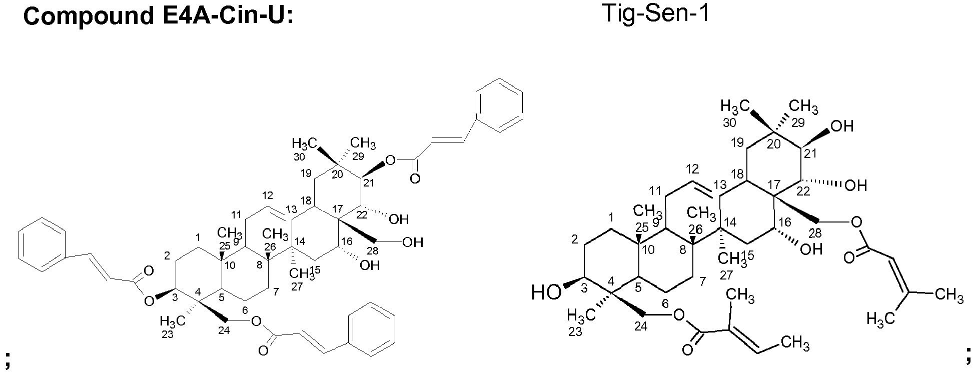

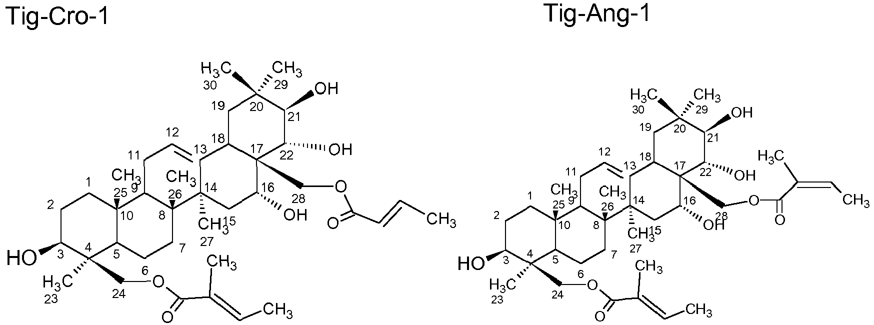

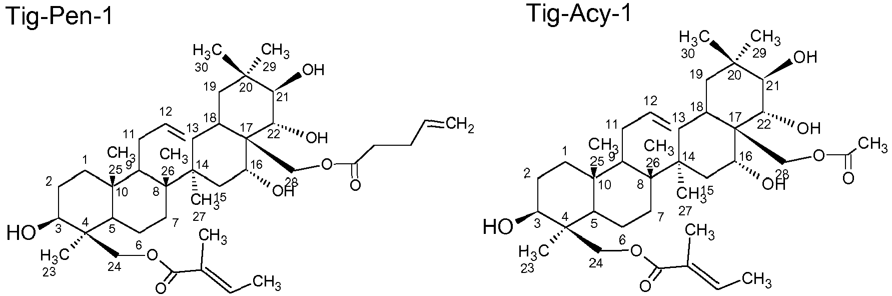

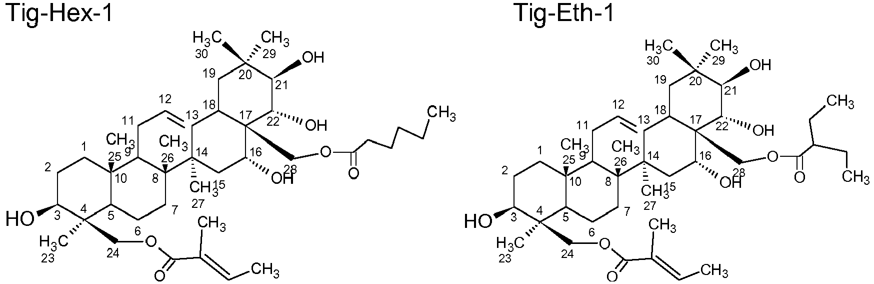

A composition comprising an effective amount of compound selected from the above formula or a salt, ester, metabolite or derivative thereof can be used as a medicament for blocking the invasion, migration, metastasis of cancer cells, inhibiting tumor or cancer cell growth and for treating cancer, wherein the cancers comprise breast cancer, leukocytic cancer, liver cancer, ovarian cancer, bladder cancer, prostatic cancer, skin cancer, bone cancer, brain cancer, leukemia cancer, lung cancer, colon cancer, CNS cancer, melanoma cancer, renal cancer, cervical cancer, esophageal cancer, testicular cancer, spleenic cancer, kidney cancer, lymphhatic cancer, pancreatic cancer, stomach cancer and thyroid cancer.

This invention provides a composition comprising the compounds specified in the claims for treating cancers; for inhibiting viruses; for preventing cerebral aging; for improving memory; improving cerebral functions; for curing enuresis, frequent micturition, urinary incontinence; dementia, Alzheimer's disease, autism, brain trauma, Parkinson's disease or other diseases caused by cerebral dysfunctions; for treating arthritis, rheumatism, poor circulation, arteriosclerosis, Raynaud's syndrome, angina pectoris, cardiac disorder, coronary heart disease, headache, dizziness, kidney disorder; cerebrovascular diseasea; inhibiting NF-Kappa B activation; for treating brain edema, severe acute respiratory syndrome, respiratory viral diseases, chronic venous insufficiency, hypertension, chronic venous disease, oedema, inflammation, hemonhoids, peripheral edema formation, varicose vein disease, flu, post traumatic edema and postoperative swelling; for inhibiting blood clots, for inhibiting ethanol absorption; for lowering blood sugar; for regulating adrenocorticotropin and corticosterone levels. This invention provides a composition for AntiMS, antianeurysm, antiasthmatic, anti-oedematous, anti-inflammatory, antibradykinic, anticapillarihemorrhagic, anticephalagic, anticervicobrachialgic, antieclamptic, antiedemic, antiencaphalitic, antiepiglottitic, antiexudative, antiflu, antifracture, antigingivitic, antihematomic, antiherpetic, antihistaminic, antihydrathritic, antimeningitic, antioxidant, antiperiodontic, antiphlebitic, antipleuritic, antiraucedo, antirhinitic, antitonsilitic, antiulcer, antivaricose, antivertiginous, cancerostatic, corticosterogenic, diuretic, fungicide, hemolytic, hyaluronidase inhibitor, lymphagogue, natriuretic, pesticide, pituitary stimulant, thymolytic, vasoprotective, inhibiting leishmaniases, modulating adhesion or angiogenesis of cancer cells, antiparasitic; increase the expression of the genes: ANGPT2, DDIT3, LIF and NFKB1Z, and manufacturing an adjuvant composition and venotonic treatment.

Alkenyl means unsaturated linear or branched structures and combinations thereof, having formula R2C=CR2, one or more double bonds therein. Examples of alkenyl groups include vinyl, propenyl, isopropenyl, butenyl, s- and t-butenyl, pentenyl, hexenyl, butadienyl, pentadienyl, and hexadienyl.

An aryl is a functional group of organic molecule derived from an aromatic compound such as benzene, a 6-14 membered carbocyclic aromatic ring system comprising 1-3 benzene rings. If two or more aromatic rings are present, then the rings are fused together, so that adjacent rings share a common bond. Examples include phenyl and naphthyl. The aryl group may be substituted with one or more substitutes independently selected from halogen, alkyl or alkoxy.

Acyl is a functional group which can be obtained from an organic acid by the removal of the carboxyl. Acyl groups can be written using the general formula -COR, where there is a double bond between the carbon and oxygen. The names of acyl groups typically end in -yl, such as formyl, acetyl, propionyl, butyryl and benzoyl.
Benzoyl is one of the acyls, C₆H₅COR, obtained from benzoic acid by the removal of the carboxyl.

A heterocyclic compound is a compound containing a heterocyclic ring which refers to a non-aromatic ring having 1-4 heteroatoms, said ring being isolated or fused to a second ring selected from 3- to 7-membered alicyclic ring containing 0-4 heteroatoms, aryl and heteroaryl , wherein heterocyclic compounds include pyrrolidinyl , pipyrazinyl , morpholinyl, trahydrofuranyl, imidazolinyl, thiomorpholinyl, and the like.

Heterocyclyl groups are derived from heteroarenes by removal of a hydrogen atom from any ring atom.
Alkanoyl is the general name for an organic functional group RCO-, where R represents hydrogen or an alkyl group. Examples of alkanoyls are acetyl, propionoyl, butyryl, isobutyryl, pentanoyl and hexanoyl.
Alkenoyl is an alkenylcarbonyl in which the alkenyl is defined above. Examples are pentenoyl(tigloyl) and hexenoyl(angeloyl).
Alkyl is a radical containing only carbon and hydrogen atoms arranged in a chain, branched, cyclic or bicyclic structure or their combinations, having 1-18 carbon atoms. Examples include but are not limited to methyl, ethyl, propyl isopropyl, butyl, s- and t-butyl, pentyl, hexyl, heptyl, octyl, nonyl, undecyl, dodecyl, tridecyl, tetradecyl, pentadecyl, cyclopropyl, cyclobutyl, cyclopentyl and cyclohexyl.
Benzoyl alkyl substituted alkanoyl refers to straight or branched alkanoyl substituted with at least one benzoyl and at least one alkyl, wherein the benzoyl is attached to a straight or branched alkyl. An example of a benzoyl alkyl substituted alkanoyl is benzoyl methyl isobutanoyl.
A sugar moiety is a segment of molecule comprising one or more sugars or derivatives thereof or alduronic acid thereof.
Isobutyryl is a synonym of 2-Methylpropanoyl
(Y)Y3, Y and Y3 represent the same compound.
YM and (ACH-Y) represent the same compound.
Connecting moiety is a substructure or a group of atoms which connect the functional group to a core compound. Example: angeloyl group is connected by a sugar moiety to a triterpene core.

The building blocks used herein including triterpenes, hydroxylated triterpenes, acetyl, angeloyl, tigloyl, senecioyl, Crotonoyl, 0-3,3-Dimethylartyloyl, Cinnamoyl, Pentenoyl, Hexanoyl, benzoyl, Ethylbutyryl, alkyl, dibenzoyl, benzoyl, methylbutanoyl, methylpropanoyl, alkanoyl, alkenoyl, benzoyl alkyl substituted alkanoyl, alkanoyl substituted phenyl, alkenoyl substituted phenyl, aryl, acyl, heterocylic, heteroraryl, alkenylcarbonyl, acetyl chloride, angeloyl chloride, tigloyl chloride, senecioyl chloride, Crotonoyl chloride, 0-3,3-Dimethylartyloyl chloride, Cinnamoyl chloride, Pentenoyl chloride, Hexanoyl chloride, benzoyl chloride, Ethylbutyryl chloride.

Acetyl chloride 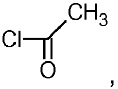 angeloyl chloride 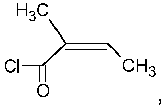 tigloyl chloride 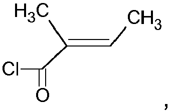 senecioyl chloride 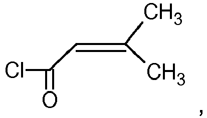 Crotonoyl chloride 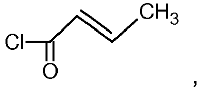 O-3,3-Dimethylartyloyl chloride 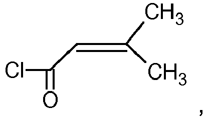 Cinnamoyl chloride 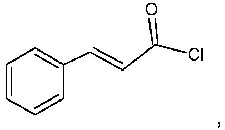 Pentenoyl chloride 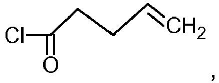 Hexanoyl chloride 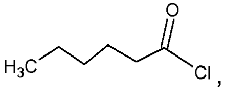 benzoyl chloride 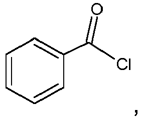 Ethylbutyryl chloride

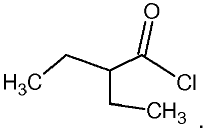

In the presented experiments, concentrations of drug that inhibit 15% cell-growth or less (i.e. 85% of control or above) as compared to the no-drug control (DMSO) are considered non-cytotoxic concentrations. In an embodiment, the concentrations of drug that inhibit 10% cell-growth or less (i.e. 90% of control or above) as compared to the no-drug control (DMSO) are considered non-cytotoxic concentrations. In an embodiment, the concentrations of drug that inhibit 5% cell-growth or less (i.e. 95% of control or above) as compared to the no-drug control (DMSO) are considered non-cytotoxic concentrations. In an embodiment, the concentrations of drug that inhibit 20% cell-growth or less (i.e. 80% of control or above) as compared to the no-drug control (DMSO) are considered non-cytotoxic concentrations. In an embodiment, the concentrations of drug that inhibit 25% cell-growth or less (i.e. 75% of control or above) as compared to the no-drug control (DMSO) are considered non-cytotoxic concentrations. In an embodiment, the concentrations of drug that inhibit 30% cell-growth or less as compared to the no-drug control (DMSO) are considered non-cytotoxic concentrations. In an embodiment, the concentrations of drug that inhibit 45% cell-growth or less as compared to the no-drug control (DMSO) are considered non-cytotoxic concentrations.

The triterpene compound or compounds selected from this invention as defined in the claims can be administered to a subject in need thereof, treating the subject, wherein including preventing cancer, or providing an adjuvant effect to the subject, or inhibiting the initation or promotion of cancer, or killing the cancer/tumor cells, or inhibiting cancer cell invasion. In an embodiment the compounds inhibit the activation of nuclear factor-kB, wherein inhibiting the localization or wherein binding the DNA. In an embodiment the compounds induce apoptosis in cancer cells.
Table 1 to 12, Effect of Y and YM on gene expression (Table of 1 to 12 PCT/US2008/002086, 1188-ALA-PCT, filed February 15, 2008) Table 13 to 19, Effect of Y and YM on gene expression (Table of 13 to 19 PCT/US2009/034115, 1188-D-PCT, filed February 15, 2008)
**Determination of gene expression by Real-time PCR method (Brilliant QPCR, Agilent Technologies):** The real-time polymerase chain reactions further confirm the results obtained from microarray analysis. The Real-time PCR results (shown below) confirmed that Compound Y3 and YM increase the expression of the genes: ANGPT2, DDIT3, LIF and NFKB1Z, wherein the results in Table 19-21 disclosed in PCT/US09/34115, filed February 13, 2009 are incorporated herein by reference.
The saponins are partially hydrolyzed into a mixture of products which can be separated by HPLC. Specific partial hydrolysis of saponins can also be achieved with enzymes. The glycosidases catalyze the hydrolysis of the glycosidic linkage. Galactosidase is an enzyme which catalyzes the hydrolysis of galactosides. Glucosidase is an enzyme which breaks glucose from saponin. Other enzyme examples are xylanases, lactase, amylase, chitinase, sucrase, maltase, and neuraminidase.
The sugar moiety of the triterpenoid saponin (example Xanifolia Y) can be removed by acid hydrolysis. The synthetic compound of ACH-Y is obtained. ACH-Y is a triterpene with acyl groups but no sugar moiety. The acyl group of the saponin (example Xanifolia Y) can be removed by alkaline hydrolysis. The synthetic compound AKOH-Y can be obtained. AKOH-Y is a pentacyclic triterpene with sugar moieties. A pentacyclic triterpene can be obtained by acid and alkaline hydroysis of saponins from natural sources. A pentacyclic triterpene can be obtained by synthetic methods (Reference: Surendra et al., Rapid and Enantioselective Synthetic Approches to Germanicol and Other Pentacyclic Triterpenes, Journal of the American Chemical Society, 2008, 130(27), 8865-8869). Pentacyclic triterpenes with sugar moieties can also be obtained by synthesis (Reference: Ple et al., Synthesis of L-arabinopyranose containing hederagenin saponins, Tetrahedron 61 (2005) 4347-4362). Acylation is the process of adding an acyl group to a compound. The Friedel-Crafts reaction is an example of this process. An active compound can be obtained by acylating a pentacyclic triterpenes, or hydroxylated triterpenes. In an embodiment, acylating C24, C28, C21 and C22 of a pentacyclic triterpenes, or hydroxylated triterpenes produce compounds for inhibiting cancer growth, cancer invasion, cell invasion, cancer cell invasion, cell attachment adhesion, or cell circulation. In an embodiment, the acyl group(s) may be at C3. In an embodiment, a sugar moiety is at C21, 22, or 28, wherein the sugar moiety is attached with 2 acyl groups. In an embodiment, acylating the compounds of (A), (B), (C), (D), (F), (G), (H), produce the compounds for inhibiting cancer invasion, cells invasion or cancer cell invasion; cancer metastasis; or cancer growth The building blocks in the present application are used to synthesise active saponins.
Acylating the compound (G) with angeloyl or tigloyl group gives the following compounds 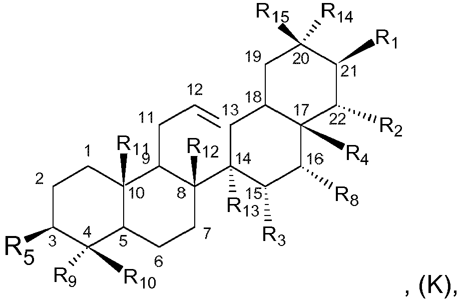 wherein R1, R2, R5, R8 represent OH or O-angeloyl; R3 represents OH, H or O-angeloyl; R4, R10 represent CH3, CH2OH or CH20angeloyl; R3 represents OH, H or O-angeloyl; R9, R11, R12, R13, R14, R15 represent CH3; or wherein R1, R2, R5, R8 represent OH or O-tigloyl; R3 represents OH, H or O- tigloyl; R4, R10 represent CH3, CH2OH or CH2O tigloyl; R9, R11, R12, R13, R14, R15 represent CH3; wherein the compounds inhibit cancer growth, cancer invasion, cells invasion or cancer cell invasion.

Acylating the compound (G) with angeloyl, tigloyl, senecioyl, acetyl, Crotonoyl, 3,3-Dimethylartyloyl, Cinnamoyl, Pentenoyl, Hexanoyl, benzoyl, Ethylbutyryl, alkyl, dibenzoyl, benzoyl, alkanoyl, alkenoyl, benzoyl alkyl substituted O-alkanoyl, alkanoyl substituted phenyl, alkenoyl substituted phenyl, aryl, acyl, heterocylic, heteroraryl, CH20-alkenylcarbonyl, alkane, alkene give the compound (K) wherein R1, R2, R5, R8 represent OH, O-angeloyl, O-tigloyl, O-senecioyl, O-acetyl, O-Crotonoyl, O-3,3-Dimethylartyloyl, O-Cinnamoyl, O-Pentenoyl, O-Hexanoyl, O-benzoyl, O-Ethylbutyryl,O-alkyl, O-dibenzoyl, O-benzoyl, O-alkanoyl, O-alkenoyl, O-benzoyl alkyl substituted O-alkanoyl, O-alkanoyl substituted phenyl, O-alkenoyl substituted phenyl, O-aryl, O-acyl, O-heterocylic, O-heteroraryl, O-alkenylcarbonyl; R4, R10 represent CH3, CH2OH, CH20-angeloyl, CH20-tigloyl, CH20-senecioyl, CH20-acetyl, CH20-Crotonoyl, CH2O-3,3-Dimethylartyloyl, CH2O-Cinnamoyl, CH20-Pentenoyl, CH2O-Hexanoyl, CH2O-benzoyl, CH20-Ethylbutyryl, CH20-alkyl, CH20-dibenzoyl, CH20-benzoyl, CH2O-alkanoyl, CH20-alkenoyl, CH20-benzoyl alkyl substituted O-alkanoyl, CH20-alkanoyl substituted phenyl, CH20-alkenoyl substituted phenyl, CH20-aryl, CH20-acyl, CH2O-heterocylic, CH20-heteroraryl, CH20-alkenylcarbonyl,alkane, alkene; R3 is absent of represents OH, H, O-angeloyl, O-tigloyl, O-senecioyl, O-acetyl, O-Crotonoyl, O-3,3-Dimethylartyloyl, O-Cinnamoyl, O-Pentenoyl, O-Hexanoyl, O-benzoyl, O-Ethylbutyryl,O-alkyl, O-dibenzoyl, O-benzoyl, O-alkanoyl, O-alkenoyl, O-benzoyl alkyl substituted O-alkanoyl, O-alkanoyl substituted phenyl, O-alkenoyl substituted phenyl, O-aryl, O-acyl, O-heterocylic, O-heteroraryl, O-alkenylcarbonyl; wherein R9, R11, R12, R13, R14, R15 represent CH3; wherein the compounds inhibit cancer growth, cancer invasion, cells invasion or cancer cell invasion; wherein the compound for use as mediator or inhibitor of adhesion protein or angiopoietin; wherein the compounds use as mediator modulating the secretion, expression, or synthesis of adhesion protein comprises reducing the fibronectin for inhibiting cell attachment, cell adhesion or cell circulation; wherein the adhesion proteins comprise fibronectin, integrins family, myosin, vitronectin, collagen, laminin, polyglycans, cadherin, heparin, tenascin, CD₅₄, and CAM; the compounds use for anti adhesion therapy and targeting adhesion molecules for therapy.

Applicant further states that anti adhesion therapy and targeting adhesion molecules for therapy is a new direction for development of drugs. Some examples of anti-adhesion drugs in clinical trials are Efalizumab, Odulimomab, Alicaforsen, Aselizumab etc, which target varies adhesion proteins. Please see TEXT BOOK, Adhesion Molecules: Function and Inhibition, (Reference 2), edited by Klaus Ley page 289-291, 297.

**Adhesion molecules in inflammatory disease,** (Reference 4), Abstract, line 7-8 "Blockade of the function of expression of CAM has emerged as a new therapeutic target in inflammatory diseases". Applicants' invention is an anti adhesion therapy which is a new use of the compound as a mediator or inhibitor of adhesion proteins and angiopoietins. It inhibits excess adhesion and inhibits cell attachment.
In the present application, Applicants have used compounds selected from structure (2A) for anti adhesion therapy, as a mediator or inhibitor of adhesion proteins and angiopoietins, and modulation of the cell attachment, and cell adhesion.

### EXPERIMENTAL DETAILS

Experiment details of herb extraction, analysis of extract components by HPLC, determination of the cell-growth activity effected by Xanifolia Y with cells derived from different human organs using MTT Assay, purification of the bioactive components from plant extract, fractionation of plant extracts with FPLC, isolation of component Ys with preparative HPLC, determination of the chemical structure, cell experiments and animal studying are disclosed in PCT/US05/31900, U.S. Serial No. 11/289142, U.S. Serial 10/906303, U.S. Serial No. 11/131551 and U.S. Serial Nos.11/683198, filed on March 7, 2007, PCT/US2007/077273, filed August 30, 2007, U.S. Serial No. 60/890380, filed on February 16, 2007, U.S. Nos. 60/947,705, filed on July 3, 2007, PCT/US2008/002086, 1188-ALA-PCT, filed February 15, 2008, App'l No. PCT/US09/34115, filed February 13, 2009.

### Experiment 1: Removal of the sugar moiety from saponin by acid hydrolysis

15mg saponin was dissolved in 1ml of Methanol. 1ml of 2N HCl was then added. The mixture was refluxed in 80C water bath for 5 hours. The solution was then neutralized by adding 2ml of 1N NaOH (to final pH 4-6). The aglycone was then extracted with ethylacetate 3ml x 2. The extracts were collected and pooled. Further isolation of aglycone (sugar-removed saponin) was achieved by HPLC with isocratic elution of 80-100% acetonitrile.

### Experiment 2: Removal of the acyl group by alkaline hydrolysis

**Methods:** 20mg of saponin was dissolved in 0.5ml of 1N NaOH. The solution was incubated in 80C water bath for 4 hours. It was cooled to room temperature before neutralized with 0.5ml 1N HCl (adjust pH to about 3). The mixture was extracted with 2 ml 1-butanol 3 times. The butanol fractions were collected and lyophilized. The hydrolyzed saponin with further purified with HPLC in a C-18 column eluted with 25% acetonitrile.

### Experiment 3: Adding the acyl group to triterpene by esterification

Method: 40 mg of triterpene core (fraction IV) was dissolved in 1 ml pyridine in a 50 ml tube. Reaction is started by adding 0.2 ml of acyl chloride (Tigloyl chloride, angeloyl chloride, Acetyl chloride, Crotonoyl chloride, 3,3-Dimethylartyloyl chloride(senecioyl chloride), Cinnamoyl chloride, Pentenoyl chloride, Hexanoyl chloride, benzoyl chloride or Ethylbutyryl chloride). The mixture is stirred for 5 sec, 1 min, 2 min, 5 min, 10 min, 30 min, 1 hr, 2 hr, 18 hr, 2 days or 3 days at 0C, 25C or 75C temperature. At the end of reaction, 5 ml of 2N HCl or 1M NaHCO3 is added to the reaction mixture. The solution is then extracted 3 times with 10 ml of ethyl acetate which is then evaporated under vacuum and at 45C and lyophilization. The reaction product is dissolved in 80% acetonitrile - 0.005% Trifluoroacetic acid or DMSO; and was separated with HPLC. Selecting the HPLC fractions for isolation is according to the cytotoxic activity of the reaction product obtained at a specific reaction time. The active esterification products are purified with HPLC. The reaction product of mixtures and individual compounds are tested with MTT cytotoxic assay. See examples Figures 1-12

### Experiment 4: Preparation of E4A

1. Beta-Escin dissolved in 1M NaOH (20 mg/ml) was incubated at 70C for 5 hours.
2. The hydrolyzed solution was neutralized with HCl and the water was evaporated by lyophilization.
3. The product was dissolved in 50% methanol and 1N HCl. The mixture was incubated at 70C for 5 hours.
4. The solution was neutralized with NaOH.
5. The hydrolyzed product was extracted with ethylacetate, which was subsequently removed by evaporation.
6. Further purification of the hydrolyzed product (E4A) was archived with FPLC chromatography in a C18 column equilibrated with 70% acetonitrile/TFA at the flow rate of 1 ml/min.

### Experiment 5: Esterification of E4A with Tigloyl Chloride

1. 50 mg of E4A in 1 ml pyridine, stir gently in a 50 ml tube. Esterification was carried out at 25C by adding 200 ul Tigloyl chloride.
2. Stir for 1 minute; then immediately add 5 ml of 2N HCl.
3. Stir for 1 hour and sit at room-Temp over night.
4. Extract the esterification products with 10 ml ethylacetate.
5. Evaporate the ethylacetate.
6. Dissolve the sample with 1 ml DMSO.
7. Fractionate the reaction products with HPLC.
8. Collect samples.

### Experiment 6: Isolation of E4A-Tig active compounds with HPLC

1. Column: ZORBAX ODS 9.4x250 mm, 5 um
2. Solvents: A: 45% AN/TFA; B: 100% AN/TFA
3. Chromatography conditions: a) Elution: Solvent A to B in 80 min; then with solvent B for 40 min; b) flow rate: 1 ml/mim. c) Monitor OD: at 207 nm;

### Experiment 7: MTT Experiment

**Cells.** HTB-9 (bladder), HeLa-S3 (cervix), DU145 (prostate), H460 (lung), MCF-7 (breast), K562 (leukemia), HCT116 (colon), HepG2 (liver), U2OS (bone), T98G (brain), SK-MEL-5 (Skin) and OVCAR 3, ES2 (ovary), Pancreas(Capan), Mouth(KB), Kidney(A498).

**MTT Assay.** The procedure for MTT assay followed the method described by Carmichael et al.(1987) with modifications. The cells were seeded into a 96-well plate at for 24 hours before drug-treatment. The cells were then exposed to the drugs for 48, 72, or 96 hours. After the drug-treatment, MTT (0.5 mg/mL) was added to cultures and incubated for an hour. The formazan (product of the reduction of tetrazolium by viable cells) formed and was dissolved with DMSO and the O.D. at 490nm, and was measured by an ELISA reader. The MTT level of the cells before drug-treatment was also measured (T0). The % cell-growth (%G) is calculated as: %G = (TD-T0 / TC-T0) x 100(1), where TC or TD represents O.D. readings of control or drug-treated cells. When T0 > TD, then the cytotoxicity (LC) expressed as % of the control is calculated as: %LC = (TD-T0 / T0) x 100(2).

### Experiment 8: Chemical synthesis, Isolation and characterization of E4A-Tig-R

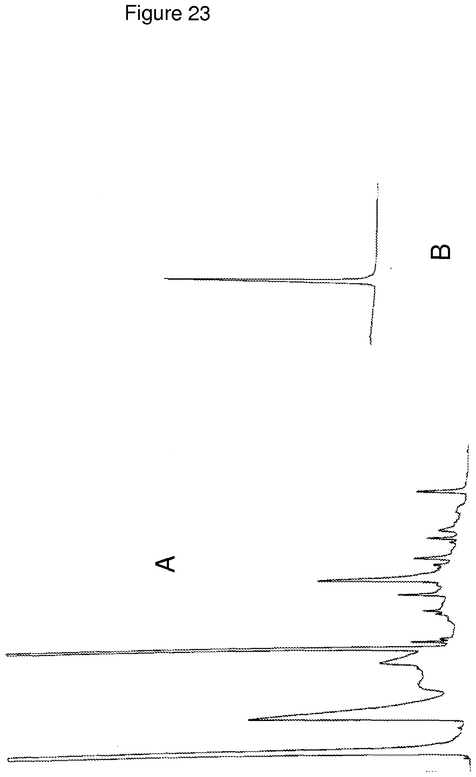

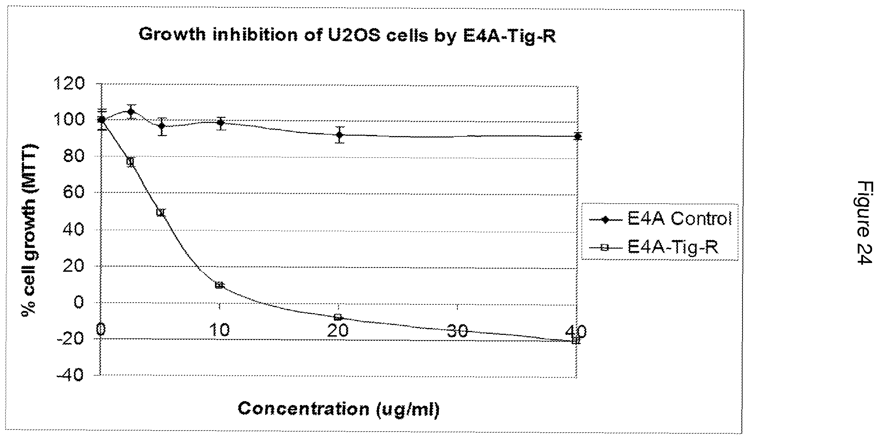

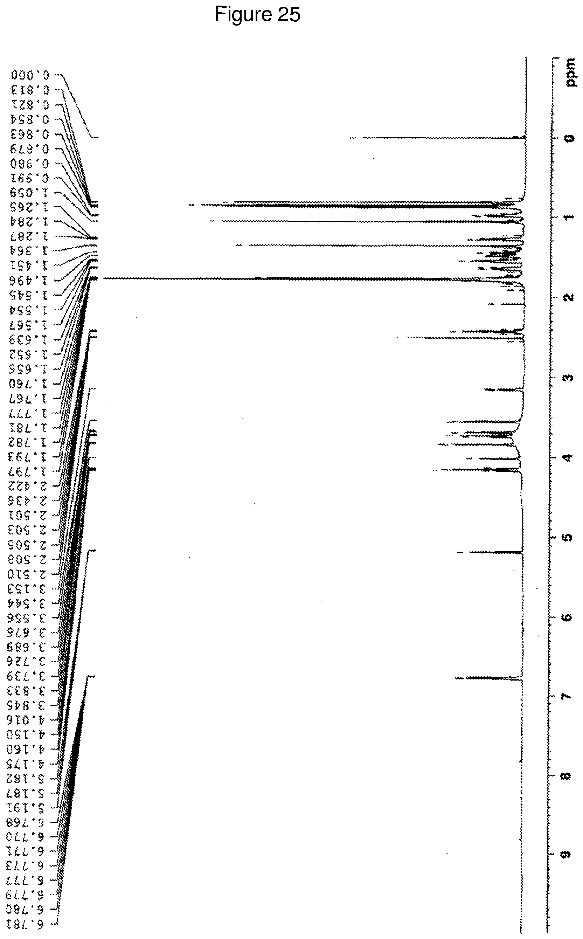

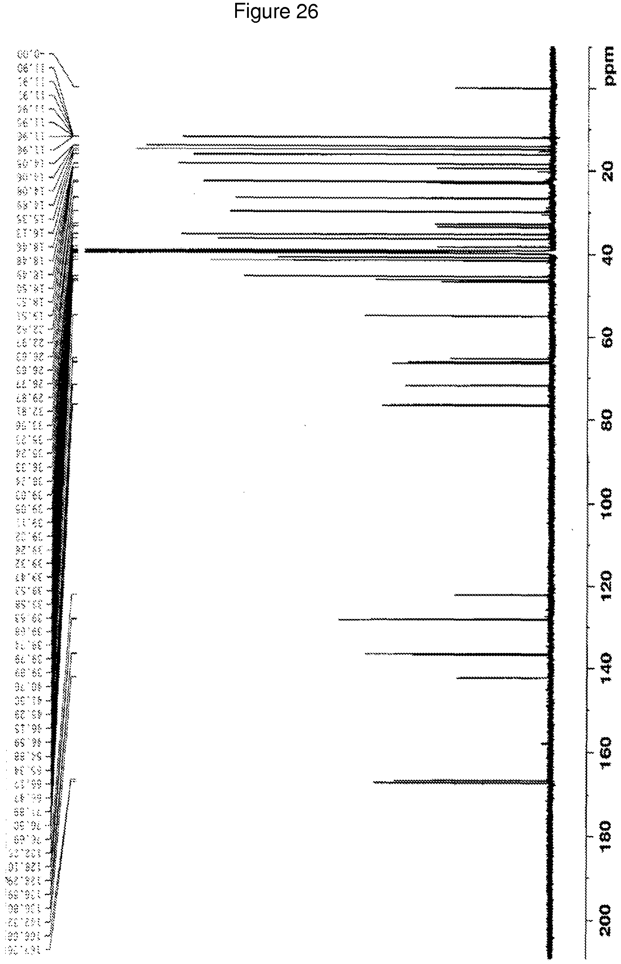

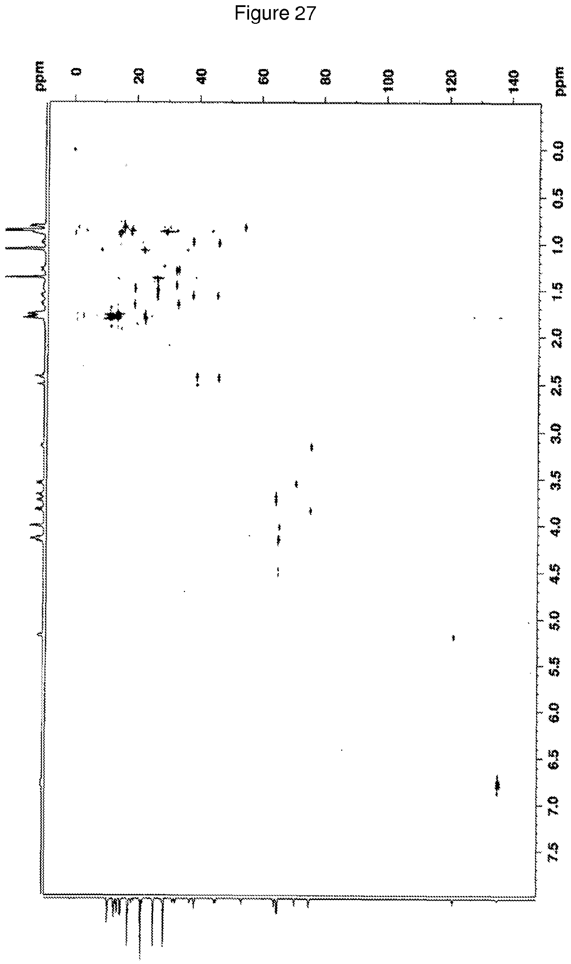

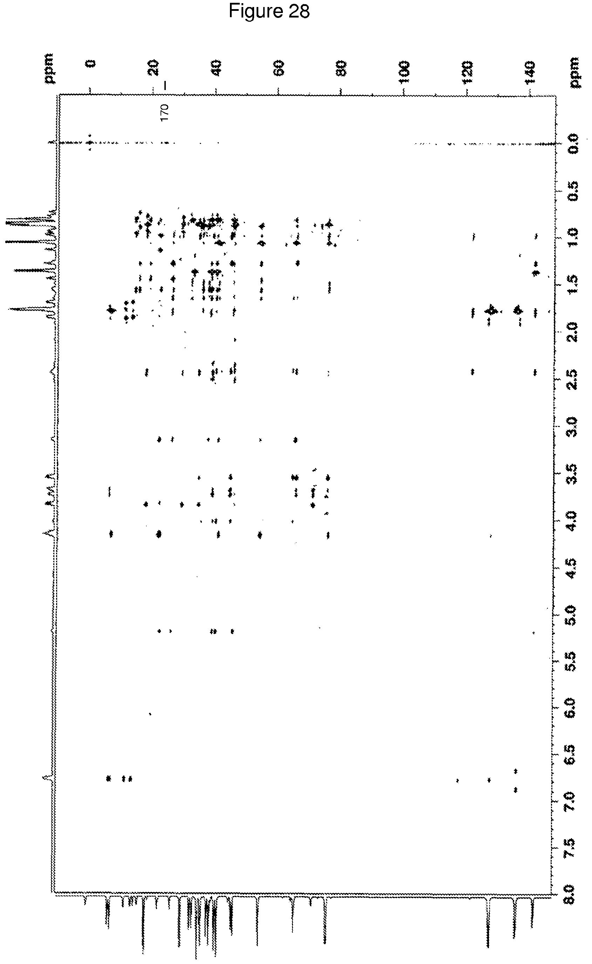

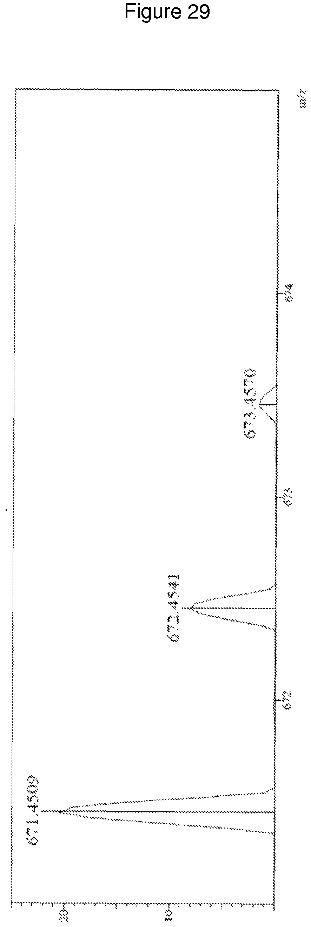

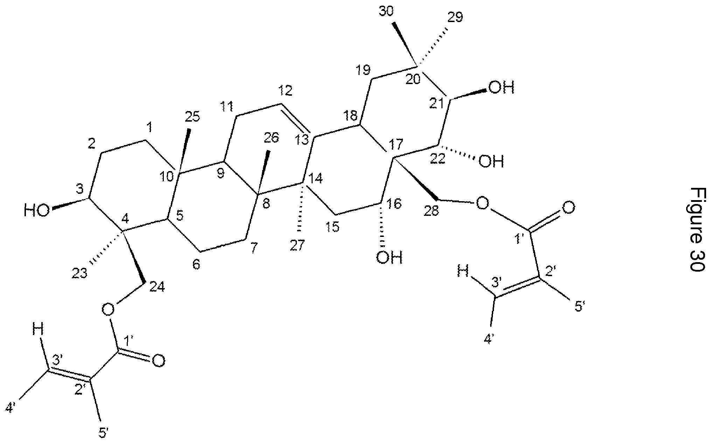

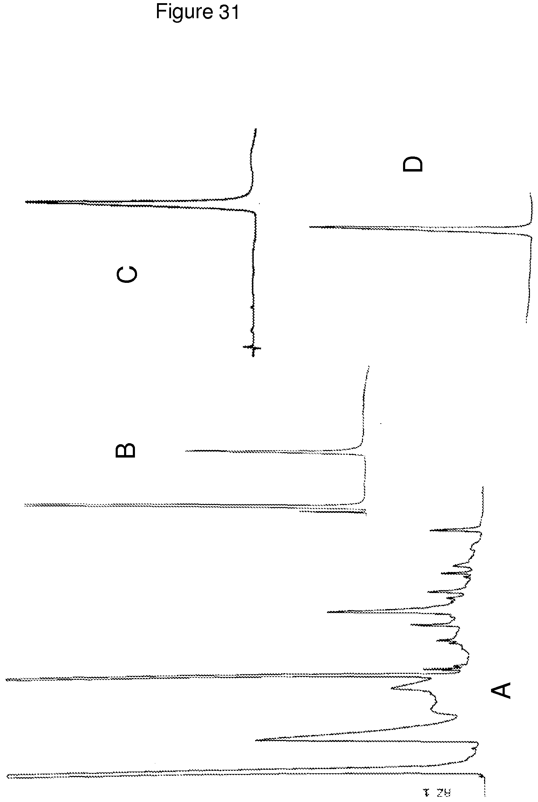

Chemical synthesis of E4A-Tig-R: 1. Preparation of E4A; 2. Esterification of E4A with Tigloyl Chloride; 3. Isolation of E4A-Tig-R with HPLC
Cytotoxic activity determination: 1. MTT assay
Chemical structure determination: 1. NMR analysis; 2. Mass Spectrum analysis
**See** **Figure 23-30**
**See Table 1**

### Compound E4A-Tig-R

### 24,28-O-Tigloyl-3β,16α, 21β, 22α, 24β, 28-hexahydroxyolean-12-ene

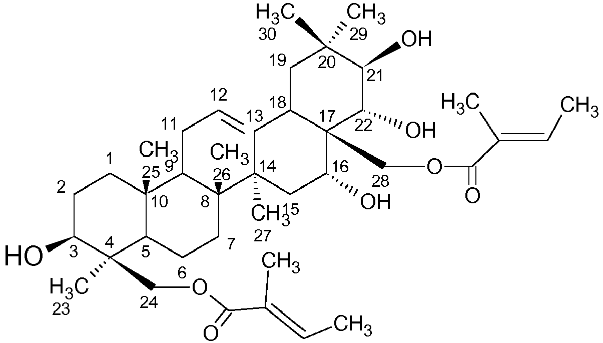

### Experiment 9: Chemical synthesis, Isolation and characterization of E4A-Tig-N

Chemical synthesis of E4A-Tig-R: 1. Preparation of E4A; 2. Esterification of E4A with Tigloyl Chloride; 3. Isolation of E4A-Tig-N with HPLC
Cytotoxic activity determination: 1. MTT assay
Chemical structure determination: 1. NMR analysis; 2. Mass Spectrum analysis

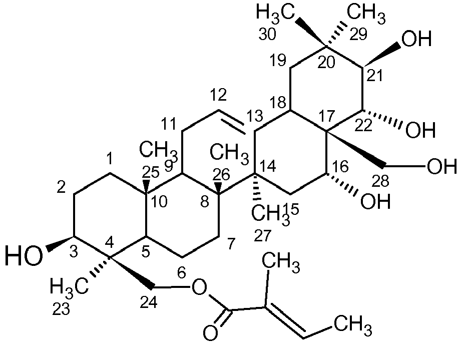

### Experiment 10: Chemical synthesis, Isolation and characterization of E4A-Tig-Q

Chemical synthesis of E4A-Tig-R: 1. Preparation of E4A; 2. Esterification of E4A with Tigloyl Chloride; 3. Isolation of E4A-Tig-Q with HPLC
Cytotoxic activity determination: 1. MTT assay
Chemical structure determination: 1. NMR analysis; 2. Mass Spectrum analysis

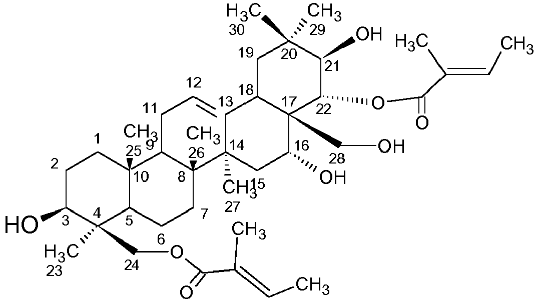

### Experiment 11: Chemical synthesis, Isolation and characterization of E4A-Tig-V

Chemical synthesis of E4A-Tig-V: 1. Preparation of E4A; 2. Esterification of E4A with Tigloyl Chloride; 3. Isolation of E4A-Tig-V with HPLC
Cytotoxic activity determination: 1. MTT assay
Chemical structure determination: 1. NMR analysis; 2. Mass Spectrum analysis

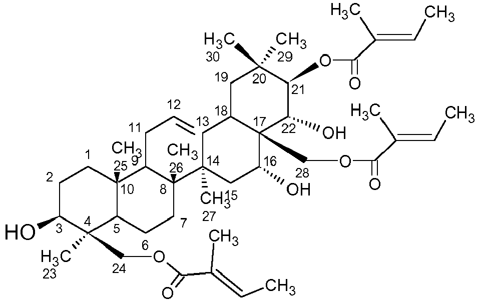

### Experiment 12: Chemical synthesis, Isolation and characterization of E4A-Tig-T

Chemical synthesis of E4A-Tig-T: 1. Preparation of E4A; 2. Esterification of E4A with Tigloyl Chloride; 3. Isolation of E4A-Tig-T with HPLC
Cytotoxic activity determination: 1. MTT assay
Chemical structure determination: 1. NMR analysis; 2. Mass Spectrum analysis

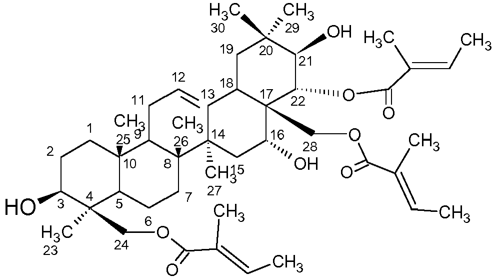

### Experiment 13: Chemical synthesis, Isolation and characterization of E4A-Tig-U

Chemical synthesis of E4A-Tig-S: 1. Preparation of E4A; 2. Esterification of E4A with Tigloyl Chloride; 3. Isolation of E4A-Tig-S with HPLC
Cytotoxic activity determination: 1. MTT assay
Chemical structure determination: 1. NMR analysis; 2. Mass Spectrum analysis

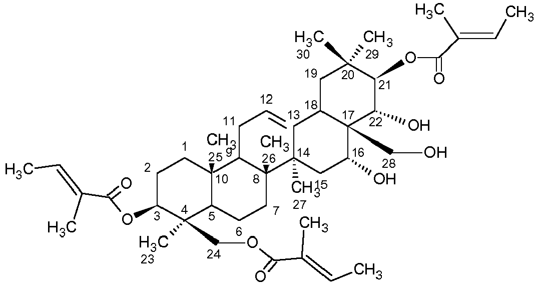

### Experiment 14: Chemical synthesis, Isolation and characterization of E4A-Tig-S

Chemical synthesis of E4A-Tig-S: 1. Preparation of E4A; 2. Esterification of E4A with Tigloyl Chloride; 3. Isolation of E4A-Tig-S with HPLC
Cytotoxic activity determination: 1. MTT assay
Chemical structure determination: 1. NMR analysis; 2. Mass Spectrum analysis

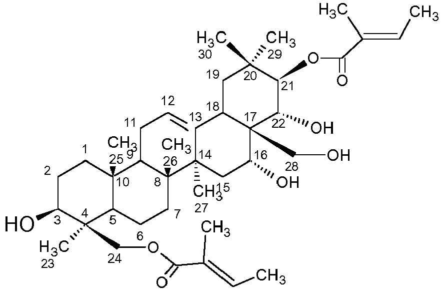

**Experiment 15: Using method in Experiment 8,** esterification of E4A with acetyl, angeloyl, tigloyl, senecioyl, Crotonoyl, Cinnamoyl, Pentenoyl, Hexanoyl, Ethylbutyryl, gave the following compounds

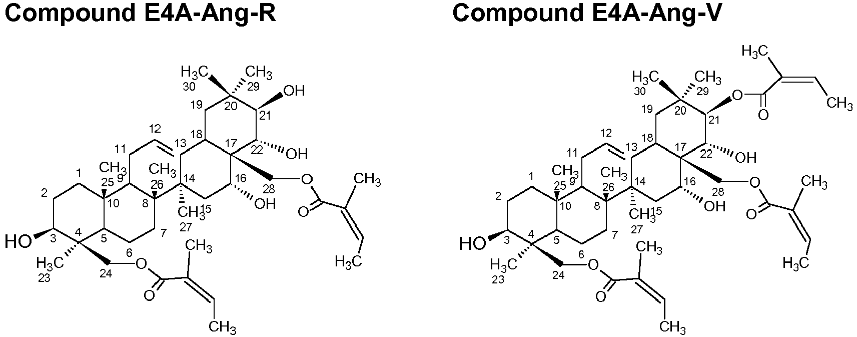

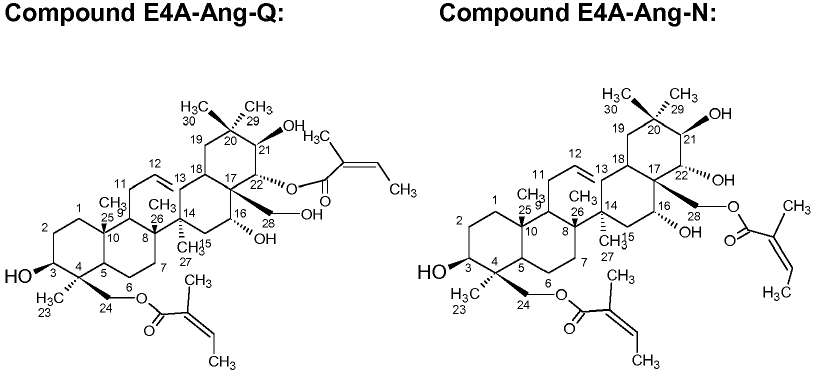

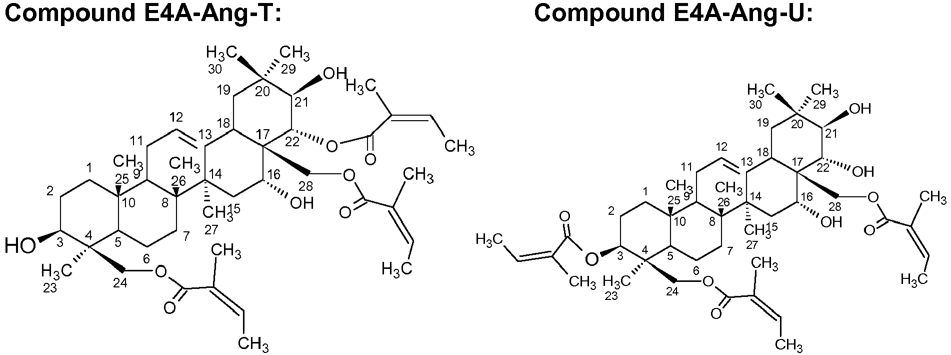

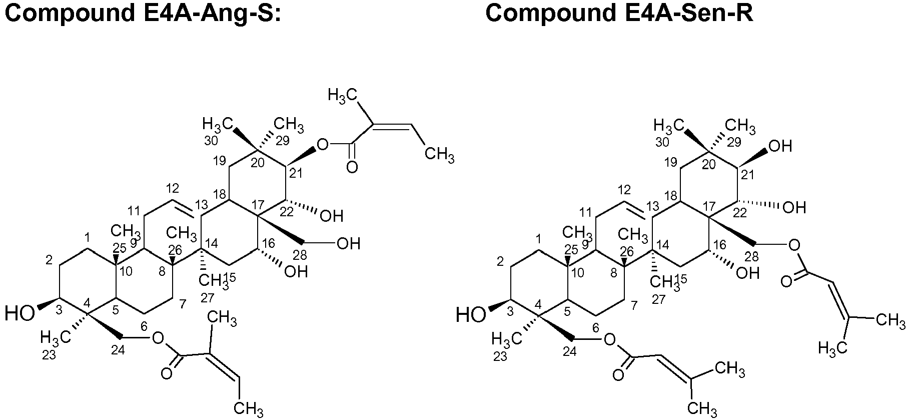

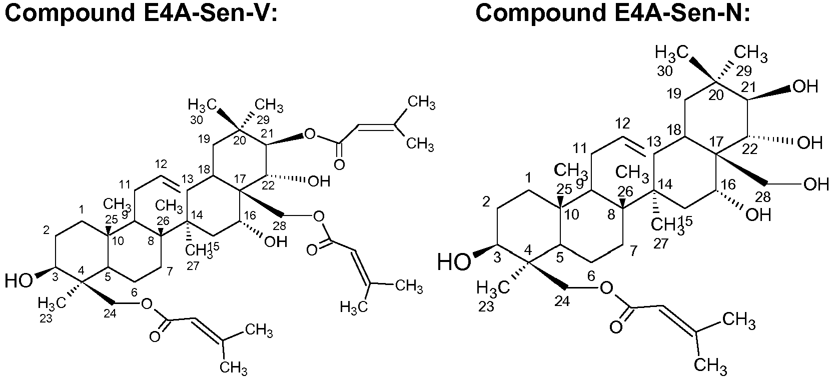

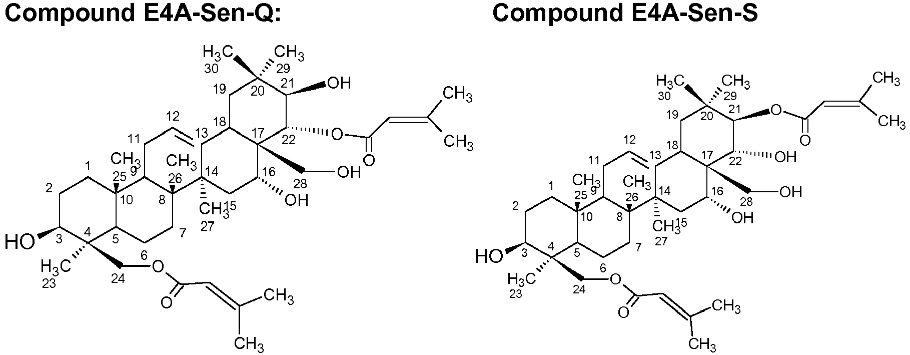

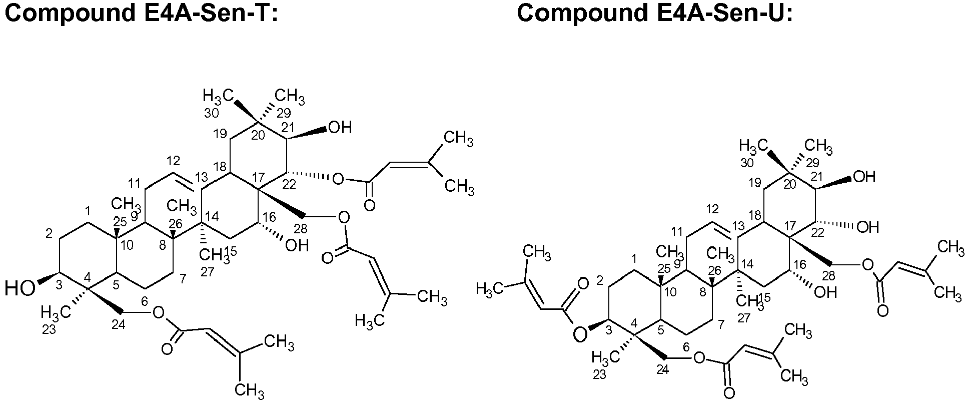

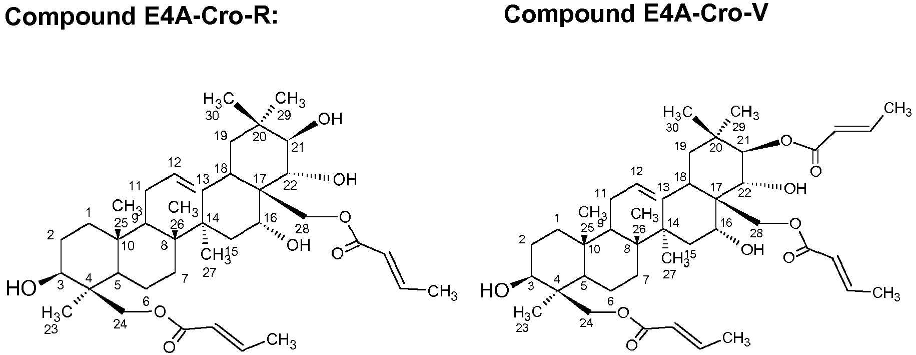

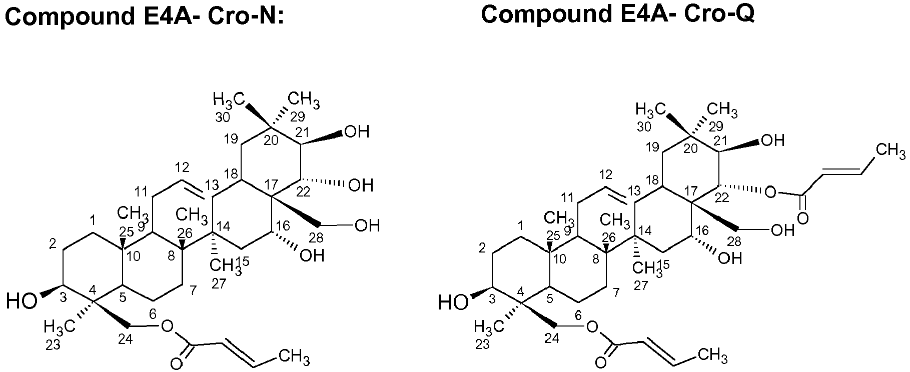

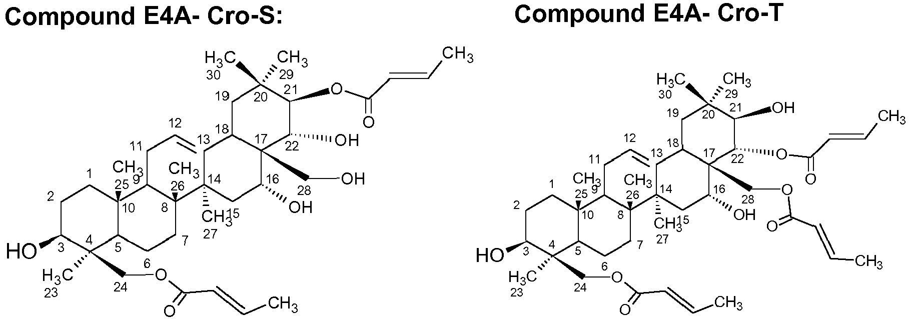

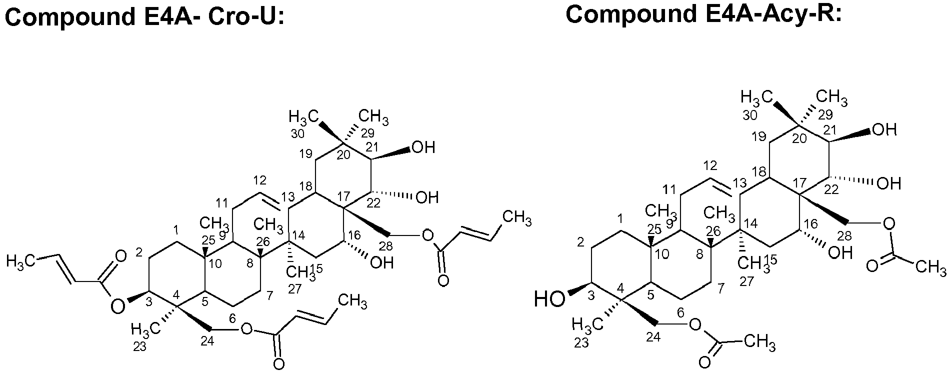

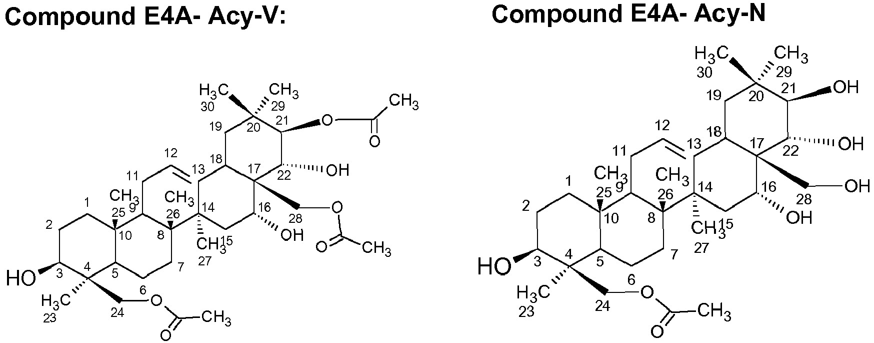

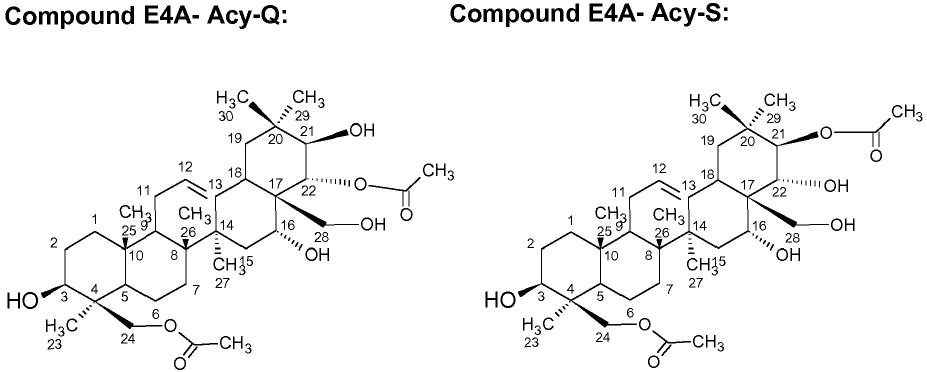

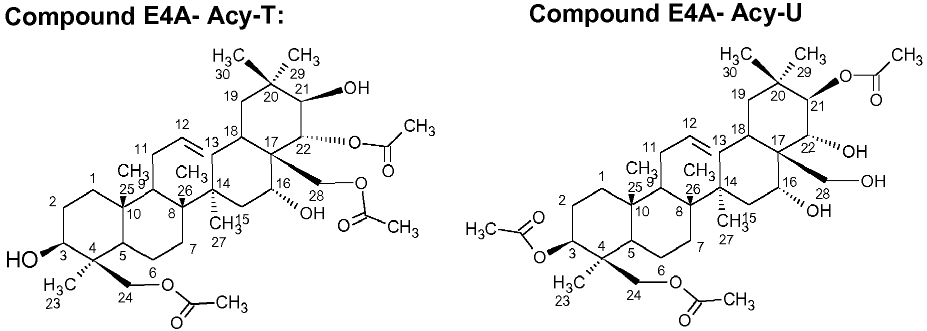

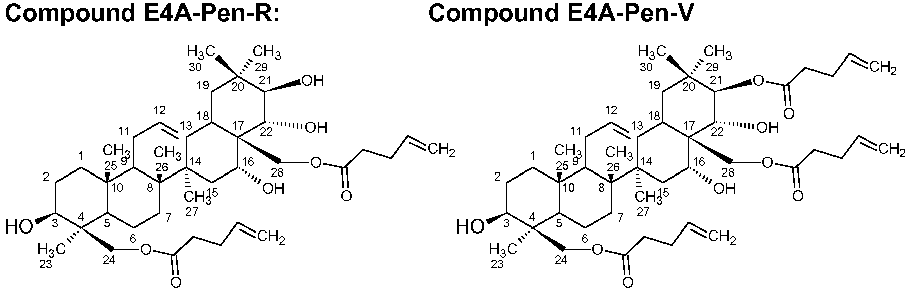

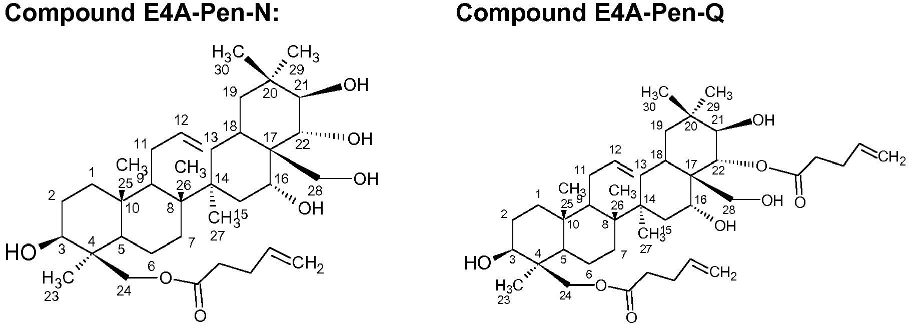

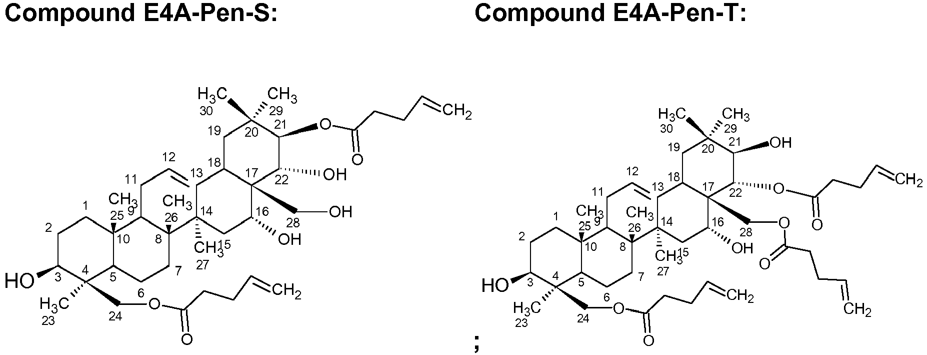

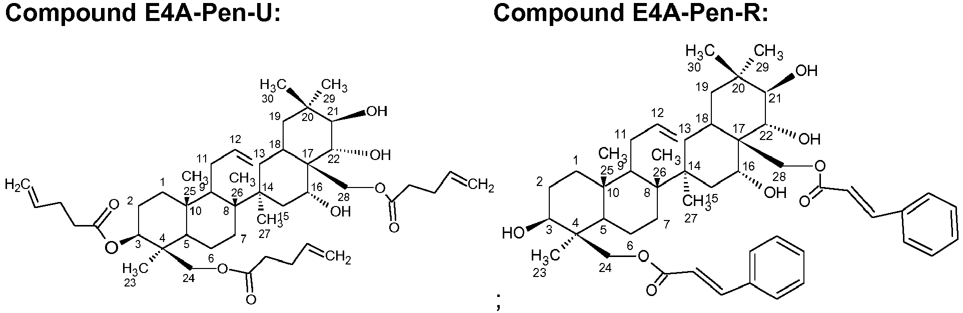

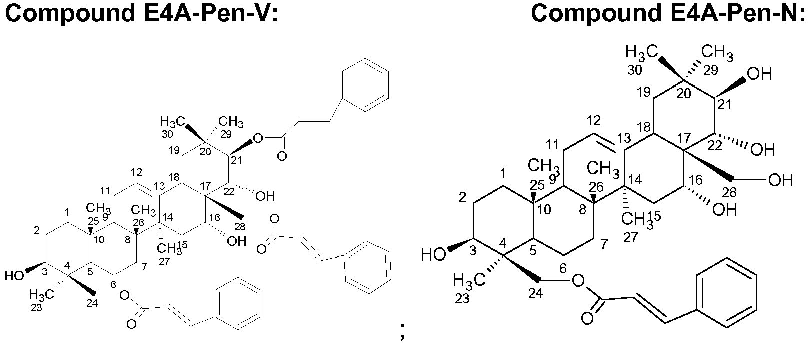

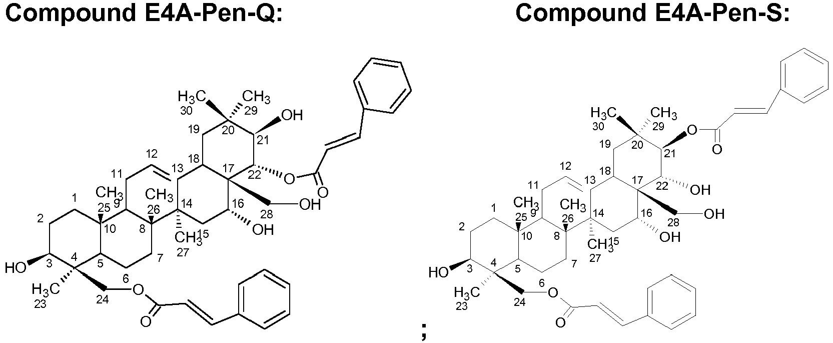

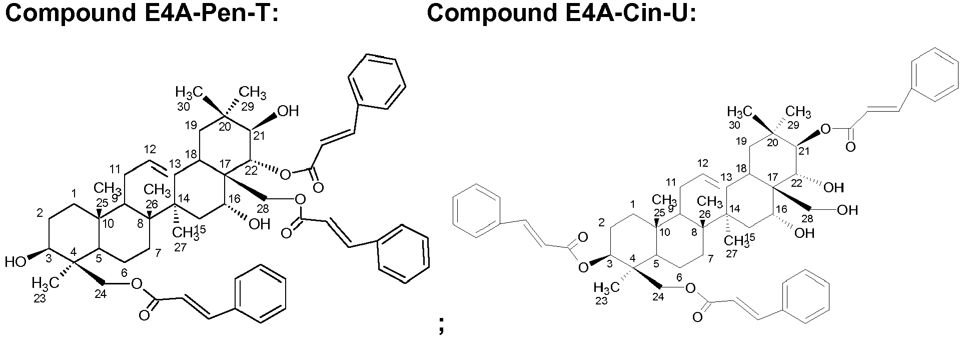

### Experiment 16: Esterification of E4A-Tig-N with senecioyl chloride

Chemical synthesis of E4A-Tig-Sen-1: 1. Esterification of E4A-Tig-N with Senecioyl Chloride; 3. Isolation of E4A-Tig-Sen-1 with HPLC
Cytotoxic activity determination: 1. MTT assay
Chemical structure determination: 1. NMR analysis; 2. Mass Spectrum analysis

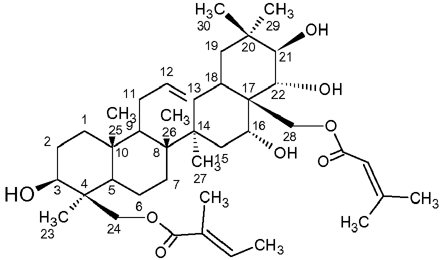

**Experiment 17: Esterification of E4A-Tig-N with** angeloyl chloride, Acetyl chloride, Crotonoyl chloride, 3,3-Dimethylartyloyl chloride, senecioyl chloride, Cinnamoyl chloride, Pentenoyl chloride, Hexanoyl chloride, benzoyl chloride or Ethylbutyryl chloride; Isolation with HPLC; Cytotoxic activity determination; Chemical structure determination with the method of Experiment 8, gave the following compounds:

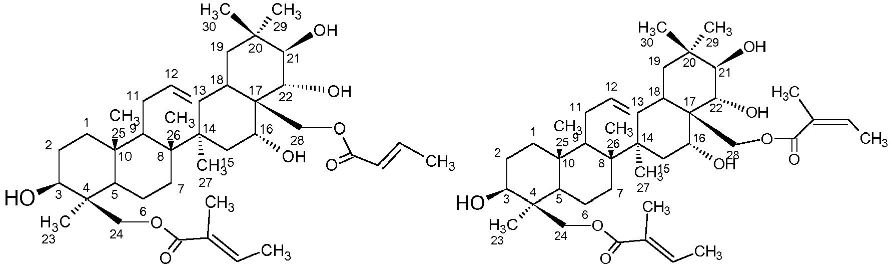

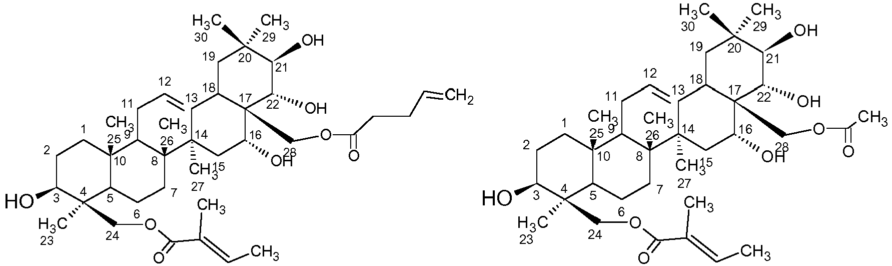

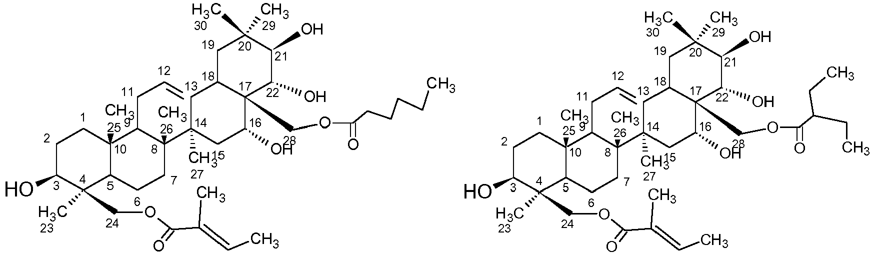

### Experiment 18: Inhibition of cell adhesion.

Methods and Results. ES2 or Hey8A cells were plated in T25 flasks with medium containing 5 ug/ml of compounds selected from structure (2A) including E4A-Tig-R, E4A-Tig-V, E4A-Tig-S, E4A-Tig-N, E4A-Tig-Q, E4A-Tig-T. Cultures were incubated for 5 hours. Attached cells were removed from flasks by trypsinization and the amounts were counted. Compare to no drug controls, 80 ± 4 % of ES2 cells and 60 ± 4 % of Hey8A cells were found attached to flasks under this condition. At 5 ug/ml of above compounds, over 90% of unattached cells are alive as determined by the trypan Blue exclusion assay and by their ability to re-attach to flasks when plating in medium without tested compounds. However, with 10 ug/ml tested compounds, less than 40% of cells attached to flasks and many of them are dead cells. This experiment shows that tested compounds inhibit cells adhesion process.

### Experiment 19: Fibronectin secretion experiment

Western blot is applied in this invention as a method to detect the specific proteins in treated and untreated cells with compounds in this invention, wherein the cells are bladder, cervix, prostate, lung, breast, leukemia, colon, liver, bone, brain, Skin, ovary, Pancreas(Capan), Mouth(KB), Kidney

Cells: targeted cells were grown in RPMI 1640 medium. 1.5 million cells were seeded in a T25 flask and grown for 24 hours before drug-treatment.

Drug-treatment: Cells cultures were replaced with fresh RPMI medium containing either 2.5 ul of DMSO (as control) [D]; or 10, 20, 30, 40, 80 ug/ml of tested compounds.
After 24 hours, aliquot of culture medium was taken out for Fibronectin determination (Western blot method).

Cell viability at 24 hours was determined by MTT assay. Cultures were replaced with RPMI medium (5 ml) with MTT and incubated for an hour. The formation of formazan was dissolved in 10 ml of DMSO and OD at 570nm was measured (MTT units).

Western Blot: Spent culture medium was mixed with SDS sample buffer, boiled for 3 minutes before loading to SDS gel. Samples were applied to a 6-10% SDS gel and electrophoresis was conducted with 100 volts for 2 hours. Protein was transferred to a nitrocellulose membrane electrophoretically. The nitrocellulose blot was incubated with the first antibody and second antibody (AP conjugated, Promega S3721). The immuno-bands were developed with BCIP/NBT color development system.

Determination of Western band intensity: The band-images of Western blot were captured with a digital camera and the intensity of bands was determined using "Image J" software.

Results show that compounds of E4A-Tig-R, E4A-Tig-V, E4A-Tig-S, E4A-Tig-N, E4A-Tig-Q, E4A-Tig-T inhibit fibronectin secretion from 20-40%.in bladder, cervix, prostate, lung, breast, leukemia, colon, liver, bone, brain, Skin, ovary, Pancreas(Capan), Mouth(KB), Kidney.

**Table 1:**

| Table. ¹³C and ¹H NMR data for E4A-Tig-R (in DMSO-*d*₆)*^{a}* | | | |
|---|---|---|---|
| Position | C | H | Key HMBC correlations |
| 1 | 38.24 | 0.96, t, 1.56, t | C-25 |
| 2 | 26.77 | 1.52, br, m | - |
| 3 | 76.69 | 3.15, 1H, dd | C23, C24 |
| 4 | 41.5 | - | - |
| 5 | 54.88 | 0.82, 1H | C23, C24, C25 |
| 6 | 19.51 | 1.47, 1.65, | C5 |
| 7 | 32.81 | 1.28, 1.43 | C26 |
| 8 | 39 | - | C27, C26 |
| 9 | 46.1 | 1.55 m | C25, C26 |
| 10 | 36.33 | - | C9,C25, C26 |
| 11 | 22.97 | 1.79 m | C9 |
| 12 | 122.25 | 5.18, 1H, t | C9, C11, C14, C18 |
| 13 | 142.32 | - | C18, C27 |
| 14 | 40.7 | - | C26, C27 |
| 15 | 33.56 | 1.28, 1.64 | C27 |
| 16 | 66.47 | 4.01, 1H, s | C22, C28 |
| 17 | 45.3 | - | C22, C28 |
| 18 | 39.9 | 2.41, br, m, | C12, C28 |
| 19 | 46.59 | 0.98, 2.42 m | C29, C30 |
| 20 | 35.23 | - | C29, C30 |
| 21 | 76.50 | 3.84, 1H, d, 9.6 Hz | C22, C29, C30 |
| 22 | 71.89 | 3.55, 1H, d, 9.6 Hz | C21, C28, |
| 23 | 22.62 | 1.06, 3H, s | C3, C5, C24, |
| 24 | 66.17 | 4.14, 1H, d, 12 Hz | C3, C5,C-23 |
| | | 4.17, 1H, d, 12 Hz | 24-*O*-Tig-C1' |
| 25 | 14.89 | 0.88, 3H, s | C-1, C-5, C-9, C-10 |
| 26 | 16.13 | 0.81, 3H, s | C-7, C-8, C-9, C-14 |
| 27 | 26.65 | 1.36, 3H, s | C-8, C-18, C14, C-15 |
| 28 | 65.34 | 3.68, 1H, d, 10.4 Hz, | C17, C-18, C-22 |
| | | 3.73, 1H, d, 10.4 Hz, | 28-*O*-Tig C1' |
| 29 | 29.87 | 0.86, 3H, s | C-19, C20, C-21, C-30 |
| 30 | 18.49 | 0.85, 3H, s | C-19, C20, C-21, C-29 |

| 24-*O*-Tig | | | |
|---|---|---|---|
| 1' | 167.24 | - | C24, Tig C-3', |
| 2' | 128.29 | - | Tig-C3', Tig C-4', Tig C-5' |
| 3' | 136.8 | 6.77, 1H, | Tig C-4', Tig C-5' |
| 4' | 11.9 | 1.78, 3H, | Tig C-1', C-2', C-3' |
| 5' | 13.99 | 1.77, 3H, | Tig C-1', C-2', C-3' |

| 28-*O*-Tig | | | |
|---|---|---|---|
| 1' | 166.68 | - | C28, Tig C-3' |
| 2' | 128.1 | - | Tig C-3', Tig C-4', Tig C-5' |
| 3' | 136.5 | 6.77, 1H, | Tig C-4', Tig C-5' |
| 4' | 11.9 | 1.78, 3H, | Tig C-1', C-2', C-3' |
| 5' | 14.08 | 1.77, 3H, | Tig C-1', C-2', C-3' |

**Table 2:**

| Table. ¹³C and ¹H NMR data for E4A-Tig-V (in DMSO-*d*₆)*^{a}* | | | |
|---|---|---|---|
| Position | C | H | Key HMBC correlations |
| 1 | 38.20 | 0.98, 1.57 | C-25 |
| 2 | 26.75 | 1.54, br, m | - |
| 3 | 76.65 | 3.15, 1H, dd | C23, C24 |
| 4 | 41.48 | - | - |
| 5 | 54.82 | 0.82, 1H | C23, C24, C25 |
| 6 | 19.49 | 1.47, 1.65, | C5 |
| 7 | 32.71 | 1.29, 1.46 | C26 |
| 8 | 39 | - | C27, C26 |
| 9 | 46.09 | 1.57 m | C25, C26 |
| 10 | 36.31 | - | C5,C9,C25, |
| 11 | 22.97 | 1.81 m | |
| 12 | 122.65 | 5.22, 1H, t | C9, C11, C14, C18 |
| 13 | 141.83 | - | C18, C19, C27 |
| 14 | 40.68 | - | C12, C18, C26, C27 |
| 15 | 33.59 | 1.29, 1.66 | C27 |
| 16 | 66.14 | 4.03, 1H, s | C18, C22, C28 |
| 17 | 45.69 | - | C18, C22, C28 |
| 18 | 39.5 | 2.5, br, m, | C12, C19, C28 |
| 19 | 46.17 | 1.07, 2.56 m | C18, C29, C30 |
| 20 | 35.33 | - | C29, C30 |
| 21 | 79.74 | 5.57 1H, d, 9.6 Hz | C20, C22, C29, C30 21-*O*-*Tig*-C1, |
| 22 | 69.39 | 3.79, 1H, d, 9.6 Hz | C21, C28, |
| 23 | 22.60 | 1.06, 3H, s | C3, C4, C5, C24, |
| 24 | 66.14 | 4.15 (dd 16.8, 12 Hz) | C3, C4, C5,C-23 24-*O*- *Tig*-C1' |
| 25 | 14.87 | 0.88, 3H, s | C-1, C-5, C-9, C-10 |
| 26 | 16.09 | 0.81, 3H, s | C-7, C-8, C-9, C-14 |
| 27 | 26.7 | 1.38, 3H, s | C-8, C13, C14, C-15 |
| 28 | 65.09 | 3.72 (dd 28.4, 10.4) | C16, C17, C-18, C-22 28-*O*-Tig C1' |
| 29 | 29.24 | 0.74, 3H, s | C-19, C20, C-21, C-30 |
| 30 | 19.35 | 0.98, 3H, s | C-19, C20, C-21, C-29 |

| 21-*O*-Tig | | | |
|---|---|---|---|
| 1' | 167.05 | - | C21, Tig C-3', |
| 2' | 128.04 | - | Tig-C3', Tig C-4', Tig C-5' |
| 3' | 135.61 | 6.77, 1H, | Tig C-4', Tig C-5' |
| 4' | 11.94 | 1.79, br, m, 3H, | Tig C-1', C-2', C-3' |
| 5' | 13.84 | 1.78, br, m, 3H, | Tig C-1', C-2', C-3' |

| 24-*O*-Tig | | | |
|---|---|---|---|
| 1' | 167.26 | - | C24, Tig C-3' |
| 2' | 128.26 | - | Tig C-3', Tig C-4', Tig C-5' |
| 3' | 136.60 | 6.77, 1H, | Tig C-4', Tig C-5' |
| 4' | 11.94 | 1.79, br, m, 3H, | Tig C-1', C-2', C-3' |
| 5' | 13.96 | 1.78, br, m, 3H, | Tig C-1', C-2', C-3' |

| 28-*O*-Tig | | | |
|---|---|---|---|
| 1' | 166.64 | - | C28, Tig C-3' |
| 2' | 128.71 | - | Tig C-3', Tig C-4', Tig C-5' |
| 3' | 136.96 | 6.77, 1H, | Tig C-4', Tig C-5' |
| 4' | 12.09 | 1.79, br, m, 3H, | Tig C-1', C-2', C-3' |
| 5' | 14.06 | 1.78, br, m, 3H, | Tig C-1', C-2', C-3' |

## Claims

1. A compound of formula 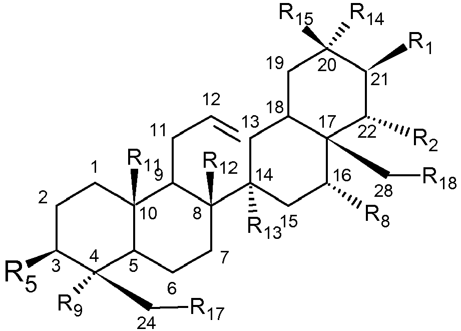 or a pharmaceutically acceptable salt thereof,
wherein
R9, R11, R12, R13, R14, and R15 represent CH3; and
**(i)** three of R1, R2, R5 and R8 are OH; and
R17, R18 and one of R1, R2, R5 and R8 are the same and are selected from the group consisting of O-angeloyl, O-senecioyl, O-4-pentenoyl, O-hexanoyl, O-2-ethylbutyryl, O-crotonoyl, O-cinnamoyl, and O-benzoyl; or
**(ii)** R18 and three of R1, R2, R5 and R8 are OH; and
R17 and one of R1, R2, R5 and R8 are the same and are selected from the group consisting of O-angeloyl, O-senecioyl, O-4-pentenoyl, O-hexanoyl, O-2-ethylbutyryl, O-acetyl, O-crotonoyl, O-cinnamoyl, and O-benzoyl; or
**(iii)** R8 and two of R1, R2, and R5 are OH;
R17 is O-tigloyl; and
R18 and one of R1, R2, and R5 are the same and are selected from the group consisting of O-senecioyl, O-crotonoyl, O-acetyl, O-4-pentenoyl, O-hexanoyl, O-cinnamoyl, O-angeloyl, and O-2-ethylbutyryl; or
**(iv)** R5, R8, R18, and one of R1 and R2 are OH;
R17 is O-tigloyl; and
one of R1 and R2 is selected from the group consisting of O-senecioyl, O-crotonoyl, O-acetyl, O-4-pentenoyl, O-hexanoyl, O-cinnamoyl, O-angeloyl, and O-2-ethylbutyryl.

2. The compound of claim 1, or a pharmaceutically acceptable salt thereof,
wherein
**(i)** three of R1, R2, R5 and R8 are OH; and
R17, R18, and one of R1, R2, R5 and R8 are the same and are selected from the group consisting of O-angeloyl, O-senecioyl, O-4-pentenoyl, O-hexanoyl, O-2-ethylbutyryl, O-crotonoyl, O-cinnamoyl, and O-benzoyl; or
**(ii)** R18 and three of R1, R2, R5 and R8 are OH; and
R17 and one of R1, R2, R5 and R8 are the same and are selected from the group consisting of O-angeloyl, O-senecioyl, O-4-pentenoyl, O-hexanoyl, O-2-ethylbutyryl, O-acetyl, O-crotonoyl, O-cinnamoyl, and O-benzoyl.

3. The compound of claim 2, or a pharmaceutically acceptable salt thereof,
wherein
**(i)** R2, R5 and R8 are OH; and
R1, R17 and R18 are the same and are selected from the group consisting of O-angeloyl, O-senecioyl, O-4-pentenoyl, O-hexanoyl, O-2-ethylbutyryl, O-crotonoyl, O-cinnamoyl, and O-benzoyl; or
**(ii)** R2, R5, R8 and R18 are OH; and
R1 and R17 are the same and are selected from the group consisting of O-angeloyl, O-senecioyl, O-4-pentenoyl, O-hexanoyl, O-2-ethylbutyryl, O-acetyl, O-crotonoyl, O-cinnamoyl, and O-benzoyl.

4. The compound of claim 3, or a pharmaceutically acceptable salt thereof,
wherein
**(i)** R2, R5 and R8 are OH; and R1, R17 and R18 are O-angeloyl; or
**(ii)** R2, R5, R8 and R18 are OH; and R1 and R17 are O-angeloyl.

5. The compound of claim 3, or a pharmaceutically acceptable salt thereof,
wherein
**(i)** R2, R5 and R8 are OH; and R1, R17 and R18 are O-senecioyl; or
**(ii)** R2, R5, R8 and R18 are OH; and R1 and R17 are O-senecioyl.

6. The compound of claim 3, or a pharmaceutically acceptable salt thereof,
wherein
**(i)** R2, R5 and R8 are OH; and R1, R17 and R18 are O-crotonoyl; or
**(ii)** R2, R5, R8 and R18 are OH; and R1 and R17 are O-crotonoyl.

7. The compound of claim 3, or a pharmaceutically acceptable salt thereof,
wherein
**(i)** R2, R5 and R8 are OH; and R1, R17 and R18 are O-cinnamoyl; or
**(ii)** R2, R5, R8 and R18 are OH; and R1 and R17 are O-cinnamoyl.

8. The compound of claim 3, or a pharmaceutically acceptable salt thereof,
wherein
**(i)** R2, R5 and R8 are OH; and R1, R17 and R18 are O-4-pentenoyl; or
**(ii)** R2, R5, R8 and R18 are OH; and R1 and R17 are O-4-pentenoyl.

9. The compound of claim 3, or a pharmaceutically acceptable salt thereof,
wherein
**(i)** R2, R5 and R8 are OH; and R1, R17 and R18 are O-hexanoyl; or
**(ii)** R2, R5, R8 and R18 are OH; and R1 and R17 are O-hexanoyl.

10. The compound of claim 3, or a pharmaceutically acceptable salt thereof,
wherein
**(i)** R2, R5 and R8 are OH; and R1, R17 and R18 are O-2-ethylbutyryl; or
**(ii)** R2, R5, R8 and R18 are OH; and R1 and R17 are O-2-ethylbutyryl.

11. The compound of claim 3, or a pharmaceutically acceptable salt thereof,
wherein
**(i)** R2, R5 and R8 are OH; and R1, R17 and R18 are O-benzoyl; or
**(ii)** R2, R5, R8 and R18 are OH; and R1 and R17 are O-benzoyl.

12. The compound of any one of claims 1 to 11, or a pharmaceutically acceptable salt thereof, for use as a medicament.

13. The compound of any one of claims 1 to 11, or a pharmaceutically acceptable salt thereof, for use in treating cancer; inhibiting cancer growth, cancer invasion, wherein the cancer is selected from the group consisting of breast cancer, leukocytic cancer, liver cancer, ovarian cancer, bladder cancer, prostatic cancer, skin cancer, bone cancer, brain cancer, leukemia cancer, lung cancer, colon cancer, CNS cancer, melanoma cancer, renal cancer, cervical cancer, esophageal cancer, testicular cancer, spleenic cancer, kidney cancer, lymphhatic cancer, pancreatic cancer, stomach cancer and thyroid cancer.

14. A compound of any one of claims 1 to 11, or a pharmaceutically acceptable salt thereof, for use in treating cell adhesion, cell attachment, cell circulating; inhibiting viruses; preventing cerebral aging; improving memory; improving cerebral functions; curing enuresis, frequent micturition, urinary incontinence; dementia, Alzheimer's disease, autism, brain trauma, Parkinson's disease or other diseases caused by cerebral dysfunctions; treating arthritis, rheumatism, poor circulation, arteriosclerosis, Raynaud's syndrome, angina pectoris, cardiac disorder, coronary heart disease, headache, dizziness, kidney disorder; cerebrovascular disease; inhibiting NF-Kappa B activation; treating brain edema, severe acute respiratory syndrome, respiratory viral diseases, chronic venous insufficiency, hypertension, chronic venous disease, oedema, inflammation, hemorrhoids, peripheral edema formation, varicose vein disease, flu, post traumatic edema and postoperative swelling; inhibiting blood clots, inhibiting ethanol absorption; lowering blood sugar; or regulating adrenocorticotropin and corticosterone levels.

15. A composition comprising an effective amount of a compound of any one of the claims 1 to 11, or a pharmaceutically acceptable salt thereof, as a medicament.

## Patentansprüche

1. Eine Verbindung der Formel 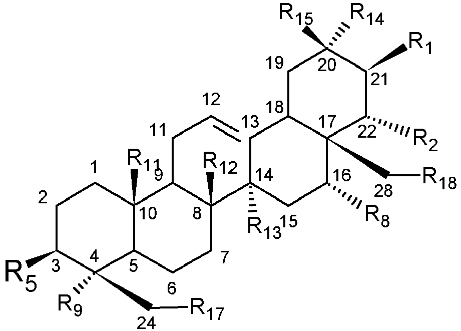 oder ein pharmazeutisch verträgliches Salz davon,
wobei
R9, R11, R12, R13, R14 und R15 für CH3 stehen; und
(i) drei von R1, R2, R5 und R8 für OH stehen; und
R17, R18 und einer von R1, R2, R5 und R8 gleich sind und ausgewählt sind aus der Gruppe bestehend aus O-Angeloyl, O-Senecioyl, O-4-Pentenoyl, O-Hexanoyl, O-2-Ethylbutyryl, O-Crotonoyl, O-Cinnamoyl und O-Benzoyl; oder
(ii) R18 und drei von R1, R2, R5 und R8 für OH stehen; und
R17 und einer von R1, R2, R5 und R8 gleich sind und ausgewählt sind aus der Gruppe bestehend aus O-Angeloyl, O-Senecioyl, O-4-Pentenoyl, O-Hexanoyl, O-2-Ethylbutyryl, O-Acetyl, O-Crotonoyl, O-Cinnamoyl und O-Benzoyl; oder
(iii) R8 und zwei von R1, R2 und R5 für OH stehen;
R17 für O-Tigloyl steht; und
R18 und eines von R1, R2 und R5 gleich sind und ausgewählt sind aus der Gruppe bestehend aus O-Senecioyl, O-Crotonoyl, O-Acetyl, O-4-Pentenoyl, O-Hexanoyl, O-Cinnamoyl, O-Angeloyl und O-2-Ethylbutyryl; oder
(iv) R5, R8, R18 und einer von R1 und R2 für OH stehen;
R17 für O-Tigloyl steht; und
einer von R1 und R2 ausgewählt ist aus der Gruppe bestehend aus O-Senecioyl, O-Crotonoyl, O-Acetyl, O-4-Pentenoyl, O-Hexanoyl, O-Cinnamoyl, O-Angeloyl und O-2-Ethylbutyryl.

2. Die Verbindung nach Anspruch 1 oder ein pharmazeutisch verträgliches Salz davon, wobei
(i) drei von R1, R2, R5 und R8 für OH stehen; und
R17, R18 und eines von R1, R2, R5 und R8 gleich sind und ausgewählt sind aus der Gruppe bestehend aus O-Angeloyl, O-Senecioyl, O-4-Pentenoyl, O-Hexanoyl, O-2-Ethylbutyryl, O-Crotonoyl, O-Cinnamoyl und O-Benzoyl; oder
(ii) R18 und drei von R1, R2, R5 und R8 für OH stehen; und
R17 und eines von R1, R2, R5 und R8 gleich sind und ausgewählt sind aus der Gruppe bestehend aus O-Angeloyl, O-Senecioyl, O-4-Pentenoyl, O-Hexanoyl, O-2-Ethylbutyryl, O-Acetyl, O-Crotonoyl, O-Cinnamoyl und O-Benzoyl.

3. Die Verbindung nach Anspruch 2 oder ein pharmazeutisch verträgliches Salz davon, wobei
(i) R2, R5 und R8 für OH stehen; und
R1, R17 und R18 gleich sind und ausgewählt sind aus der Gruppe bestehend aus O-Angeloyl, O-Senecioyl, O-4-Pentenoyl, O-Hexanoyl, O-2-Ethylbutyryl, O-Crotonoyl, O-Cinnamoyl und O-Benzoyl; oder
(ii) R2, R5, R8 und R18 für OH stehen; und
R1 und R17 gleich sind und ausgewählt sind aus der Gruppe bestehend aus O-Angeloyl, O-Senecioyl, O-4-Pentenoyl, O-Hexanoyl, O-2-Ethylbutyryl, O-Acetyl, O-Crotonoyl, O-Cinnamoyl und O-Benzoyl.

4. Die Verbindung nach Anspruch 3 oder ein pharmazeutisch verträgliches Salz davon, wobei
(i) R2, R5 und R8 für OH stehen; und R1, R17 und R18 für O-Angeloyl stehen; oder
(ii) R2, R5, R8 und R18 für OH stehen; und R1 und R17 für O-Angeloyl stehen.

5. Die Verbindung nach Anspruch 3 oder ein pharmazeutisch verträgliches Salz davon, wobei
(i) R2, R5 und R8 für OH stehen; und R1, R17 und R18 für O-Senecioyl stehen; oder
(ii) R2, R5, R8 und R18 für OH stehen; und R1 und R17 für O-Senecioyl stehen.

6. Die Verbindung nach Anspruch 3 oder ein pharmazeutisch verträgliches Salz davon, wobei
(i) R2, R5 und R8 für OH stehen; und R1, R17 und R18 für O-Crotonoyl stehen; oder
(ii) R2, R5, R8 und R18 für OH stehen; und R1 und R17 für O-Crotonoyl stehen.

7. Die Verbindung nach Anspruch 3 oder ein pharmazeutisch verträgliches Salz davon, wobei
(i) R2, R5 und R8 für OH stehen; und R1, R17 und R18 für O-Cinnamoyl stehen; oder
(ii) R2, R5, R8 und R18 für OH stehen; und R1 und R17 für O-Cinnamoyl stehen.

8. Die Verbindung nach Anspruch 3 oder ein pharmazeutisch verträgliches Salz davon, wobei
(i) R2, R5 und R8 für OH stehen; und R1, R17 und R18 für O-4-Pentenoyl stehen; oder
(ii) R2, R5, R8 und R18 für OH stehen; und R1 und R17 für O-4-Pentenoyl stehen.

9. Die Verbindung nach Anspruch 3 oder ein pharmazeutisch verträgliches Salz davon, wobei
(i) R2, R5 und R8 für OH stehen; und R1, R17 und R18 für O-Hexanoyl stehen; oder
(ii) R2, R5, R8 und R18 für OH stehen; und R1 und R17 für O-Hexanoyl stehen.

10. Die Verbindung nach Anspruch 3 oder ein pharmazeutisch verträgliches Salz davon, wobei
(i) R2, R5 und R8 für OH stehen; und R1, R17 und R18 für O-2-Ethylbutyryl stehen; oder
(ii) R2, R5, R8 und R18 für OH stehen; und R1 und R17 für O-2-Ethylbutyryl stehen.

11. Die Verbindung nach Anspruch 3 oder ein pharmazeutisch verträgliches Salz davon, wobei
(i) R2, R5 und R8 für OH stehen; und R1, R17 und R18 für O-Benzoyl stehen; oder
(ii) R2, R5, R8 und R18 für OH stehen; und R1 und R17 für O-Benzoyl stehen.

12. Die Verbindung nach einem der Ansprüche 1 bis 11 oder ein pharmazeutisch verträgliches Salz davon zur Verwendung als ein Medikament.

13. Die Verbindung nach einem der Ansprüche 1 bis 11 oder ein pharmazeutisch verträgliches Salz davon zur Verwendung bei der Behandlung von Krebs; Hemmung des Krebswachstums, der Krebsinvasion, wobei der Krebs ausgewählt ist aus der Gruppe bestehend aus Brustkrebs, leukozytärem Krebs, Leberkrebs, Eierstockkrebs, Blasenkrebs, Prostatakrebs, Hautkrebs, Knochenkrebs, Hirnkrebs, Leukämiekrebs, Lungenkrebs, Dickdarmkrebs, ZNS-Krebs, Melanomkrebs, Nierenkrebs, Gebärmutterhalskrebs, Speiseröhrenkrebs, Hodenkrebs, Milzkrebs, Nierenkrebs, lymphatischem Krebs, Bauchspeicheldrüsenkrebs, Magenkrebs und Schilddrüsenkrebs.

14. Eine Verbindung nach einem der Ansprüche 1 bis 11 oder ein pharmazeutisch verträgliches Salz davon zur Verwendung bei der Behandlung von Zelladhäsion, Zellanhaftung, Zellzirkulation; Hemmung von Viren; Vorbeugung von Hirnalterung; Verbesserung des Gedächtnisses; Verbesserung der Hirnfunktionen; Heilung von Enuresis, häufigem Urinieren, Harninkontinenz; Demenz, Alzheimer-Krankheit, Autismus, Hirntrauma, Parkinson-Krankheit oder anderen Krankheiten, die durch zerebrale Funktionsstörungen verursacht werden; Behandlung von Arthritis, Rheuma, schlechter Durchblutung, Arteriosklerose, Raynaud-Syndrom, Angina pectoris, Herzstörung, koronarer Herzkrankheit, Kopfschmerzen, Schwindel, Nierenerkrankung; zerebrovaskulärer Erkrankung; Hemmung der NF-Kappa B-Aktivierung; Behandlung von Hirnödemen, schwerem akutem Atemwegssyndrom, viralen Atemwegserkrankungen, chronischer venöser Insuffizienz, Bluthochdruck, chronischer Venenerkrankung, Ödemen, Entzündungen, Hämorrhoiden, peripherer Ödembildung, Krampfadererkrankung, Grippe, posttraumatischen Ödemen und postoperativen Schwellungen; Hemmung von Blutgerinnseln, Hemmung der Ethanolabsorption; Senkung des Blutzuckers oder Regulierung der Adrenocorticotropin- und Kortikosteronspiegel.

15. Eine Zusammensetzung, umfassend eine wirksame Menge einer Verbindung nach einem der Ansprüche 1 bis 11 oder ein pharmazeutisch verträgliches Salz davon als ein Medikament.

## Revendications

1. Composé de formule 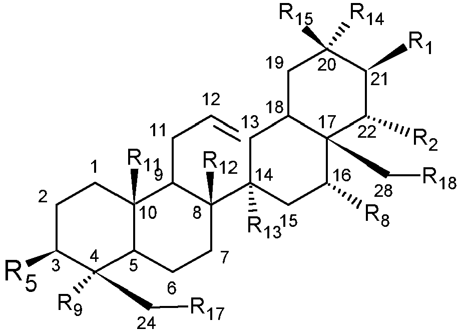 ou sel pharmaceutiquement acceptable de celui-ci,
dans lequel
R9, R11, R12, R13, R14, et R15 représentent CH3 ; et
(i) trois de R1, R2, R5 et R8 sont OH ; et
R17, R18 et l'un de R1, R2, R5 et R8 sont identiques et sont sélectionnés dans le groupe consistant en un O-angéloyle, un O-sénécioyle, un O-4-penténoyle, un O-hexanoyle, un O-2-éthylbutyryle, un O-crotonoyle, un O-cinnamoyle, et un O-benzoyle ; ou
(ii) R18 et trois de R1, R2, R5 et R8 sont OH ; et
R17 et l'un de R1, R2, R5 et R8 sont identiques et sont sélectionnés dans le groupe consistant en un O-angéloyle, un O-sénécioyle, un O-4-penténoyle, un O-hexanoyle, un O-2-éthylbutyryle, un O-acétyle, un O-crotonoyle, un O-cinnamoyle, et un O-benzoyle ; ou
(iii) R8 et deux de R1, R2, et R5 sont OH ;
R17 est un O-tigloyle ; et
R18 et l'un de R1, R2, et R5 sont identiques et sont sélectionnés dans le groupe consistant en un O-sénécioyle, un O-crotonoyle, un O-acétyle, un O-4-penténoyle, un O-hexanoyle, un O-cinnamoyle, un O-angéloyle, et un O-2-éthylbutyryle ; ou
(iv) R5, R8, R18, et l'un de R1 et R2 sont OH ;
R17 est un O-tigloyle ; et
l'un de R1 et de R2 est sélectionné dans le groupe consistant en un O-sénécioyle, un O-crotonoyle, un O-acétyle, un O-4-penténoyle, un O-hexanoyle, un O-cinnamoyle, un O-angéloyle, et un O-2-éthylbutyryle.

2. Composé selon la revendication 1, ou sel pharmaceutiquement acceptable de celui-ci,
dans lequel
(i) trois de R1, R2, R5 et R8 sont OH ; et
R17, R18, et l'un de R1, R2, R5 et R8 sont identiques et sont sélectionnés dans le groupe consistant en un O-angéloyle, un O-sénécioyle, un O-4-penténoyle, un O-hexanoyle, un O-2-éthylbutyryle, un O-crotonoyle, un O-cinnamoyle, et un O-benzoyle ; ou
(ii) R18 et trois de R1, R2, R5 et R8 sont OH ; et
R17 et l'un de R1, R2, R5 et R8 sont identiques et sont sélectionnés dans le groupe consistant en un O-angéloyle, un O-sénécioyle, un O-4-penténoyle, un O-hexanoyle, un O-2-éthylbutyryle, un O-acétyle, un O-crotonoyle, un O-cinnamoyle, et un O-benzoyle.

3. Composé selon la revendication 2, ou sel pharmaceutiquement acceptable de celui-ci,
dans lequel
(i) R2, R5 et R8 sont OH ; et
R1, R17 et R18 sont identiques et sont sélectionnés dans le groupe consistant en un O-angéloyle, un O-sénécioyle, un O-4-penténoyle, un O-hexanoyle, un O-2-éthylbutyryle, un O-crotonoyle, un O-cinnamoyle, et un O-benzoyle ; ou
(ii) R2, R5, R8 et R18 sont OH ; et
R1 et R17 sont identiques et sont sélectionnés dans le groupe consistant en un O-angéloyle, un O-sénécioyle, un O-4-penténoyle, un O-hexanoyle, un O-2-éthylbutyryle, un O-acétyle, un O-crotonoyle, un O-cinnamoyle, et un O-benzoyle.

4. Composé selon la revendication 3, ou sel pharmaceutiquement acceptable de celui-ci,
dans lequel
(i) R2, R5 et R8 sont OH ; et R1, R17 et R18 sont un O-angéloyle ; ou
(ii) R2, R5, R8 et R18 sont OH ; et R1 et R17 sont un O-angéloyle.

5. Composé selon la revendication 3, ou sel pharmaceutiquement acceptable de celui-ci,
dans lequel
(i) R2, R5 et R8 sont OH ; et R1, R17 et R18 sont un O-sénécioyle ; ou
(ii) R2, R5, R8 et R18 sont OH ; et R1 et R17 sont un O-sénécioyle.

6. Composé selon la revendication 3, ou sel pharmaceutiquement acceptable de celui-ci,
dans lequel
(i) R2, R5 et R8 sont OH ; et R1, R17 et R18 sont un O-crotonoyle ; ou
(ii) R2, R5, R8 et R18 sont OH ; et R1 et R17 sont un O-crotonoyle.

7. Composé selon la revendication 3, ou sel pharmaceutiquement acceptable de celui-ci,
dans lequel
(i) R2, R5 et R8 sont OH ; et R1, R17 et R18 sont un O-cinnamoyle ; ou
(ii) R2, R5, R8 et R18 sont OH ; et R1 et R17 sont un O-cinnamoyle.

8. Composé selon la revendication 3, ou sel pharmaceutiquement acceptable de celui-ci,
dans lequel
(i) R2, R5 et R8 sont OH ; et R1, R17 et R18 sont un O-4-penténoyle ; ou
(ii) R2, R5, R8 et R18 sont OH ; et R1 et R17 sont un O-4-penténoyle.

9. Composé selon la revendication 3, ou sel pharmaceutiquement acceptable de celui-ci,
dans lequel
(i) R2, R5 et R8 sont OH ; et R1, R17 et R18 sont un O-hexanoyle ; ou
(ii) R2, R5, R8 et R18 sont OH ; et R1 et R17 sont un O-hexanoyle.

10. Composé selon la revendication 3, ou sel pharmaceutiquement acceptable de celui-ci,
dans lequel
(i) R2, R5 et R8 sont OH ; et R1, R17 et R18 sont un O-2-éthylbutyryle ; ou
(ii) R2, R5, R8 et R18 sont OH ; et R1 et R17 sont un O-2-éthylbutyryle.

11. Composé selon la revendication 3, ou sel pharmaceutiquement acceptable de celui-ci,
dans lequel
(i) R2, R5 et R8 sont OH ; et R1, R17 et R18 sont un O-benzoyle ; ou
(ii) R2, R5, R8 et R18 sont OH ; et R1 et R17 sont un O-benzoyle.

12. Composé selon l'une quelconque des revendications 1 à 11, ou sel pharmaceutiquement acceptable de celui-ci, pour utilisation en tant que médicament.

13. Composé selon l'une quelconque des revendications 1 à 11, ou sel pharmaceutiquement acceptable de celui-ci, pour utilisation dans le traitement du cancer; dans l'inhibition de la croissance du cancer, de l'invasion du cancer, dans lequel le cancer est sélectionné dans le groupe consistant en le cancer du sein, le cancer leucocytaire, le cancer du foie, le cancer de l'ovaire, le cancer de la vessie, le cancer de la prostate, le cancer de la peau, le cancer des os, le cancer du cerveau, le cancer leucémique, le cancer du poumon, le cancer du côlon, le cancer du SNC, le mélanome cancéreux, le cancer rénal, le cancer du col de l'utérus, le cancer de l'œsophage, le cancer des testicules, le cancer de la rate, le cancer du rein, le cancer lymphatique, le cancer du pancréas, le cancer de l'estomac et le cancer de la thyroïde.

14. Composé selon l'une quelconque des revendications 1 à 11, ou sel pharmaceutiquement acceptable de celui-ci, pour utilisation dans le traitement de l'adhérence cellulaire, de la fixation cellulaire, de la circulation cellulaire ; dans l'inhibition des virus ; dans la prévention du vieillissement cérébral ; dans l'amélioration de la mémoire ; dans l'amélioration des fonctions cérébrales ; dans le traitement de l'énurésie, de la miction fréquente, de l'incontinence urinaire ; de la démence, de la maladie d'Alzheimer, de l'autisme, d'un traumatisme cérébral, de la maladie de Parkinson ou d'autres maladies provoquées par des dysfonctions cérébrales ; dans le traitement de l'arthrite, des rhumatismes, d'une mauvaise circulation, de l'artériosclérose, du syndrome de Raynaud, de l'angine de poitrine, d'un trouble cardiaque, d'une maladie coronarienne, des céphalées, des vertiges, d'un trouble rénal; d'une maladie cérébrovasculaire ; dans l'inhibition de l'activation du NF-Kappa B ; dans le traitement d'un œdème cérébral, du syndrome respiratoire aigu sévère, de maladies respiratoires virales, de l'insuffisance veineuse chronique, de l'hypertension, d'une maladie veineuse chronique, d'un œdème, d'une inflammation, des hémorroïdes, d'une formation d'œdème périphérique, d'une maladie des veines variqueuses, de la grippe, d'un œdème post-traumatique et d'un gonflement post-opératoire ; dans l'inhibition des caillots de sang, dans l'inhibition de l'absorption d'éthanol ; dans la réduction de la glycémie ; ou dans la régulation des niveaux d'adrénocorticotropine et de corticostérone.

15. Composition comprenant une quantité efficace d'un composé selon l'une quelconque des revendications 1 à 11, ou d'un sel pharmaceutiquement acceptable de celui-ci, en tant que médicament.
